# EUROPEAN PATENT SPECIFICATION

(11) **EP 1 660 539 B1**
(45) Date of publication and mention of the grant of the patent: **02.06.2010**
(21) Application number: 04739024.0
(22) Date of filing: 06.08.2004
(51) Int. Cl.: C07K 19/00, C07K 14/705, C07K 14/78, C07K 14/52, C12N 9/64, A61K 38/17, A61K 38/43, A61K 38/19, A61K 38/39

(54) **COMPOUNDS COMPRISING LPA**
LPA-HALTIGE VERBINDUNGEN
COMPOSES COMPRENANT UN LPA (ASSEMBLAGE DE PRESENTATION DE LIGANDS)

(30) Priority: 07.08.2003 DK 200301141; 25.05.2004 DK 200400814
(43) Date of publication of application: 31.05.2006
(73) Proprietor: Enkam Pharmaceuticals A/S, 2100 Copenhagen 0 (DK)
(72) Inventor: ALBRECHTSEN, Morten, DK-2920 Charlottenlund (DK); BOCK, Elisabeth, DK-2920 Charlottenlund (DK); BEREZIN, Vladimir, DK-2200 Copenhagen N (DK); HOLM, Arne, DK-2942 Skodsborg (DK)
(74) Representative: Høiberg A/S
(86) International application number: PCT/DK2004/000527
(87) International publication number: WO 2005/014623

(56) References cited:
- WO-A-00/18791
- WO-A-01/16166
- WO-A-03/016351
- WO-A-2004/056865
- STAHLHUT M ET AL: "NCAM-FIBRONECTIN-TYPE-III-DOMAIN SUBSTRATA WITH AND WITHOUT A SIX-AMINO-ACID-LONG PROLINE-RICH INSERT INCREASE THE DENDRITIC AND AXONAL ARBORIZATION OF SPINAL MOTONEURONS" JOURNAL OF NEUROSCIENCE RESEARCH, WILEY-LISS, US, vol. 48, 1997, pages 112-121, XP009024260 ISSN: 0360-4012
- KISELYOV VV ET AL.: "Structural Basis for a Direct Interaction between FGFR1 and NCAM and Evidence for a Regulatory Role of ATP" STRUCTURE, vol. 11, June 2003 (2003-06), pages 691-701, XP002319882 cited in the application

### SEQUENCE LISTING

<110> ENKAM Pharmaceutical A/S
<120> Compounds comprising LPA
<130> P 770 PC00
<160> 146
<170> PatentIn version 3.1
<210> 1
   <211> 15
   <212> PRT
   <213> Artificial sequence
<220>
   <223> NCAM Fn III, 2 [Swiss-Prot: P13591]: FGFR binding motif
<400> 1
   Glu Val Tyr Val Val Ala Glu Asn Gln Gln Gly Lys Ser Lys Ala
   1               5                   10                  15

## Description

### Field of invention.

The present invention relates to peptide compounds capable of binding to fibroblast growth factor receptor (FGFR), said compounds comprising two identical amino acid sequences, wherein the two amino acid sequences are capable of binding to FGFR. The invention discloses the amino acid sequences of the compounds and features pharmaceutical compositions comprising thereof. Invention also relates to uses of the compounds and pharmaceutical compositions for treatment and/or prevention of different pathological conditions, wherein FGFR plays a role in pathology and/or recovery from the disease. The peptide compounds of the invention are obtainable by the ligand presenting assembly (LPA) method.

### Background of invention

Brain plasticity and the mechanisms controlling plasticity are central to learning and memory as well as the recovery of function after brain injury. While it is clear that neurotrophic factors are one of the molecular classes that continue to regulate brain plasticity in the adult central nervous system (CNS), less appreciated but equally profound is the role of cell adhesion molecules (CAMs) in plasticity mechanisms such as long term potentiation, preservation of neurons and regeneration. Ironically, however, CAMs can also reorganise the extra-cellular space and cause disturbances that drive the development of brain pathology in conditions such as Alzheimer's disease and multiple sclerosis. Candidate molecules include the amyloid precursor protein, which shares many properties of a classical CAM and beta-amyloid, which can masquerade as a pseudo CAM. Beta-Amyloid serves as a nidus for the formation of senile plaques in Alzheimer's disease and like CAMs provides an environment for organising neurotrophic factors and other CAMs. Inflammatory responses evolve in this environment and can initiate a vicious cycle of perpetuated neuronal damage that is medicated by microglia, complement and other factors (Cotman et al. (1998) Prog Neurobiol. 55:659-69).

Neural cell adhesion molecules (CAMs) of the immunoglobulin superfamily nucleate and maintain groups of cells at key sites during early development and in the adult. In addition to their adhesive properties, CAMs homophylic and heterophylic interactions can affect intracellular signalling. Their ability to influence developmental events, including cell migration, proliferation, and differentiation may therefore result from both their adhesive and signalling properties.

The neural cell adhesion molecule, NCAM, was the first discovered neural CAM. Since the discovery NCAM has been intensively studied and now it is well characterised (Ronn et al. (2000) Int J Dev Neurosci 18:193-9) NCAM belongs to the immunoglobulin (Ig) superfamily. Its extracellular part consists of five Ig-like and two fibronectin type III (F3) modules. NCAM assists both the cell-cell and cell-substratum interactions. NCAM binds to various extracellular matrix components such as heparin/heparan sulfate, chondroitin sulfate proteoglycans, and different types of collagen. Cell-cell interactions are mostly assisted by the NCAM homophilic interaction. The different modules of NCAM have been shown to perform distinct functions. Thus, NCAM homophilic binding is now believed to depend on the first three Ig modules. The heparin binding sequence is localised to the Ig2 module. NCAM also binds to the neural cell adhesion molecule L1. This interaction is believed to take place between the fourth Ig module of NCAM and an oligomannosidic moiety expressed on L1. The two membrane-proximal F3 modules of NCAM have been shown involved in fibroblast growth factor receptor (FGFR) binding.

A number of research groups has now accumulated a large body of evidence indicating that intracellular signalling cascades underlying the NCAM-mediated axonal outgrowth are similar to signal transduction cascades which are activated due to stimulation of FGFR (Povisen et al. (2003) Neurochem Res 1:127-41).

Fibroblast growth factor receptors (FGFRs) are a family of four closely related receptor protein tyrosine kinases consisting extracellularly of three Ig-like modules and intracellularly of a split tyrosine-kinase module (Powers et al. (2000) Endocr Relat Cancer 7:165-97). The receptors are known as key regulators of morphogenesis, development, angiogenesis, and wound healing. FGFR activation and signalling are dependent on dimerization of the receptor which is induced by high affinity binding of FGFR natural ligand, fibroblast growth factor (FGF), and it also requires participation of cell surface heparin or heparan sulphate proteoglycans. Fibroblast growth factors (FGFs) and their receptors constitute an elaborate signaling system that participates in many developmental and repair processes of virtually all mammalian tissues, in particular, they play a prominent role in functioning of the peripheral and central neural system. Thus, among 23 members of the FGF family, ten have been identified in the brain (Jungnickel et al, (2004) Mol Cell Neurosci. 25:21-9; Reuss and von Bohlen und Halbach (2003) Cell Tissue Res. 313:139-57).

NCAM has recently been regarded as a member of a new class of putative alternative ligands of FGFR, the low affinity binding ligands. There has been obtained evidence for a direct interaction between NCAM and the receptor (Kiselyov et al. (2003) Structure (Camb) 11:691-701).

The identified NCAM fragment having the sequence EVYVVAENQQGKSKA (FGL peptide) involved in the direct interaction between NCAM and FGFR has recently been suggested as a new candidate drug for the treatment of a variety of pathologic disorders where the regulation of activity of FGFR may play the key role (WO 03/016351). WO 03/016351 describes some biological effects of the FGL peptide due to binding and activating FGFR. According to WO 031016351, presentation of a single copy (monomer) of the peptide to neuronal cells in vitro is enough to promote cell survival and differentiation. However, rather a high concentration of the peptide is needed to achieve the effects.

Multiple presentation of peptide sequences, e.g. antigenic peptides, has been shown to be a valuable mean to amplify biological responses of peptide sequences in vitro (Berezin and Bock (2004) J Mol Neurosci.22:33-39; Ronn et al (1999) Nat Biotechnol 17:1000-5), and it has also been effectively used in animal models in vivo (Cambon et al. (2004) J Neurosci. 17:4197-204).

Synthetic dendritic polymers of biologically active peptide sequences are now used in research and some clinical applications. A dendritic polymer consists of a number of copies of a monomeric peptide sequence, which is attached to a core molecule at multiple sites forming thereby a branching polymer. Lysine is most commonly used amino acid in the core matrix because it has two reactive amino groups available for branching reactions. The multimerization of peptide sequences on the lysine core is known as the *m*ultiple *a*ntigen *p*eptide (MAP) presentation. A method for the synthesis of MAP(s) (dendrimeric peptides) was described in PCT/US90/02039.

In general, two routs can be used for the synthesis of a dendrimeric peptide, namely the direct and indirect route. In both cases, the C-terminal core is first assembled on a solid support using bi-protected Boc-Lys(Boc) in case of Boc chemistry, or Fmoc-Lys(Fmoc) in the case of Fmoc chemistry in order to obtain the desired degree of branching.

In the direct route synthesis is done by stepwise assembling of the particular peptide on the lysinyl core by the well-known Merrifield method. This stepwise method produces peptides with a C to N orientation. The peptide chains may be synthesised in tandem, or, alternatively, each peptide can be synthesised on the different lysine arms of the core using orthogonal deprotection methods.

Although this approach for dendrimeric peptide synthesis is convenient, the quality of the final product may be questionable and problems of obtaining a well-defined product have been widely discussed in the literature. Problems arise during chain assembly. The MAP type dendrimers are macromolecules, where each single peptide chains may uncontrolled interact with each other and prevent thereby high efficiency coupling to the core leading to production of non-homogenous compound, which require effort to purification. Characterisation of these products by, e.g. electrospray mass spectroscopy (ES-MS) is difficult and as a result, dendritic peptide products are often used without full characterisation.

In the indirect route the synthesis is done by coupling of purified unprotected peptide fragments to the core matrix. Two general methods for this type of ligation may be used, which, respectively, are based on thiol and carbonyl chemistries.

The thiol chemistry may be carried out by incorporation of chloroacetyl group(s) on the lysine matrix and subsequent coupling to a purified, synthetic N-terminal cysteinyl peptide to yield a dendrimer with unambiguous structure. The reverse placement may be achieved with thiol on the core matrix by using an S-acetyl group on the lysinyl core and the haloacetyl group on the peptide. In the carbonyl chemistry the condensation takes place between a carbonyl group and a weak base. There are two types of weak bases, which may be used for this reaction. The first includes hydroxylamines and hydrazines, and the second includes 1,2-di-substituted moieties such as 1,2-aminoethanthiol and 1,2-aminoethanol. The last two groups are found in N-terminal Cys and Thr or Ser respectively, and the condensation products are thiazolidine and oxazolidine. The carbonyl group on the core matrix may be obtained by periodate oxidation of N-terminal Ser, Thr or Cys. Both methods are well known in the art (see e. g. Lu et al., (1991) Mol Immunol 28:623-630; Defoort et al., (1992) int J Pept Prot Res 40:214-221; Drijfhout et al. (1991) Int J Pept Prot Res 37:27-32).

Another problem associated with the MAP type compounds is orientation of peptide chains. In the direct route with stepwise assembling of the desired peptide on the lysinyl core only a compound having peptide chains with C to N orientation can only be obtained. The indirect route allows the both orientations, C to N and N to C, but the process has other major disadvantages, e. g. undesirable side reactions due to oxidation of disulphides, leading to impure products, interference with cystein residues present in the native peptide chains, and the procedure demands optimisation for every synthesis.

Finding the optimal number of multiplication of a peptide sequence is another problem to be solved. Most studies of dendritic peptide polymers synthesised by the MAP method have been carried out using tetra- or octameric polymers. However, application of the compounds comprising 4-8 sequences of 15 or more amino acids in vivo is challenged by a problem of the capability of a compound to penetrate the blood-brain barrier, which is crucial if the compound is for the treatment of the brain. Dimers of the peptide sequences may be a solution to this problem, in case the dimers possess biological activity of the dendritic tetramer analogues. Production and use of biologically active peptide dimers comprising different linkers is also well known in the art (see e.g. WO00/18791, WO00/24770).

### Summary of Invention

Although, a dendrimeric version of the FGL peptide consisting of four sequences of FGL has been shown to be effective in vivo in rat (Cambon et al. (2004) J Neurosci. 17:4197-204), the athours of the present invention found that it is preliminary to consider using of the dendrimer as a drug candidate for the treatment of human patients. Final purification of the dendritic tetramer of the FGL peptide revealed that the product of the MAP synthesis is highly heterogenous and its characterisation is difficult. To solve this problem the authors of the present invention attempted to synthesise different dimers of FGF, using known synthetic procedures. Surprisingly, the only FGL dimer that had biological activity similar to the FGL dendrimer was a dimer produced by the ligand presenting assembly method (LPA), as disclosed in WO00/18791. Other dimers of FGL, such as a dimer wherein the FGL sequences were linked by a Cys residue, as disclosed in Goodwin et al. (1998) Bioorg Med Chem Lett 8:2231-2234, or by a Lys residue, as disclosed in Rajagopalan et al. (1995) Int J Pept Protein Res 45:173-179, did not have same activity.

Accordingly in the first aspect the present invention relates to a compound comprising two peptide fragments comprising the identical amino acid sequence, said amino acid sequence being EVYVVAENQQGKSKA (SEQ ID NO:1), or a fragment thereof, said fragment being an amino acid sequence which has at least 40% of the length of SEQ ID NO:1 and which is capable of binding to fibroblast growth factor receptor,
wherein
said peptide sequences are connected to each other through a linker of the formula

X[(A)nCOOH][(B)mCOOH],

wherein
n and m independently are an integer of from 1 to 20,
A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, or substituted or unsubstituted aromatic moiety,
X is HN, H₂N(CR₂)pCR, RHN(CR₂)pCR, HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, [HOOC(A)n][HOOC(B)m]CR(CR₂)pCR, H₂N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, or [HOOC(A)n][HOOC(B)m](CR₂)p , wherein p is 0 or integer of from 1 to 20, each R indepentently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety.

The above compound is according to the invention is obtainable by the LPA method.

In another aspect, the invention relates to a pharmaceutical composition comprising a compound as defined above.

In still another aspect, the invention concerns using the above compound for the manufacture of a medicament for
a) treatment of conditions of the central and peripheral nervous system associated with postoperative nerve damage, traumatic nerve damage, impaired myelination of nerve fibers, postischaemic damage, e.g. resulting from a stroke, Parkinson's disease, Alzheimer's disease, Huntington's disease, dementias such as multiinfarct dementia, sclerosis, nerve degeneration associated with diabetes mellitus, disorders affecting the circadian clock or neuro-muscular transmission, and schizophrenia, mood disorders, such as manic depression;
b) treatment of diseases or conditions of the muscles including conditions with impaired function of neuro-muscular connections, such as after organ transplantation, or such as genetic or traumatic atrophic muscle disorders; or for treatment of diseases or conditions of various organs, such as degenerative conditions of the gonads, of the pancreas such as diabetes mellitus type I and II, of the kidney such as nephrosis and of the heart, liver and bowel;
c) promotion of wound-healing;
d) prevention of death of heart muscle cells, such as after acute myocardial infarction, or after angiogenesis;
e) promotion of revascularsation;
f) stimulation of the ability to learn and/or the short and/or long-term memory;
g) prevention of cell death due to ischemia;
h) prevention of body damages due to alcohol consumption;
i) treatment of prion diseases;
j) treatment of cancer.

### Description of Drawings

**Figure 1** shows the flow chart of the synthesis of the LPA-type FGL dimer (FGL_{L})
**Figure 2** presents the HPLC purification profile of FGL_{L}
**Figure 3** presents the HPLC purification profile of FGL_{D}
**Figure 4** demonstrates the effect of the FGL peptide on survival of primary neurons treated with various neurotoxic agents.
   Dopaminergic neurons (DN) (**a** and **b**) from day 15 rat embryos grown at a density of 150,000 cells/cm² for six days without or with various concentrations of peptide on 24-well cell culture plates coated with poly-D-lysine were exposed to 100 µM 6-OHDA for two hours. Medium was changed and various concentrations of FGL_{d} were added. The neurons were grown for another 24 hours before the cultures they were fixed and immunostained for tyrosine hydroxylase.
   Hippocampal neurons (HN) (**c** and **d**) from day 19 rat embryos were seeded at a density of 40,000 cells/cm² on poly-L-lysine coated 8-well permanox chamber slides and grown for 24 hours in medium containing 20 µM Amyloid-β 25-35 peptide (Aβ 25-35) at the presence of various concentrations of FGL_{d}, before they were fixed and stained with Hoechst 33258.
   Cerebella granular meurons (CGN) (**e** and **f**) from postnatal day 7 rats were grown at a density of 100,000 cells/cm² for 7 days on poly-L-lysine coated microtiter plates in the medium containing 40 mM KCI, then the medium was substituted to a 5 mM KCl containing medium suplemented with various concentrations of FGL_{d}. After two days of incubation, the cultures were fixed and stained with Hoechst 33258.
   (a) - Effect of 10 ng/ml GDNF on survival of dopaminergic neurons treated with 6-OHDA.
   (b) Effect of various concentrations of FGL_{d} on survival of dopaminergic neurons treated with 6-OHDA.
   (c) Effect of 50 ng/ml BDNF on hippocampal cultures treated with Aβ 25-35.
   (d) Effect of various concentrations of FGL_{d} on survival of hippocampal neurons treated with Aβ 25-35.
   (e) Effect of 50 ng/ml IGF-1 on CGN cultures induced to undergo apoptosis by depriving the neurons of high potassium.
   (f) Effect of various concentrations of FGLd on CGN cultures induced to undergo apoptosis.
      Results from at least four independent experiments for each type of culture are expressed as percentage ± SEM of live neurons as compared to the total number of neurons. Control cultures induced to undergo cell death without FGL-treatment were set at 100 %. + p<0.05, ++ p<0.01 when compared to the untreated controls. * p<0.05, ** p<0.01 and *** p<0.001 when compared to the cultures induced to undergo cell death.

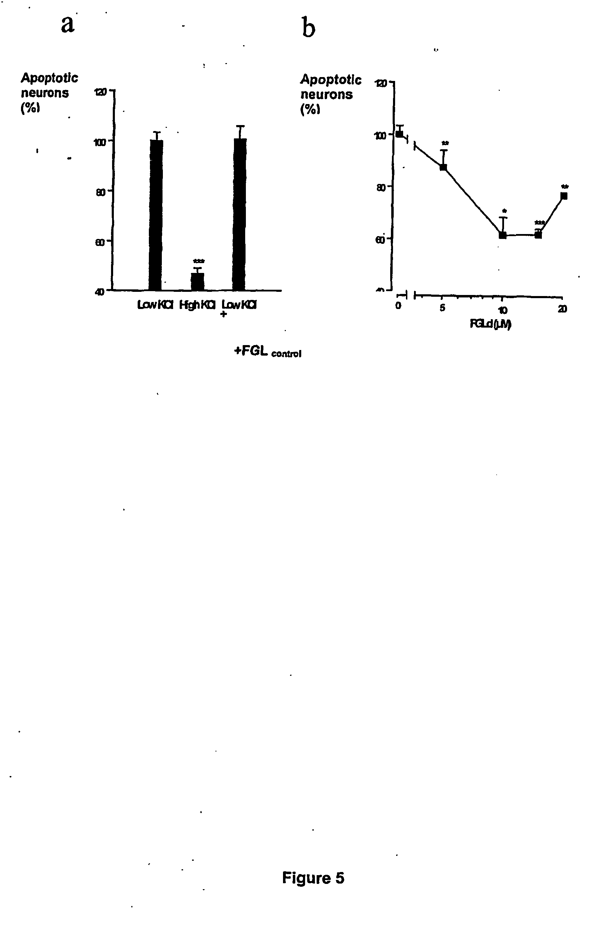

**Figure 5** demonstrates the effect of the FGL peptide on DNA-fragmentation in CGN cultures induced to undergo apoptosis by changing the medium after the neurons were grown for six days in high KCI medium (40 mM) to a low KCl medium (5 mM KCl). The number of neurons with fragmented DNA was measured using the ApoAlert apoptosis detection kit and the fraction of TUNEL-positive neurons was estimated by counting. Results from at least four independent experiments are shown as percentage of cells showing DNA-fragmentation relative to the total number of cells ± SEM with control cultures in low KCI set at 100 %.
   (a) Effect of depriving CGN cultures of high KCI on number of cells undergoing apoptosis.
   (b) Effect of FGLd in apoptosis-induced CGN cultures.
      * p<0.05, ** p<0.01 and *** p<0.001 when compared to low KCI-treated CGN cultures.

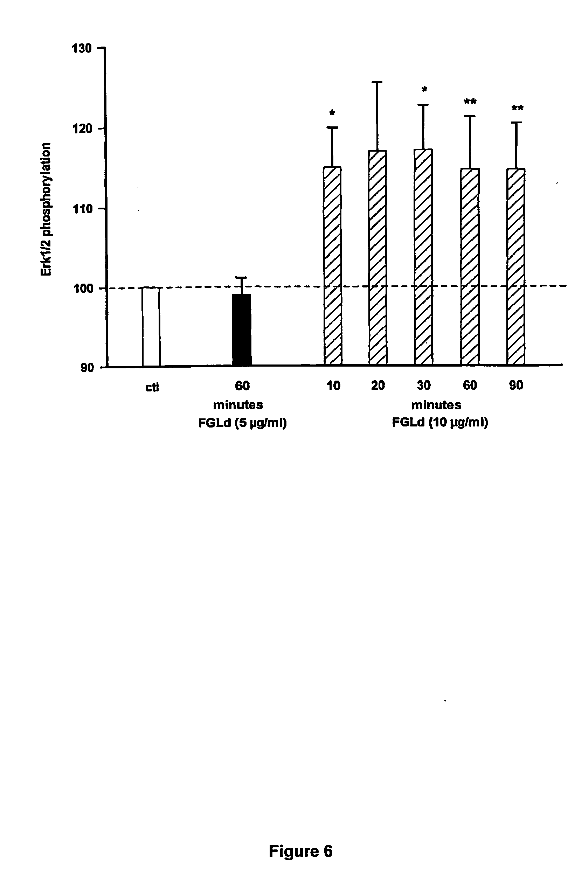

**Figure 6** shows the effect of FGL on phosphorylation of Erk1/2. CGN were grown for three days at a density of 290,000 cells/cm² on poly-L-lysine coated microtiter plates, were subsequently treated with FGL_{d} for 10 - 90 min, fixed and stained with antibodies against phospho-p42/44. The total number of neurons was estimated using crystal violet staining. Results from at least three independent experiments are expressed as percentage ± SEM with untreated cultures set at 100 %. * p<0.05 and ** p<0.01 when compared to the controls.

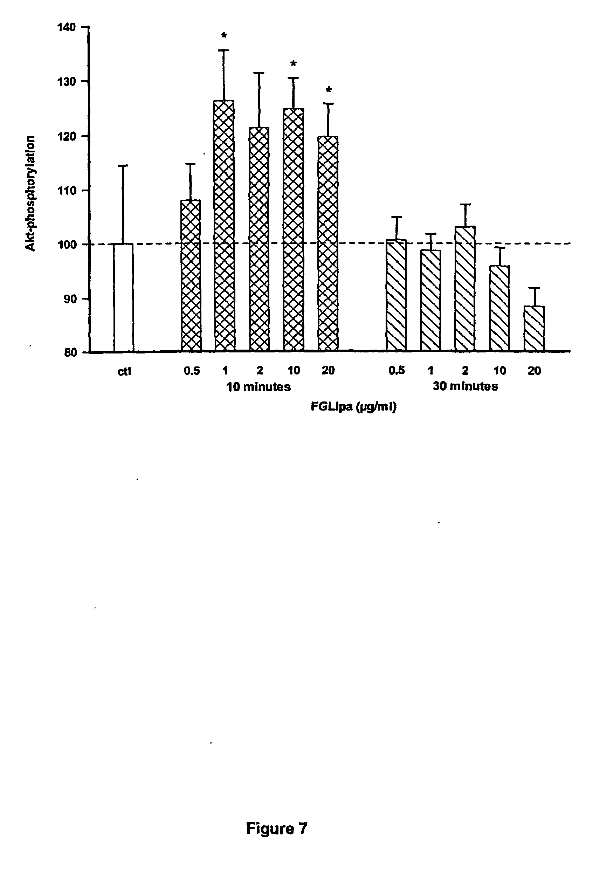

**Figure 7** shows the effect of FGL on phosphorylation of Akt. CGN were grown for three days at a density of 290,000 cells/cm² on poly-L-lysine coated microtiter plates, were subsequently treated with FGL_{L} at various concentrations for 10 or 30 minutes respectively and subsequently fixed and stained with antibodies against Akt phosphorylated on Ser473. The total number of neurons was estimated using crystal violet staining. Results from at least three independent experiments are expressed as percentage ± SEM with untreated cultures set at 100 %. * p<0.05 when compared to the controls.

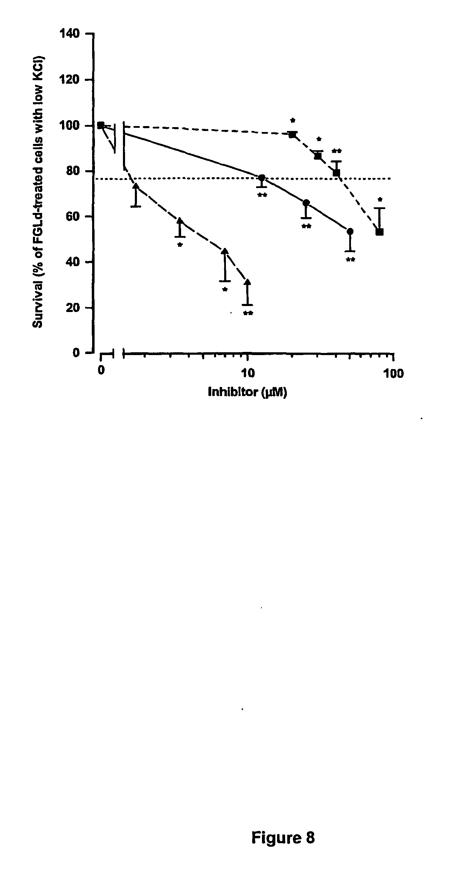

**Figure 8** shows the effect of inhibitors of FGFR, MEK and PI3K on FGL-induced survival of CGN induced to undergo apoptosis. CGN were grown at a density of 100,000 cells/cm² for seven days on poly-L-lysine coated 8-well permanox slides in medium containing 40 mM KCI, before the medium was changed to a 5 mM KCl containing medium together with FGL_{d}. Thereafter the MEK inhibitor PD98059 (•), PI3K inhibitor LY294002 (A) and FGFR inhibitor SU5402 (■) were added at various concentrations. The addition of the inhibitors followed in all cases by the addition of 10 µg/ml PGL_{d}. After two days of incubation with the peptide and inhibitors, the cultures were fixed and stained with Hoechst 33258. Results from at least four individual experiments are shown as percentage of the number of live neurons relative to the total number of neurons ± SEM, with control cultures treated with FGL_{d} set at 100%.

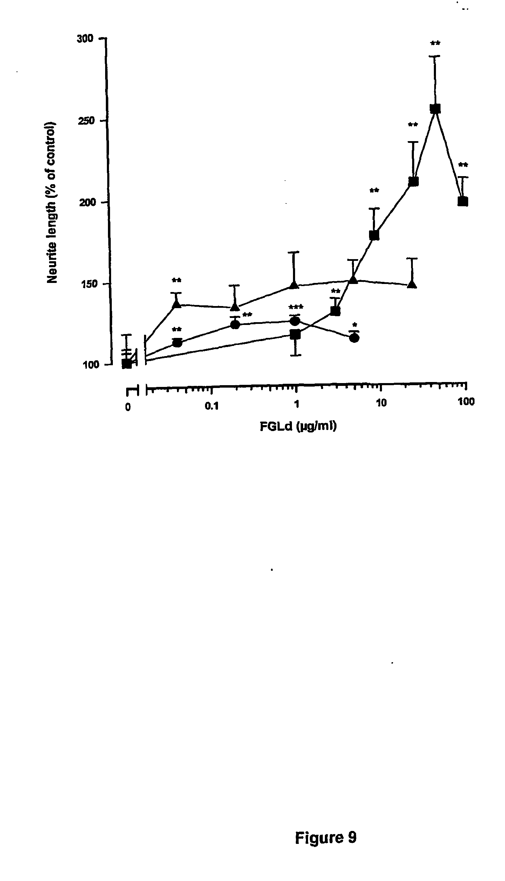

**Figure 9** shows the effect of the FGL peptide on neurite outgrowth from dopaminergic (•), hippocampal (A) and cerebellar granule neurons (■). Dopaminergic neurons were grown at a density of 100,000 cells/cm² on poly-D-lysine coated 24-well cell culture plates for 72 hours with various concentrations of PGL_{d}. The cultures were subsequently immunostained for tyrosine hydroxylase. Hippocampal neurons and CGN were plated at a density of 10,000 cells/cm² on 8-well permanox chamber slides and incubated for 24 hours in the presence of various concentrations of FGL_{d}. Subsequently the neurons were immunostained for GAP-43. Results from at least five independent experiments for each neuronal culture are shown as percentage ± SEM with the untreated controls set at 100 %. * p<0.05, ** p<0.01, *** p<0.001 when compared to the controls.

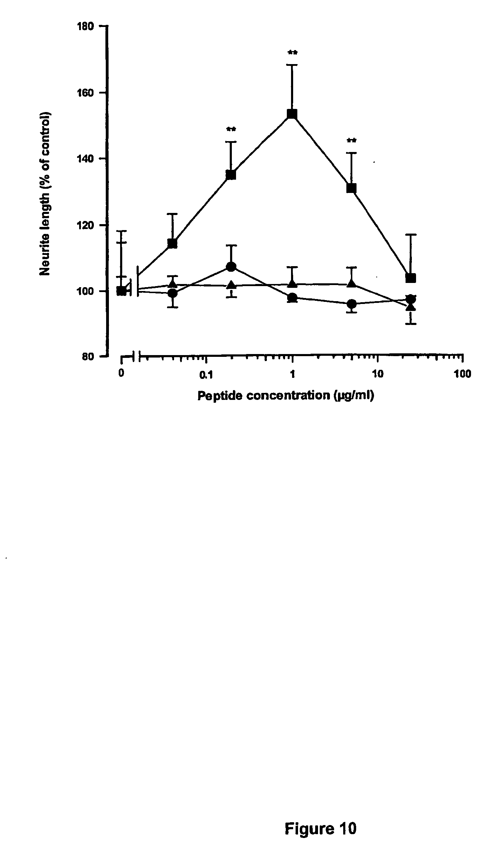

**Figure 10** shows the effect of three dimeric versions of the FGL peptide on neurite outgrowth of hippocampal neurons from newborn rats. Primary cultures of hippocampal neurons were grown for 24 hours at a density of 10,000 cells/cm² with various concentrations of FGL_{L} (■), FGL_{lys} (▲) or FGL_{cys} (•) and then were immunostained for GAP-43. Results from at least four independent experiments are shown as percentage ± SEM with untreated controls set at 100 %. ** p<0.01 when compared to the controls.

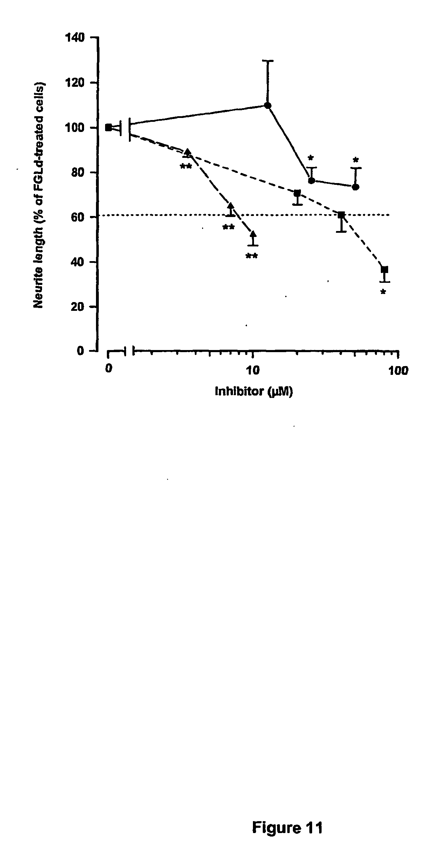

**Figure 11** shows the effect of inhibitors of FGFR, MEK and PI3K on FGL-induced neurite outgrowth in CGN cultures. CGN cultures were grown for 24 hours at a density of 10,000 cells/cm² on 8-well permanox slides in the presence of the FGL peptide and an inhibitor. PD98059 (•) was added at concentrations of 12.5, 25 and 50 µM simultaneously with 10 µg/ml FGL_{d}; LY294002 (A) was added at concentrations of 3.5, 7 and 10 µM simultaneously with 27 µg/ml FGL_{d}. SU5402 (■) was added in concentrations of 20, 40 and 80 µM simultaneously with 27 µg/ml FGL_{d}. After the treatment cells were fixed and immunostained for GAP-43. Results from at least four independent experiments are shown as percentage ± SEM, with FGLd-treated control cultures set at 100 %. The dashed line indicates the average neurite length in cultures without FGL_{d}-treatment. * p<0.05, ** p<0.01 when compared to the FGL_{d}-treated controls.

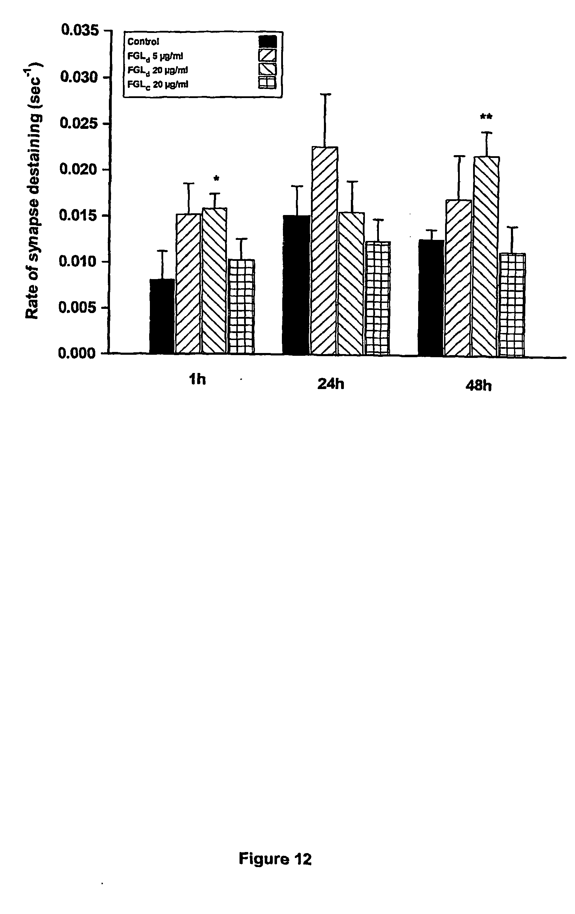

**Figure 12** shows the effect of FGL_{d}, FGL_{control} and vehicle on the rate of FM 1-43 destaining of primary cultures of hippocampal neurons from E19 rats. Each value represents the mean of 4-8 experiments and error bars indicate S.E.M. Neither treatment with 5 µg/ml FGL_{d} for any time period, nor the treatment with 20 µg/ml for 24 hours had any effect on the rate of FM 1-43 unloading. In contrast, synapses in cultures treated with 20 µg/ml FGL_{d} for 1 hour and 48 hours showed a significantly increased rate of FM 1-43 unloading when compared to control cultures, indicating an enhanced presynaptic response in these cultures. The values were compared using an unpaired t-test *p= 0.04 (1h) and **p = 0.0032 (48h). FGL_{control} is a control peptide consisting the sequence EVYWAENAAGKSKA (SEQ ID NO: 147). Mutation of Gln residues of the FGL sequence to Ala has been shown to abrogate the FGL activity (Kiselyov et al. 2003 see above. cit).

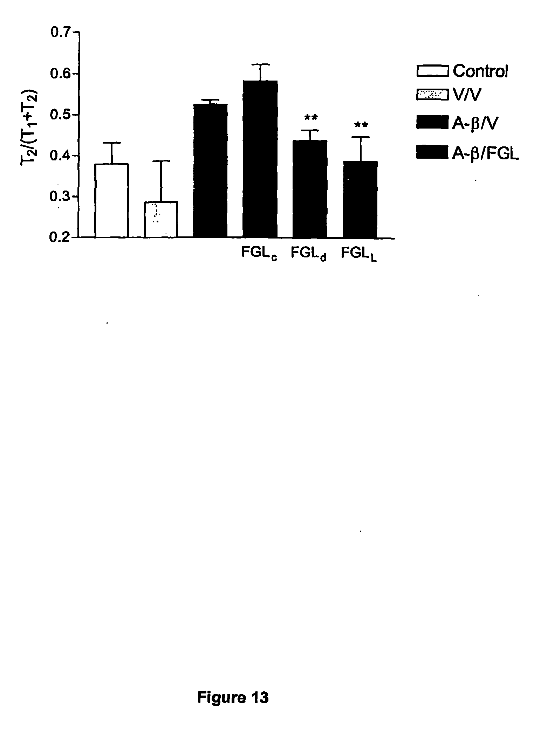

**Figure 13** demonstrates the effect of early sub-occipital administration of FGL_{L}, FGL_{D} and FGL_{control} on memory (Social Recognition test) of rats in which cognitive impairment was induced by intracerebroventricular (i.c.v.) -injection of the (25-35) β-amyloid fragment. The results are from the experiment in which the suboccipital administration of 5.0 µg FGL per administration per rat was done at days 7, 10, and 13 after A-β-induced neurotoxicity. The Social Recognition test was performed 21 days after i.c.v. administration of the (25-35) β-amyloid fragment and estimated as the ratio between the times the adult rat spent exploring a juvenile rat during a first (T₁) and a second (T₂) meeting. The number of animals in the groups varied from 4 to 22. The results were analysed using one-way ANOVA (F(2,27)=15.4, P<0.0001) and significance of results was contingent on achieving a p-value of less than 0.05 (**p<0.01), when compared to the A-β/V group (Newman-Keuls post-test). A statistically significant effect was also observed between the control and the V/V-treated group compared to the A-β/V group (p<0.001). 'A-β' and 'V' are the abbreviations of the (25-35) β-amyloid fragment and vehicle, respectively. 'V/V' stands for the rats received vehicle as replacement of A-β and FGL. FGL_{control} as above.

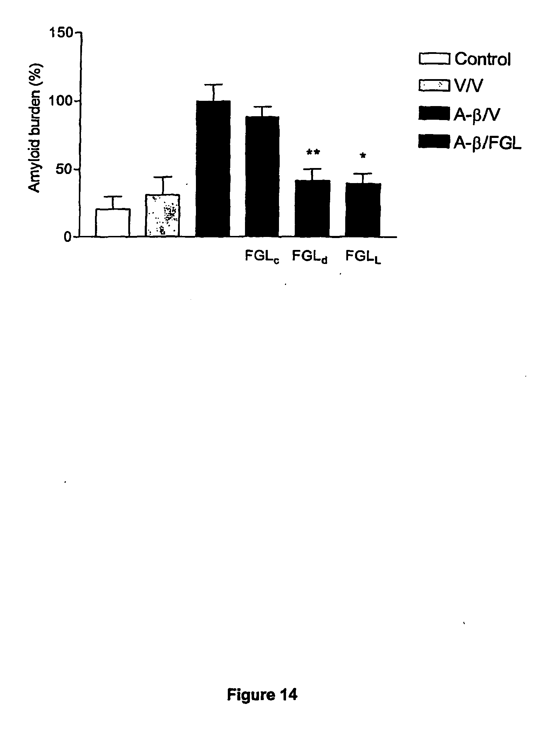

**Figure 14** shows the effect of early suboccipital administration of FGL_{L} and FGL_{d} on formation of amyloid burden in the cingulate cortex of rats in which cognitive impairment was induced by i.c.v. -injection of the (25-35) β-amyloid fragment (5.0 µg FGL per administration per rat) on days 7, 10 and 13 after (25-35) β-amyloid (A-β) fragment-induced neurotoxicity. The number of animals in the groups varied from 3 to 13. Results were analysed using one-way ANOVA (F(2,18)=5.2, p=0.016), and significance of results was contingent on achieving a p-value of less than 0.05 (*p<0.05 and **p<0.01), when compared with the A-β/V group (Newman-Keuls post-test). A statistically significant difference was observed between the V/V treated group and the A-β/V treated group (p<0.05). 'A-β' and 'V' are the abbreviations of the (25-35) β-amyloid fragment and vehicle, respectively.

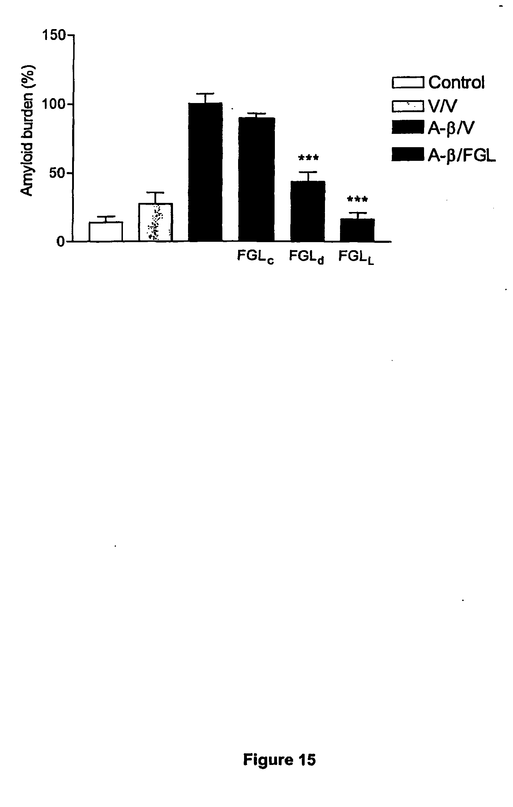

**Figure 15** shows the effect of early sub-occipital administration of FGL_{L} and FGL_{d} on formation of the amyloid burden in the CA3 area of hippocampus of rats in which cognitive impairment was induced by i.c.v. -injection of the (25-35) β-amyloid fragment (5.0 µg FGL per administration per rat) on days 7, 10 and 13 after (25-35) β-amyloid (A-β) fragment-induced neurotoxicity. The number of animals in the groups varied from 2 to 12. Results were analysed using one-way ANOVA (F(2,15)=9.54, p=0.002), and significance of results was contingent on achieving a p-value of less than 0.05 (***p<0.001), when compared with the A-β/V group (Newman-Keuls post-test). A statistically significant difference was observed between the VN treated group and the A-β/V treated group (p<0.01). 'A-β' and 'V' are the abbreviations of the (25-35) β-amyloid fragment and vehicle, respectively.

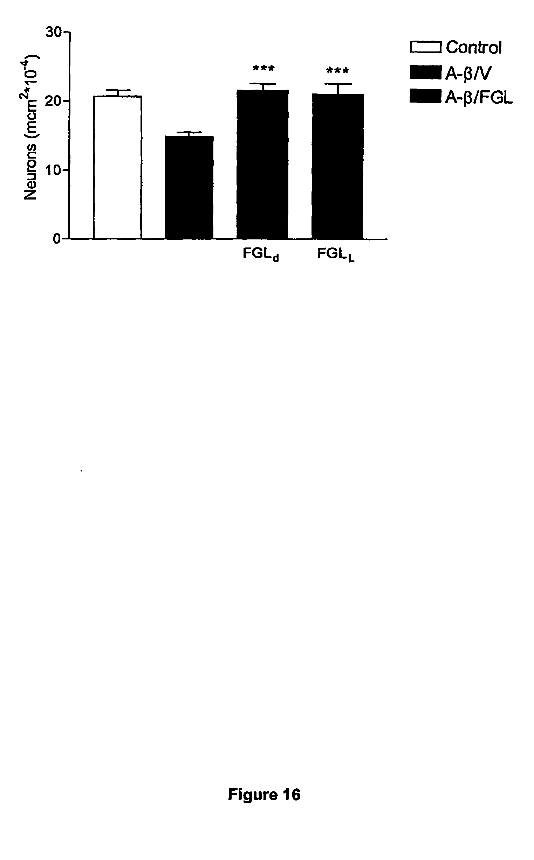

**Figure 16** shows the effect of early sub-occipital administration of FGL_{L} and FGL_{d} on neuronal death in the hippocampus of rats in which cognitive impairment was induced by i.c.v. -injection of the (25-35) β-amyloid fragment. The effect of 5.0 µg FGL per administration per rat, administered sub-occipitally on days 7, 10 and 13 after (25-35) β-amyloid (A-β) fragment-induced toxicity, on neuronal cell death in the CA3 zone of the rat hippocampus. The number of animals in the groups varied from 4 to 8. The results were analysed using one-way ANOVA (F(3,17)=13.76, p<0.001) and significance of results was contingent on achieving a p-value of less than 0.05 (***p<0.001), when compared with the A-β/V group (Newman-Keuls post-test). A statistically significant difference was observed between the Control group and the A-β/V treated group (p<0.001). 'A-β' and 'V' are the abbreviations of the (25-35) β-amyloid fragment and vehicle, respectively.

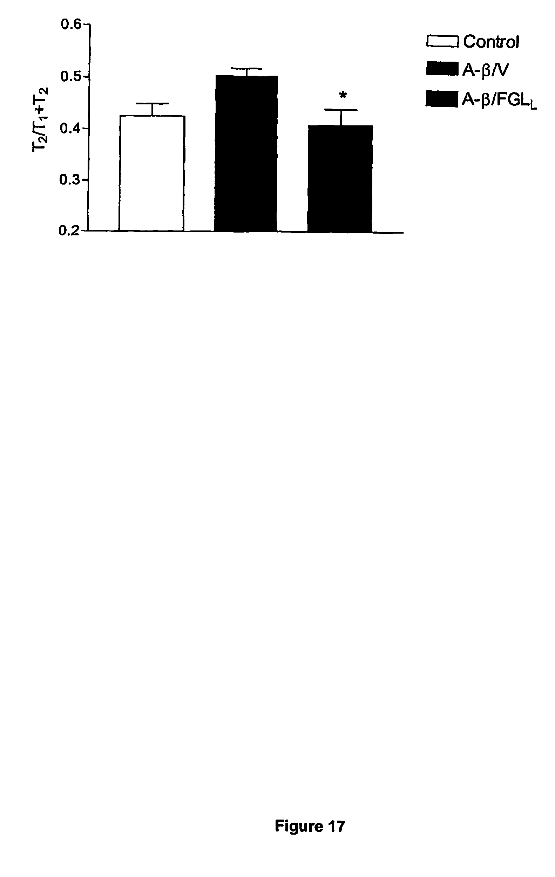

**Figure 17** demonstrates the effects of 5.0 µg FGL_{L} (per administration per rat) administered sub-occipitally on days 30, 33, and 36 after (25-35) β-amyloid fragment (A-β)-induced neurotoxicity in the Social Recognition test. The Social Recognition test was performed 44 days after i.c.v. administration of the A-β fragment and estimated as the ratio between the times the adult rat spent exploring a juvenile rat during a first (T₁) and a second (T₂) meeting. The number of animals per group varied from 4 to 5. The results were analysed using unpaired t-test and significance of results was contingent on achieving a p-value of less than 0.05 (*p<0.05), when compared to the A-β/V group. A significant impairment of short-term memory was induced by treatment with A-β (p<0.05, unpaired t-test between Control and A-β/V). 'A-β' and 'V' are the abbreviations of the (25-35) β-amyloid fragment and vehicle, respectively.

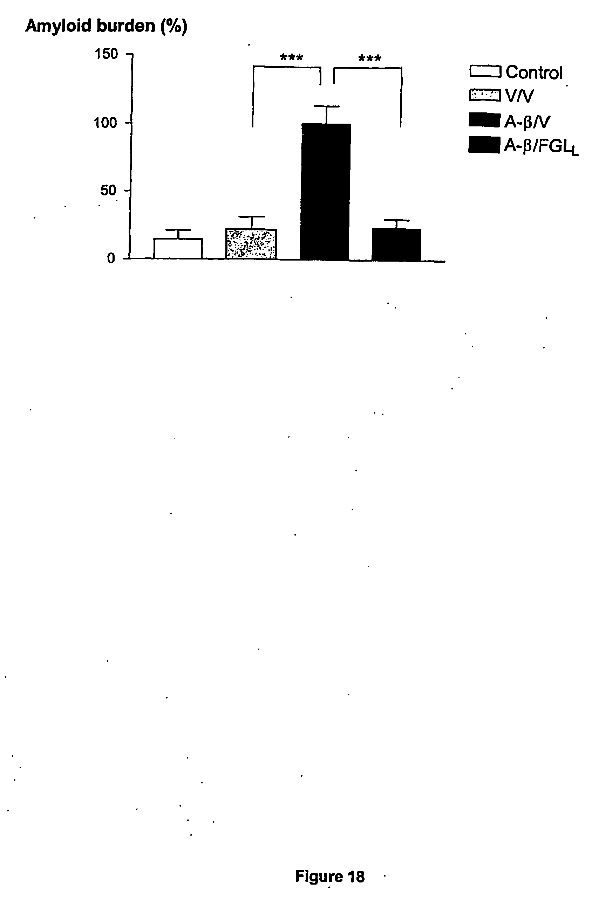

**Figure 18** shows the effects of 5.0 µg FGL_{L} (per administration per rat) administered sub-occipitally on days 30, 33 and 36 after A-β fragment-induced neurotoxicity on the amyloid burden in the cingulate cortex of rats. The number of animals per group varied from 3 to 13. The results were analysed using one-way ANOVA (F(2,12)=18.6, P=0.002) and significance of results was contingent on achieving a p-value of less than 0.05 (***p<0.001) when compared with the A-β/V group (Bonferroni's multiple comparison test). A statistically significant difference was observed between the V/V-treated group and the A-β/V-treated group (p<0.001). 'A-β' and 'V' are the abbreviations of the (25-35) β-amyloid fragment and vehicle, respectively.

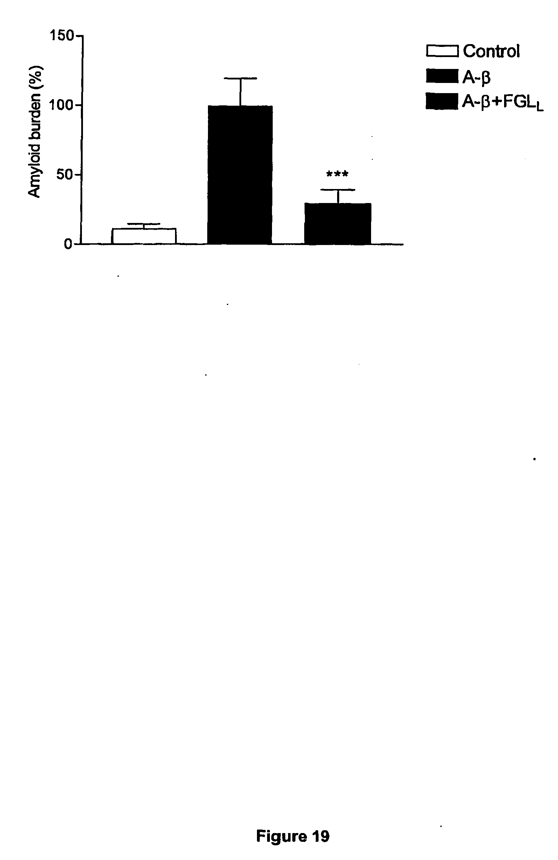

**Figure 19** shows the effects of 5.0 µg FGL_{L} (per administration per rat) administered sub-occipitally on days 30, 33 and 36 after (25-35) β-amyloid (A-β) fragment-induced neurotoxicity on the amyloid burden in the CA3 area of hippocampus of rats. The number of animals per group varied from 3 to 5. The results were analysed using one-way ANOVA (F(2,9)=10.6, P=0.004) and significance of results was contingent on achieving a p-value of less than 0.05 (***p<0.001) when compared with the A-β/V group (Newman-Keuls post-test). A statistically significant difference was observed between the Control-treated group and the A-β/V-treated group (p<0.01). 'A-β' and 'V' are the abbreviations of the (25-35) β-amyloid fragment and vehicle, respectively.

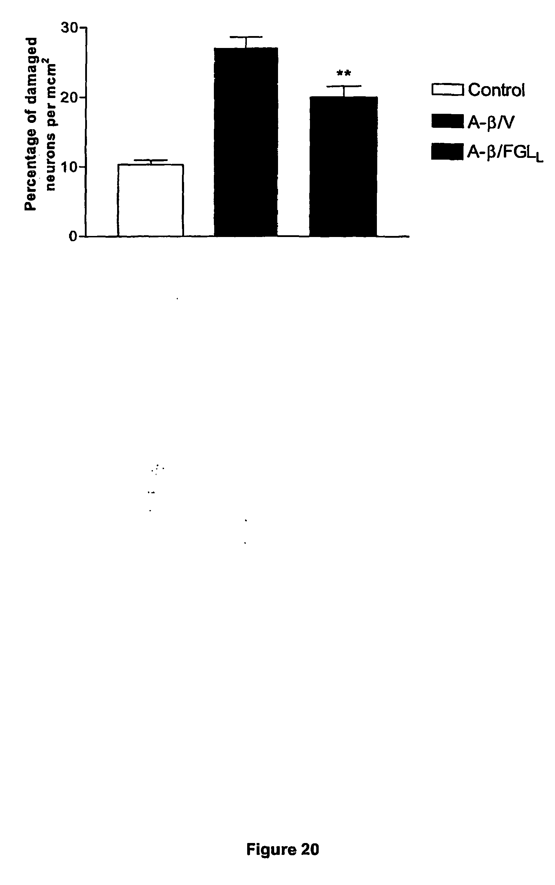

**Figure 20** shows the effect of 5.0 µg FGL_{L} (per administration per rat) administered sub-occipitally on days 7, 10 and 13 after (25-35) β-amyloid (A-β) fragment-induced toxicity on neuronal cell death in the cingulate cortex. The number of animals in the groups varied from 4 to 8. The results were analysed using one-way ANOVA (F(2,9)=26.9; p<0.001, Newmann-Keuls post-test: A-β/V compared with the control group (***p<0.001) and compared with the A-β/FGL_{L} treated group (**p<0.01). Significance of results was contingent on achieving a p-value of less than 0.05, when compared with the A-β/V group (Newman-Keuls post-test). A statistically significant difference was observed between the Control-treated group and the A-β/V-treated group (p<0.001). 'A-β' and 'V' are the abbreviations of the (25-35) β-amyloid fragment and vehicle, respectively.

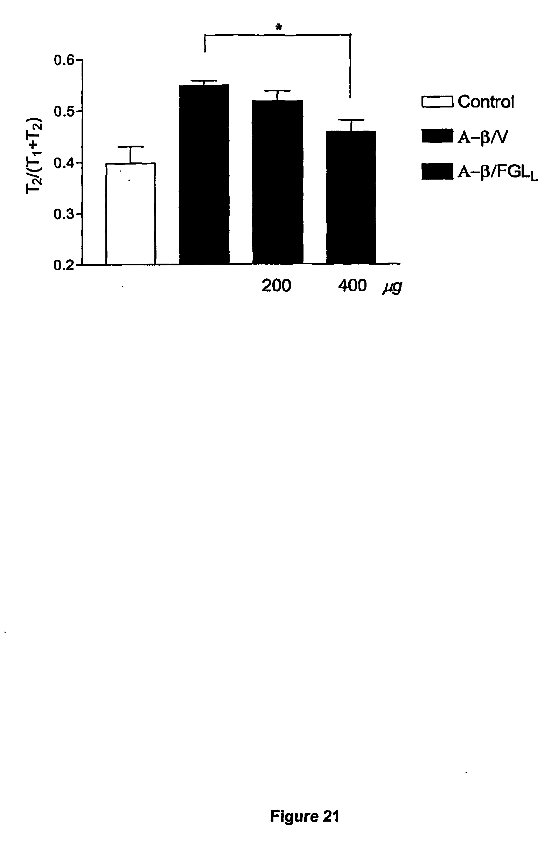

**Figure 21** shows the effect of three intranasal administrations of vehicle, 200 µg FGL_{L}, and 400 µg FGL_{L} per rat, at days 7, 10 and 13 after i.c.v. administration of the (25-35) β-amyloid (A-β) fragment in the Social Recognition test. The social recognition test was performed 21 days after i.c.v. administration of the (25-35) β-amyloid (β-A) fragment and estimated as the ratio between the times the adult rat spent on exploration of a juvenile rat during a first (T₁) and a second (T₂) meeting. Number of animals in the groups varied from 3 to 8. Results are shown as means ± SEM and analysed by unpaired t-test (p<0.001 of control vs A-β/V) and one-way ANOVA (F(2,19)=5.6; p=0.01; Newmann-Keuls post-test, p<0.05 of A-β/V vs A-β/FGL_{L,400}; and p>0.05 of A-β/V vs A-β/FGL_{L,200}; whereas p<0.05 of A-β/FGL_{L,400} vs A-β/FGL_{L,200}). Significance of results was contingent on achieving a p-value of less than 0.05 (*p<0.05) when compared to the A-β/V treated group. 'A-β' and 'V' are the abbreviations of the (25-35) β-amyloid fragment and vehicle, respectively.

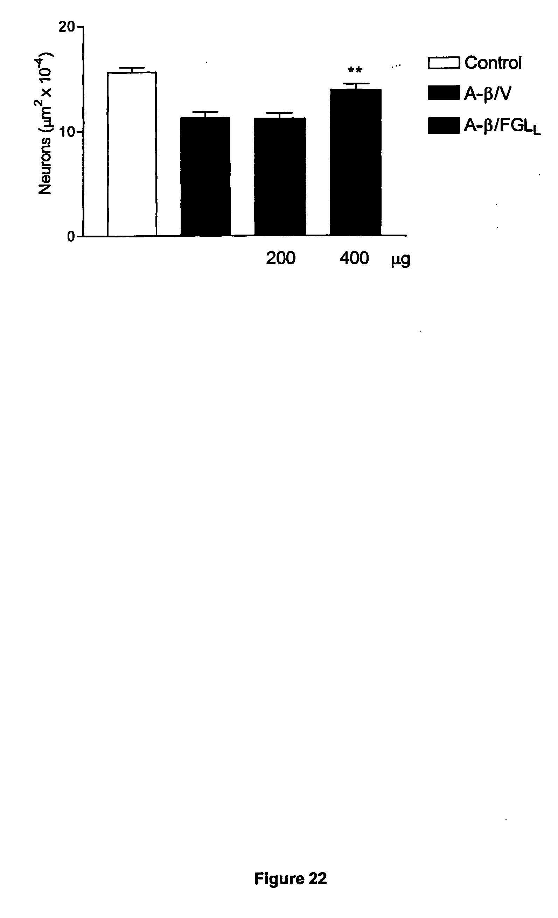

**Figure 22** shows the effect of three intranasal administrations of vehicle, 200 µg FGL_{L}, and 400 µg FGL_{L} per rat, at days 7, 10 and 13 after i.c.v. administration of the (25-35) β-amyloid (β-A) on neuronal cell death in the cingulate cortex. The number of animals in the groups varied from 4 to 8. The results were analysed using unpaired t-test (of the control group versus the A-β/V-treated group (p<0.001) and one-way ANOVA (F(2,16)=8.6; p=0.003), with Newman-Keuls post-test **p<0.01 of A-β/V vs A-β/FGL_{L,400} and p>0.05 of A-β/V vs. A-β/FGL_{L200}). Significance of results was contingent on achieving a p-value of less than 0.05 (**p<0.01), when compared with the A-β/V treated group. 'A-β' and 'V' are the abbreviations of the (25-35) β-amyloid fragment and vehicle, respectively.

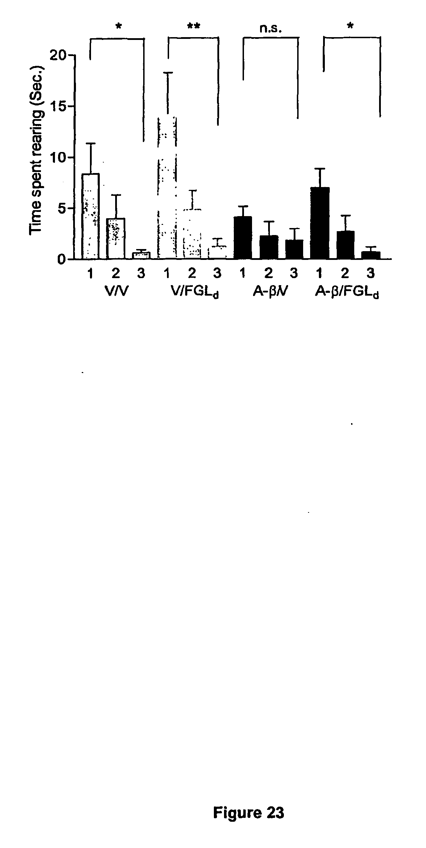

**Figure 23** shows the effect of 5.9 µg FGL_{d} administration on rats tested for the Rearing Activity in the Open Field test. The peptide was applied sub-occipitally at days 7, 10, and 13 after the (25-35) β-amyloid fragment induced neurotoxicity. The numbers '1', '2', and '3' indicate the periods of measurement: '1' denotes 1-3 min., '2' denotes 8-10 min., and '3' denotes 18-20 min. 'V/V' indicates that the rats received vehicle as replacement for the (25-35) β-amyloid fragment and FGL_{d}, The number of animals per group varied from 9 to 11. 'A-β' and 'V' are the abbreviations of the (25-35) β-amyloid fragment and vehicle, respectively. The results were analysed using unpaired t-test and significance of results was contingent on achieving a p-value of less than 0.05 (*p<0.05, **p<0.01), when compared with the first measurement period (1-3 min.).

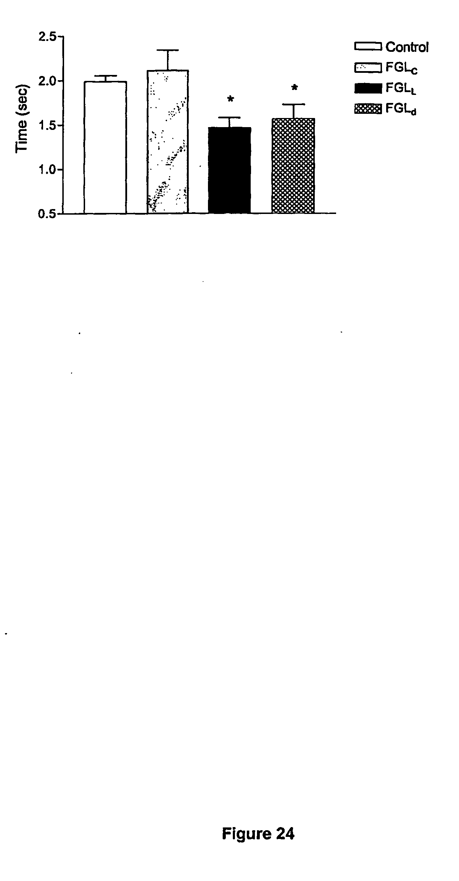

**Figure 24** shows the results of performance of the Surface Righting Reflex test by rat pups at PND4 after intranasal administration of 2.6 µg FGL_{L}, FGL_{d}, or FGL_{control} per pup at PND 1, 2, and 3. The number of litters of each treatment group was six. The results were analysed using one-way ANOVA (F=4.05; P=_{0.039}), and the Newman-Keuls test showed a statistically significant effect of FGL_{L} (P<0.05) and FGL_{d} (P<0.05) when compared to the control- and FGL_{control} groups. FGL_{control} as above.

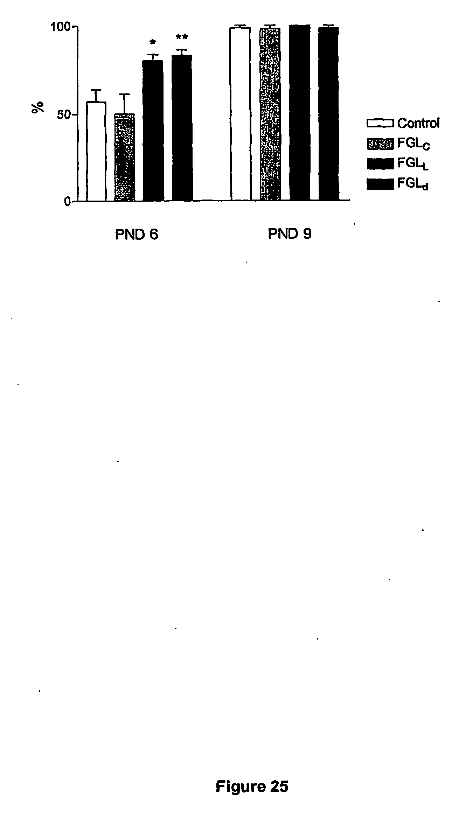

**Figure 25** shows the results of performance the Negative Geotaxis Reflex test by rat pups at PND 6 and PND 9 after intranasal administration of 2.6 µg FGL_{L}, FGL_{d}, or FGl_{control} per pup at PND 1, 2, and 3. The number of litters of each treatment group was six. The results were analysed using one-way ANOVA (F_{PND6}=5.14; P_{PND6}=0.008). At PND 6, statistically significant effects were observed for the FGL_{L} (*p<0.05) and FGL_{d} (**p<0.01) groups, when compared to the control- and FGL_{c} (FGLcontrol) groups. At PND 9, no significant effect of FGL was observed (One-way ANOVA: F_{PND9}=0.94; P_{PND9}=0.44). FGL_{control} as above.

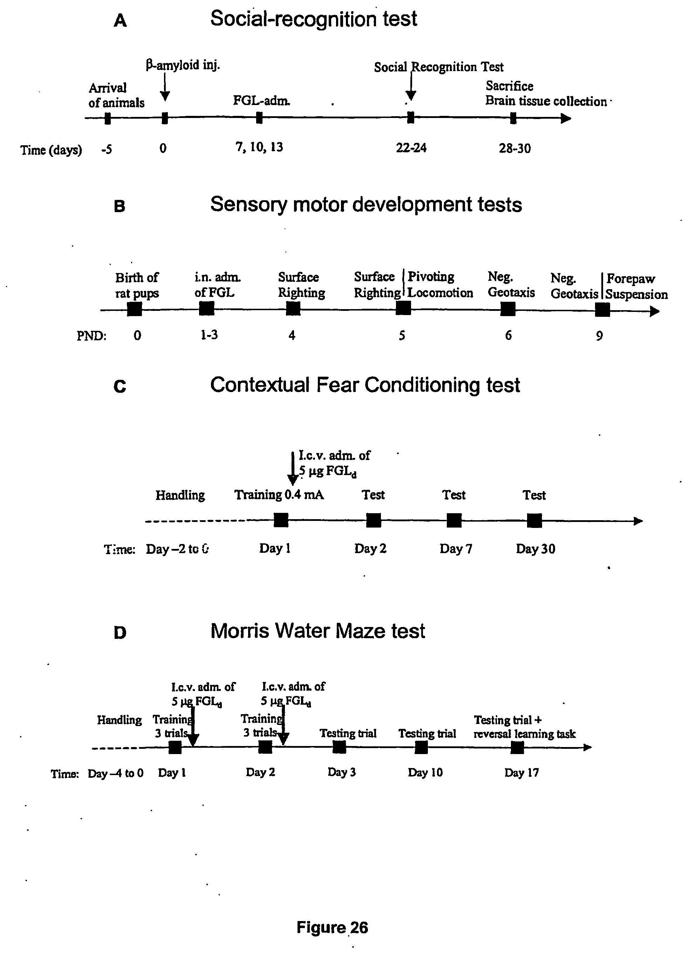

**Figure 26** shows the schematic presentation of the time course of in vivo studies.

### Detailed description of the invention

### 1. Compound

Compounds capable of modulating the function of fibroblast growth factor receptor (FGFR) are of greatest importance in view of development of effective drugs for therapeutic treatment of a variety of diseases and pathologic conditions. Thus, it is an objection of the present invention to provide novel compounds capable of binding to FGFR and modulating the FGFR activity. According to the invention the compounds comprise at least two peptide sequences, wherein at least one of the two sequences comprises a structural motif common for the amino acid sequences capable of low affinity binding to FGFR.

### 1. Peptide sequences

Thus, in one aspect the invention relates to two individual peptide sequences, wherein said two individual peptide sequences comprises an amino acid sequence of the formula

L1-A-L2-B-L3-C-L4-D-L5

wherein
one of A, B, C, D is selected from a hydrophobic amino acid residue,
one of A, B, C, D is selected from a basic amino acid residue, Asn or Gln;
one of A, B, C, D is selected from an acidic amino acid residue, Asn or Gln,
one of A, B, C, D is Gly or Ala, and
L1, L2, L3, L4 and L5 is selected from a chemical bond or an amino acid sequence having n amino acid residues, wherein n is an integer of from 0 to 5, wherein said two peptide sequences comprise the identical amino acid sequence, said amino acid sequence being EVYVVAENQQGKSKA (SEQ ID NO:1).

In the present context the standard one-letter code for amino acid residues as well as the standard three-letter code are applied. Abbreviations for amino acids are in accordance with the recommendations in the IUPAC-IUB Joint Commission on Biochemical Nomenclature Eur. J. Biochem, 1984, vol. 184, pp 9-37. Throughout the description and claims either the three letter code or the one letter code for natural amino acids are used. Where the L or D form has not been specified it is to be understood that the amino acid in question has the natural L form, cf. Pure & Appl. Chem. Vol. (56(5) pp 595-624 (1984) or the D form, so that the peptides formed may be constituted of amino acids of L form, D form, or a sequence of mixed L forms and D forms.

Where nothing is specified it is to be understood that the C-terminal amino acid of a peptide of the invention exists as the free carboxylic acid, this may also be specified as "-OH". However, the C-terminal amino acid of a compound of the invention may be the amidated derivative, which is indicated as "-NH₂". Where nothing else is stated the N-terminal amino acid of a polypeptide comprise a free amino-group, this may also be specified as "H-".

Where nothing else is specified amino acid can be selected from any amino acid, whether naturally occurring or not, such as alpha-amino acids, beta-amino acids, and/or gamma amino acids. Accordingly, the group comprises but are not limited to: Ala, Val, Leu, Ile, Pro, Phe, Trp, Met, Gly, Ser, Thr, Cys, Tyr, Asn, Gin, Asp, Glu, Lys, Arg, His Aib, Nal, Sar, Om, Lysine analogues, DAP, DAPA and 4Hyp.

Also, according to the invention modifications of the compounds/peptides may be performed, such as for example glycosylation and/or acetylation of the amino acids.

Basic amino acid residues are according to invention represented by the residues of amino acids Arg, Lys, and His, acidic amino acid residues - by the residues of amino acids Glu and Asp, and hydrophobic amino acid residues by the residues of amino acids Leu, Ile, Val, Phe, Trp, and Tyr. In a preferred embodiment a basic amino acid residue is represented by Arg or Lys.

### Origin of the peptide sequence

By the term "functional homologue" of a predetermined polypeptide is in the present context meant a molecule which is capable of one or more biological functions of the predetermined polypeptide, in a preferred embodiment the biological function(s), which is executed through the mechanism of binding and activating FGFR .

As it has been discussed above, a preferred functional cell-surface receptor of the invention is a receptor selected from the family of fibroblast growth factor receptors (FGFRs). The above molecules according to the invention comprise an alternative low affinity binding site for FGFR, which is different from the known FGFR high affinity binding site of FGFs.

A low affinity FGFR binding site of the above molecules is characterised in that it comprises the amino acid sequence EVYWAENQQGKSKA (SEQ ID NO 1), The sequence EVYVVAENQQGKSKA is derived from the neural cell adhesion molecule (NCAM) and known in the prior art as the FGL peptide of NCAM. The homology of one amino acid sequence with another amino acid sequence is defined as a percentage of identical amino acids in the two collated sequences. The homology between amino acid sequences may be calculated using well known algorithms such as BLOSUM 30, BLOSUM 40, BLOSUM 45, BLOSUM 50, BLOSUM 55, BLOSUM 60, BLOSUM 62, BLOSUM 65, BLOSUM 70, BLOSUM 75, BLOSUM 80, BLOSUM 85, or BLOSUM 90.

In still another embodiment an FGFR binding site of a new low affinity binding ligand of FGFR may comprise a fragment or a homologue of the sequence, which
i) has at least 60 %, more preferably at least 70%, more preferably at least 80%, more preferably at least 90%, more preferably 95% homology to the sequence EVYVVAENQQGKSKA (SEQ ID NO 1), or
ii) has at least 60 %, more preferably at least 70%, more preferably at least 80%, more preferably at least 90%, more preferably 95% positive amino acid matches compared to to the sequence EVYVVAENQQGKSKA (SEQ ID NO 1).

In still yet another embodiment the FGFR binding site of a new low affinity FGFR ligand of the invention may be characterised by the specific 3D feature, namely, it may essentially consists of one or more "strand-loop-strand" structural motifs. It is a preferred feature of the sequences discussed above. According to the invention the strand-loop-strand structure is a preferred 3D structure of an amino acid sequence of the formula

L1-A-L2-B-L3-C-L4-D-L5.

wherein
one of A, B, C, D is selected from a hydrophobic amino acid residue,
one of A, B, C, D is selected from a basic amino acid residue, Asn or Gln,
one of A, B, C, D is selected from an acidic amino acid residue, Asn or Gln,
one of A, B, C, D is Gly or Ala, and
L1, L2, L3, L4 and L5 is selected from a chemical bond or an amino acid sequence having n amino acid residues, wherein n is an integer of from 0 to 5, wherein said amino acid sequence is EVYVVAENQQGKSKA (SEQ ID NO:1).

According to the invention at least one of the two individual amino acid sequences preferably comprises at least one of the above identified structural motifs and is preferably derived from any of the above molecules. Such sequence according to the invention may be selected from any of the sequences identified as SEQ ID NOs: 1-146. In some embodiments one of the two individual sequences may be selected from one group of sequences of the groups 1 to 12 as identified below, and another of the two individual sequences may be selected from another group of sequences. In other embodiments the sequences may be selected from the same group of sequences.

The present invention also relates to fragments of the above peptide sequences having at least 40%, more preferably at least 50%, more preferably at least 60%, more preferably at least 70%, more preferably at least 80%, more preferably at least 90%, more preferably at least 95% of the length of the predetermined sequence set forth in SEQ ID NO: 1 wherein an amino acid sequence homology between a fragment and the predetermined sequence is 100%. The invention concerns the fragments, which remain a capability of the predetermined sequences to interact with the cell-surface receptor defined below.

In one preferred embodiment of the invention, the selected sequence is EVYVVAENQQGKSKA (SEQ ID NO: 1).

In one preferred embodiment the compound comprises two identical individual peptide sequences, wherein the sequence is EVYVVAENQQGKSKA (SEQ ID NO: 1). Other preferred embodiments encompass compounds comprising two individual amino acid sequences comprising fragments, of SEQ ID NO: 1 wherein said fragments, being as defined above.

In one embodiment at least one of the two individual amino sequences of the compound comprise, essentially comprise, or consist of two or more contiguous peptide sequences of SEQ ID NOS: 1-146 co-joined by peptide bonds in one peptide chain. In other embodiments, the co-joined peptide sequences may be identical, such as for example SEQ ID NO: 1 repeated in one peptide chain 2 to 5 times, forming thereby an oligomer (multimer) consisting of identical monomers. According to the invention, the oligomer (multimer) may consist of 2, 3, 4, 5, 6, 7, 8, 9 or 10 repeated sequences (monomers).

A compound consisting of two individual peptide sequences connected to each other through a linker, wherein each of said individual peptide sequences is the sequence of SEQ ID NO:1, Orientation of the individual peptide sequences in a compound may be either N to C, C to N or mixed. The wording "N to C orientation" means that two individual sequences are connected to a linker through their N-terminal amino acid residue, such as a compound of the structure (COOH)peptide sequence(NH2)-linker-(NH2)peptide sequence(COOH). The wording "C to N orientation"means that two predetermined sequences are connected to a linker through their C-terminal amino acid residue, such as a compound of the structure (NH2)peptide sequence(COOH)-linker-(COOH)peptide sequence(NH2). The term "mixed orientation" is meant that two predetermined sequences are connected to a linker either through their C-terminal amino acid residue or N-terminal, such as one peptide sequence though its N-terminal amino acid residue and another through its C-terminal amino acid residue, for example as a compound of the type:(NH2)peptide sequence(COOH)-linker-(NH2)peptide sequence(COOH).

### 1.3 Linker

According to the invention two individual peptide sequences of a compound are co-joined by a linker. A linker molecule is according to the invention is selected from achiral di-, tri- or tetracarboxylic acids, said acids having the general formula

X[(A)nCOOH][(B)mCOOH]

wherein n and m independently are an integer of from 1 to 20, X is HN, A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety. In another embodiment suitable achiral di-, tri- or tetracarboxylic acids to be used in the present method have the general formula

X[(A)nCOOH][(B)mCOOH]

wherein n and m are 0 or an integer of from 1 to 20, X is H₂N(CR₂)pCR, or RHN(CR₂)pCR, wherein p Is 0 or integer of from 1 to 20, wherein each R is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety. In still another embodiment suitable achiral di-, tri- or tetracarboxylic acids to be used in the present method have the general formula

X[(A)nCOOH][(B)mCOOH]

wherein n and m are 0 or an integer of from 1 to 20, X is HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, or [HOOC(A)n][HOOC(B)m]CR(CR₂)pCR, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety. In yet another embodiment suitable achiral di-, tri- or tetracarboxylic acids to be used in the present method have the general formula

X[(A)nCOOH][(B)mCOOH]

Wherein n and m are 0 or an integer of from 1 to 20, X is H₂N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, or [HOOC(A)n][HOOC(B)m](CR₂)p, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

Under the term C₁₋₁₀ alkyl is meant straight or branched chain alkyl groups having 1-10 carbon atoms, e.g. methyl, ethyl, isopropyl, butyl, and tertbutyl.

Under the term C₂₋₁₀ alkenyl is meant straight or branched chain alkenyl groups having 2-10 carbon atoms, e.g. ethynyl, propenyl, isopropenyl, butenyl, and tert-butenyl.

Under the term cyclic moiety is meant cyclohexan, and cyclopentane.

Under the term aromatic moiety is meant phenyl.

The wording "A and B forms a cyclic, heterocyclic or aromatic moiety" denotes cyclohexan, piperidine, benzene, and pyridine.

According to the invention two peptide fragments comprising the identical amino acid sequences according to the invention are connected to each other so that one of these two peptide sequences is covalently bound to one of two carboxylic group of a linker molecule selected from the molecules defined above and another of the peptide sequences is covalently bound to another carboxylic group of said linker molecule. The peptide sequences are covalently bound to the linker through their amino- or carboxy-groups of the N- or C terminal amino acid residue, respectively. Accordingly, a compound of the invention has the formula

COOH/CONH2-peptide sequence-NH-CO-linker-CO-NH-peptide sequence-COOH/CONH2

or NH2-peptide sequence-CO-NH-linker-NH-CO-peptide sequence-NH2.

### 2. Production of the compound

### 2.1 Production of individual peptide sequences

The peptide sequences of the present invention may be prepared by any conventional synthetic methods, recombinant DNA technologies, enzymatic cleavage of full-length proteins which the peptide sequences are derived from, or a combination of said methods.

### Recombinant preparation

Thus, in one embodiment the peptides of the invention are produced by use of recombinant DNA technologies.

The DNA sequence encoding a peptide or the corresponding full-length protein the peptide originates from may be prepared synthetically by established standard methods, e.g. the phosphoamidine method described by Beaucage and Caruthers, 1981, Tetrahedron Lett. 22:1859-1869, or the method described by Matthes et al., 1984, EMBO J. 3:801-805. According to the phosphoamidine method, oligonucleotides are synthesised, e.g. in an automatic DNA synthesiser, purified, annealed, ligated and cloned in suitable vectors.

The DNA sequence encoding a peptide may also be prepared by fragmentation of the DNA sequences encoding the corresponding full-length protein of peptide origin, using DNAase I according to a standard protocol (Sambrook et al., Molecular cloning: A Laboratory manual. 2 rd ed., CSHL Press, Cold Spring Harbor, NY, 1989). The present invention relates to full-length proteins selected from the groups of proteins identified above. The DNA encoding the full-length proteins of the invention may altematively be fragmented using specific restriction endonucleases. The fragments of DNA are further purified using standard procedures described in Sambrook et al., Molecular cloning: A Laboratory manual. 2 rd ed., CSHL Press, Cold Spring Harbor, NY,1989.

The DNA sequence encoding a full-length protein may also be of genomic or cDNA origin, for instance obtained by preparing a genomic or cDNA library and screening for DNA sequences coding for all or part of the full-length protein by hybridisation using synthetic oligonucleotide probes in accordance with standard techniques (cf. Sambrook et al., Molecular Cloning: A Laboratory Manual, 2nd Ed., Cold Spring Harbor, 1989). The DNA sequence may also be prepared by polymerase chain reaction using specific primers, for instance as described in US 4,683,202 or Saiki et al., 1988, Science 239:487-491.

The DNA sequence is then inserted into a recombinant expression vector, which may be any vector, which may conveniently be subjected to recombinant DNA procedures. The choice of vector will often depend on the host cell into which it is to be introduced. Thus, the vector may be an autonomously replicating vector, i.e. a vector that exists as an extrachromosomal entity, the replication of which is independent of chrcmosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding a peptides or a full-length protein should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the coding DNA sequence in mammalian cells are the SV 40 promoter (Subramani et al., 1981, Mol. Cell Biol. 1:854-864), the MT-1 (metallothionein gene) promoter (Palmiter et al., 1983, Science 222: 809-814) or the adenovirus 2 major late promoter. A suitable promoter for use in insect cells is the polyhedrin promoter (Vasuvedan et al., 1992, FEBS Lett. 311:7-11). Suitable promoters for use in yeast host cells include promoters from yeast glycolytic genes (Hitzeman et al., 1980, J. Biol. Chem. 255:12073-12080; Alber and Kawasaki, 1982, J. Mol. Appl. Gen. 1: 419-434) or alcohol dehydrogenase genes (Young et al., 1982, in Genetic Engineering of Microorganisms for Chemicals, Hollaender et al, eds., Plenum Press, New York), or the TPI1 (US 4,599,311) or ADH2-4c (Russell et al., 1983, Nature 304:652-654) promoters. Suitable promoters for use in filamentous fungus host cells are, for instance, the ADH3 promoter (McKnight et al., 1985, EMBO J. 4:2093-2099) or the tpiA promoter.

The coding DNA sequence may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector may further comprise a DNA sequence enabling the vector to replicate in the host cell in question. An example of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hydromycin or methotrexate.

The procedures used to ligate the DNA sequences coding the peptides or full-length proteins, the promoter and the terminator, respectively, and to insert them into suitable vectors containing the information necessary for replication, are well known to persons skilled in the art (cf., for instance, Sambrook et al., op.cit.).

To obtain recombinant peptides of the invention the coding DNA sequences may be usefully fused with a second peptide coding sequence and a protease cleavage site coding sequence, giving a DNA construct encoding the fusion protein, wherein the protease cleavage site coding sequence positioned between the HBP fragment and second peptide coding DNA, inserted into a recombinant expression vector, and expressed in recombinant host cells. In one embodiment, said second peptide selected from, but not limited by the group comprising glutathion-S-reductase, calf thymosin, bacterial thioredoxin or human ubiquitin natural or synthetic variants, or peptides thereof. In another embodiment, a peptide sequence comprising a protease cleavage site may be the Factor Xa, with the amino acid sequence *IEGR,* enterokinase, with the amino acid sequence *DDDDK,* thrombin, with the amino acid sequence *LVPR*/*GS,* or *Acharombacter lyticus,* with the amino acid sequence *XKX*, cleavage site.

The host cell into which the expression vector is introduced may be any cell which is capable of expression of the peptides or full-length proteins, and is preferably a eukaryotic cell, such as invertebrate (insect) cells or vertebrate cells, e.g. *Xenopus laevis* oocytes or mammalian cells, in particular insect and mammalian cells. Examples of suitable mammalian cell lines are the HEK293 (ATCC CRL-1573), COS (ATCC CRL-1650), BHK (ATCC CRL-1632, ATCC CCL-10) or CHO (ATCC CCL-61) cell lines. Methods of transfecting mammalian cells and expressing DNA sequences introduced in the cells are described in e.g. Kaufman and Sharp, J. Mol. Biol. 159, 1982, pp. 601-621; Southern and Berg, 1982, J. Mol. Appl. Genet. 1:327-341; Loyter et al., 1982, Proc. Natl. Acad. Sci. USA 79: 422-426; Wigler et al., 1978, Cell 14:725; Corsaro and Pearson, 1981, in Somatic Cell Genetics 7, p. 603; Graham and van der Eb, 1973, Virol. 52:456; and Neumann et al., 1982, EMBO J. 1:841-845.

Alternatively, fungal cells (including yeast cells) may be used as host cells. Examples of suitable yeast cells include cells of *Saccharomyces spp.* or *Schizosaccharomyces spp.,* in particular strains of *Saccharomyces cerevisiae.* Examples of other fungal cells are cells of filamentous fungi, e.g. *Aspergillus spp.* or *Neurospora spp.,* in particular strains of *Aspergillus oryzae* or *Aspergillus niger.* The use of *Aspergillus spp.* for the expression of proteins is described in, e.g., EP 238 023.

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g. in catalogues of the American Type Culture Collection).

The peptides or full-length proteins recombinantly produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. HPLC, ion exchange chromatography, affinity chromatography, or the like.

### Synthetic preparation

The methods for synthetic production of peptides are well known in the art. Detailed descriptions as well as practical advice for producing synthetic peptides may be found in Synthetic Peptides: A User's Guide (Advances in Molecular Biology), Grant G. A. ed., Oxford University Press, 2002, or in: Pharmaceutical Formulation: Development of Peptides and Proteins, Frokjaer and Hovgaard eds., Taylor and Francis, 1999.

Peptides may for example be synthesised by using Fmoc chemistry and with Acm-protected cysteins. After purification by reversed phase HPLC, peptides may be further processed to obtain for example cyclic or C- or N-terminal modified isoforms. The methods for cyclization and terminal modification are well-known in the art and described in detail in the above-cited manuals.

In a preferred embodiment the peptide sequences of the invention are produced synthetically, in particular, by the Sequence Assisted Peptide Synthesis (SAPS) method.

### Sequence Assisted Peptide Synthesis (SAPS)

Peptides may be synthesised either batchwise in a polyethylene vessel equipped with a polypropylene filter for filtration or in the continuous flow version of the polyamide solid-phase method (Dryland, A. and Sheppard, R.C., (1986) J.Chem. Soc. Perkin Trans. I, 125 - 137.) on a fully automated peptide synthesiser using 9-fluorenylmethyloxycarbonyl (Fmoc) or tert. -Butyloxycarbonyl, (Boc) as N-α-amino protecting group and suitable common protection groups for side-chain functionality's.

The following is a list of chemicals and a description of the procedure that may be helpful when using SAPS for the synthesis of the peptide fragments of the invention.

### Chemicals and procedures

### 1. Solvents

DMF (N,N-dimethylformamide, Riedelde-Haen, Germany) (may be purified by passing through a column packed with a strong cation exchange resin, for example Lewatit S 100 MB/H strong acid, Bayer AG Leverkusen, Germany, and analysed for free amines prior to use by addition of 3,4-dihydro-3-hydroxy-4-oxo-1,2,3-benzotriazine (Dhbt-Oli) giving rise to a yellow colour (Dhbt-O- anion) if free amines are present

DCM (dichloromethane, analytical grade, Riedelde-Maen, Germany) may be used directly without purification.

### 2. Amino acids:

Fmoc-protected amino acids and corresponding pentafluorophenyl (Pfp) esters may be purchased from MilliGen, UK, NovaBiochem, Switzerland, and Bachem, Switzerland, and the Dhbt-esters from NovaBiochem, Switzerland in suitable side-chain protected forms.

Bocprotected amino acids may be purchased from Bachem, Switzerland.

### 3. Coupling reagents

Diisopropylcarbodiimide (DIC) may be purchased from Riedelde-Hen, Germany and distilled prior to use.

Dicyclohexylcarbodiimide (DCC) may be purchased from Merck-Schuchardt, Niinchen, Germany, and purified by destillation.O-Benzotriazolyl-N,N,N",N"- tetramethyluronium tetrafluoroborate (TBTU) (PerSeptive Biosystems Gmbtl Hamburg, Germany).

### 4. Linkers

HMPA, Novabiochem, Switzerland;
4-hydroxymethylbenzoic acid, Novabiochem;
4-methoxymandelic acid, Aldrich, Germany;
HMPB, Novabiochem; AM, Novabiochem;
3-(4-hydroxymethylphenoxy) propionic acid, Novabiochem, to be coupled to the resin as a preformed I-hydroxybenzotriazole (HObt) ester generated by means of
DIC.
Racemic 4-methoxymandelic acid (98% pure, Aldrich, Germany) may be used directty as linker or resolved by treatment with (+)-cinchonine (85% pure, Aldrich, Germany) giving the optical active linker (+)-4-methoxymandelic acid, Iax20 =+ 146 (water) in 95.8 % optical purity and (-)-4-methoxymandelic acid, [al20 =- 128.6 (water) in 88.1 % optical purity. Resolution of (+/-)-4-methoxvmandelic acid (A. Mc Kenzie,D.J.C. Pirie, (1936), Berichte 69, 868; E. Knorr, (1904), Berichte 37, 3172). (+/-)-4-Methoxymandelic acid (10 g, 54.89 mmol; Aldrich, 98%) is dissolved in 500 ml hot water (60-800C) and the solution decanted while still warm in order to remove insoluble impurities. (+)-Cinchonine (16,16 g, 54.89 mmol, Aldrich, 85%, [alD20 = + 211 (litt.: + 228 0)) is added to the hot solution in small portions. The solution became clear after 15 min stirring at 60-800C and is cooled in ice. After 1 h the precipitate is collected by filtration and dried in an exsiccator over night, yielding 9.9 g of the chinconine salt. The salt is recrystallized from boiling water (80 ml); the solution decanted while still warm and then cooled in ice. The precipitate is collected by filtration after 1h, washed three times with cold water, and dried in an exsiccator over night yielding 7.84 g (16.45 mmol). The chinconine salt (2 g, 4,2 mmol) is dissolved in 40 ml 2NHC1 and immediately extracted with 3 x 30 ml diethylether. The ether phase was dried over Na2SO4 and evaporated to dryness yielding 0.55 g 4-methoxymandelic acid. The optical purity of the liberated 4methoxymandelic acid was estimated to 18.5% ([a]D20 =+ 270) . After a second recrystallisation of the chinconine salt followed by liberation of the mandelic acid as described above the optical purity was estimated to 69.0 % ([a]D20 =+ 100.8 0). A third recrystallisation resulted in an optical purity of 95,8% ([a]D20 = 4140.0 0).

### 5. Solid supports

Peptides produced according to the Fmoc-strategy are synthesized on three different types of solid support using 0.05 M or higher concentrations of Fmoc-protected activated amino acid in DMF.
1) PEG-PS (polyethyleneglycol grafted on polystyrene;TentaGel S NH2 resin, 0.27 mmol/g, Rapp Polymere, Germany or NovaSyn TG resin, 0.29 mmol/g, Novabiochem, Switzerland) ;
2) PepSyn Gel (polydimethylacrylamide resin functionalized with sarcosine methylester, 1.0 mmol/g; MilliGen, UK).
3) PepSyn K (Kieselguhr supported polydimethylacrylamide resin functionalized with sarcosine methylester 0.11 mmol/g; MilliGen, UK).

Peptides synthesised according to the Boc-strategy may be synthesised on a Merrifield-resin (polystyrenedivinylbenzene) with the first amino acid attached (Novabiochem, Switzerland).

### 6. Catalysts and other reagents

Diisopropylethylamine (DIEA) may be purchased from Aldrich, Germany,
Ethylenediamine from Fluka, Switzerland,
Piperidine from Riedel-deWhen, Frankfurt, Germany.
4-(N,N-dimethylamino)pyridine (DMAP) may be purchased from Fluka, Switzerland and used as a catalyst in coupling reactions involving symmetrical anhydrides.
3,4-dihydro-3-hydroxy-4-oxo-1,2,3-benzo- triazine (Dhbt-OH) may be obtained from Fluka, Switzerland,
I-hydroxybenzotriazole (HObt) from NovaBiochem, Switzerland.

### 7. Coupling procedures

The first amino acid is coupled as a symmetrical anhydride in DMF generated from the appropriate N-cc-protected amino acid and DIC. The following amino acids are coupled as Pfp- or Dhbt-esters or as preformed HObt esters made from appropriate N-protected amino acids and HObt by means of DIC or TBTU in DMF. In the case of Fmoc all acylations are checked by the ninhydrin test performed at 800C in order to prevent Fmoc deprotection during the test.

### 8. Deprotection of the N-terminal amino acid-amino (N-α-amino) protecting group.

Deprotection of the Fmoc group is performed by treatment with 20% piperidine in DMF (1x3 and 1x7 min when synthesized batchwise) or by flowing the deprotection solvent through the resin (10 min, flow rate 1 ml/min using continuous flow synthesis), followed by wash with DMF until no yellow colour (Dhbt-O-) could be detected after addition of Dhbt-OH to the drained DMF. Deprotection of the Boc group is performed by treatment with TFA in DCM 10x1.5 min and 1x20 min followed by wash 6x9 min each with DCM, neutralisation with 10% triethylamine in DCM (v/v) 2x1.5 min each, followed by 6x9 min wash with DCM.

### 9. Cleavage of peptide from resin with acid.

Peptides are cleaved from the resins by treatment with 95% triflouroacetic acid (TFA, Halocarbon Products Corporation, U.S.A.; Biesterfeld & Co. Hamburg, Germany)-water v/v at r.t. for 2 h. The filtered resins are washed with 95% TFA-water and filtrates and washings evaporated under reduced pressure. The residue is washed with ether and freeze dried from acetic acid-water. The crude freeze dried product is analysed by high-performance liquid chromatography (HPLC) and identified by matrix assisted laser desorption ionisation time of flight mass spectrometry (MALDI TOF MS) or by electrospray ionisation mass spectrometry (ES-MS).

### 10. Cleavage of peptide from resin with base.

The dried resin (1 g) is treated with 1 M sodium hydroxide (10 ml) at 4°C and left for 15 min at room temperature. The resin is filtered into a flask containing 10% aq. acetic acid. The peptide is isolated by lyophilization and submitted to gel filtration.

### 11. Cleavage of peptide from resin with TFMSA.

The dried resin (250 mg) is placed in a round-bottomed flask with a stirring bar. Thioanisole/ethanedithiol (2:1, 750FII) is added, the mixture chilled in ice, 5 mi TFA is added and the mixture is stirred for 5 - 10 min. TFMSA (500KII) is added drop wise and the reaction continued at room temperature (r.t.) for 30 - 60 min. The peptide is precipitated after addition of ether.

### 12. Deprotection of side chain protective groups

Preferably, the side chains are deprotected simultaneously with the cleavage of the peptide from the resin.

### 13. Preformed HObt-ester

### Method a.

3 eq. N-α-amino protected amino acid is dissolved in DMF together with 3 eq. HObt and 3 eq DIC. The solution was left at r.t. for 10 minutes and then added to the resin, which had been washed with a solution of 0.2 Dhbt-OH in DMF prior to the addition of the preactivated amino acid.

### Method b.

3 eq. N-α-amino protected amino acid is dissolved in DMF together with 3 eq. HObt, 3 eq TBTU and 4,5 eq. DIEA. The solution is left at r.t. for 5 minutes and then added to the resin. Preformed symmetrical anhydride 6 eq.N-u-amino protected amino acid is dissolved in DCM and cooled to 0 °C. DCC (3 eq.) is added and the reaction continued for 10 min. The solvent is removed in vacuum and the remainers dissolved in DMF. The solution is filtered and immediately added to the resin followed by 0.1 eq. of DMAP.

### 14. Estimation of the coupling yield of the first N-α-amino protected amino acid

3 - 5 mg dry Fmoc-protected peptide-resin is treated with 5 ml 20 times piperidine in DMF for 10 min at r.t. and the UV absorption for the dibenzofulvenepiperidine adduct is estimated at 301 nm. The yield is determined using a calculated extension coefficient e 301 based on a Fmoc-Ala-OH standard. In case of Boc-protection, the coupling is estimated according to the ninhydrin-method after removal of the Boc-group (Sarin, V.K. et al., (1981), Anal. Biochem, 117,147-157).

### 15. Peptide synthesis on PepSyn K resin

Dry PepSyn K (ca 500 mg) is covered by ethylenediamine and left at rt over night. The resin is drained and washed with DMF 10 x 15 ml, 5 min each. After draining the resin is washed with 10% DIEA in DMF v/v (2 x 15 ml, 5 min each) and finally washed with DMF until no yellow colour could be detected by addition of Dhbt-OH to the drained DMF. 3 eq. HMPA 3 eq. HObt and 3 eq. DIC is dissolved in 10 ml DMF and left for activation for 10 min, after which the mixture is added to the resin and the coupling continued for 24 h. The resin is drained and washed with DMF (10 x 15 ml, 5 min each), and the acylation was checked by the ninhydrin test. The first amino acid is coupled as the side chain protected preformed symmetrical anhydride (see above), and the coupling yields estimated as described above. It is in all cases to be better than 70%. The synthesis is then continued either as "continuous-flow" or as "Batchwise" as described below.

### 16. Continued peptide synthesis on PepSyn K using continuous-flow technique.

The resin (ca. 500 mg) with the first amino acid attached is placed in a column connected to the fully automated peptide synthesiser. The Fmoc group is deprotected as described above. The remaining amino acids according to the sequence are coupled as Fmocprotected, if necessary side chain protected, Pfp esters (3 eq.) with the addition of Dhbt-OH (1 eq.). The end-point of each coupling is determined automatically by monitoring the disappearance of the yellow colour of the Dhbt-OH anion spectrophotometrically. After completed synthesis the peptide-resin is washed with DMF (10 min flow rate 1 ml/min), DCM (3x5 ml, 3 min each) and finally diethyl ether (3x5 ml each) removed from the column and dried in vacuum.

### 17. Continued batchwise peptide synthesis on PepSyn K.

The resin (ca. 500 mg) with the first amino acid attached is placed in a polyethylene vessel equipped with a polypropylene filter for filtration, and the Fmoc-group deprotected as described above. The remaining amino acids according to the sequence are coupled as preformed Fmoc-protected, if necessary side chain protected, HObt esters (3 eq.) in DMF (5 ml) prepared as described above. The couplings are continued for 2 h unless otherwise specified. Excess reagent is then removed by DMF washing (12 min, flow rate 1 ml/min) All acylations are checked by the ninhydrin test performed at 80 °C. After completed synthesis the peptide-resin is washed with DMF (10 min, flow rate 1 ml/min), DCM (5x5 ml, 1 min each) and finally with diethyl ether (5x5 ml, 1 min each), and dried in vacuum.

### 18. Batchwise peptide synthesis on PEG-PS.

TentaGel S NH2 or NovaSyn TG resin (250 mg, 0.27-0.29 mmol/g) is placed in a polyethylene vessel equipped with a polypropylene filter for filtration. The resin is swelled in DMF (5 ml), and treated with 20% piperidine in DMF to secure the presence of nonprotonated amino groups on the resin. The resin is drained and washed with DMF until no yellow color could be detected after addition of Dhbt-OH to the drained DMF. HMPA (3 eq.) is coupled as a preformed HObt-ester as described above and the coupling is continued for 24 h. The resin is drained and washed with DMF (5 x 5 ml, 5 min each) and the acylation checked by the ninhydrin test. The first amino acid is coupled as a preformed symmetrical anhydride as described above. The coupling yields of the first Fmoc-protected amino acids are estimated as described above. It is in all cases to be better than 60%. The following amino acids according to the sequence are coupled as preformed Fmoc-protected, if necessary side chain protected, HObt esters (3 eq.) as described above. The couplings are continued for 2. h. The resin is drained and washed with DMF (5 x 5 ml, 5 min each) in order to remove excess reagent. All acylations are checked by the ninhydrin test performed at 80°C. After completed synthesis the peptide-resin is washed with DMF (3x5 ml, 5 min each), DCM (3x5 ml, 1 min each) and finally diethyl ether (3x5 ml, 1 min each) and dried in vacuo.

### 19. Batchwise peptide synthesis on PepSyn Gel

Dry PepSyn Gel resin (500 mg, 1 mmol/g) is placed in a polyethylene vessel equipped with a polypropylene filter for filtration. The resin is swelled in ethylenediamine (15 ml) and gently agitated by shaking for 20 h. The resin is drained and washed with DMF (10 x 15 ml, 5 min each). After draining the resin is washed with 10% DIEA in DMF v/v (2 x 15 ml, 5 min each) and finally washed with DMF (5 x 15 ml, 5 min each) until no yellow colour could be detected after addition of Dhbt-OH to the drained DMF. HMPA (3eq.) is coupled as a preactivated HObt-ester as described above (method a) and the coupling is continued for 24 h. The resin is drained and washed with DMF (5 x 15 ml, 5 min each). The acylation is checked by the ninhydrin test. The first amino acid is coupled as preformed side chain protected symmetrical anhydride as described above. The coupling yields of the first Fmoc-protected amino acids are estimated as described above. It is in all cases to be better than 70%. The remaining amino acids according to the sequence are coupled as preformed Fmoc-protected, if necessary side chain protected, HObt esters (3 eq.) as described above (method a). The couplings are continued for 2 h and, if necessary, double coupled over night. The resin is drained and washed with DMF (5 x 5 ml, 5 min each) in order to remove excess reagent. All acylations are checked by the ninhydrin test performed at 80°C. The Fmoc group is deprotected as described above. After completed synthesis the peptide-resin is washed with DMF (3x15 ml, 5 min each), DCM (3x15 ml, 2 min each) and finally diethyl ether (3x15 ml, 2 min each) and dried in vacuum.

### 19 HPLC.

HPLC may be performed on a Waters 600 E instrument equipped with a Waters 996 Photodiode array detector with a Waters Radial Pak 8 x 100 mm C18 reversed-phase column. Buffer A is 0.1 vol % TFA in water, and buffer B is 90 vol % acetonitrile, 9.9 vol % water and 0.1 vol % TFA. The Buffers are pumped through the column at a flow rate of 1.5 ml/min using the following gradient: linear gradient from 0% - 70% B (20 min), linear gradient from 70 - 100% B (1 min), isocratic 100% B (5 min). 2. Isocratic with 0% B (2 min), linear gradient from 0 - 50% B (23 min), linear gradient from 50 100% B (5 min), isocratic 100% B (5 min).

### 2.2 LPA production of the compound

According to the invention a compound of interest is preferably obtained by the LPA method.

### LPA method

The LPA method is disclosed in WO 00/18791. The method essentially comprises the following steps:
(a) providing by solid phase synthesis or fragment coupling peptide fragment(s) comprising the desired sequence(s), the peptide fragment(s) being attached to a solid phase;
(b) if necessary, deprotecting any N-terminal amino groups whole the peptide fragment(s) are still attached to a solid phase,
(c) reacting the peptide fragment(s) having unprotected N-terminal groups with an achiral di-, tri-, or tetracarboxylic acid so as to provide a construct having a ring structure, and
(d) cleaving the construct from the solid phase so as to provide an LPA comprising the peptide fragment(s) having free C-terminal groups.

In the above method, prior step (d) the following steps may be performed:
(c1) if present, deprotecting any N-protected groups originating from the carboxylic acid used in step (c),
(c2) continuing the solid phase synthesis or fragment coupling so as to provide peptide fragment(s) comprising desired sequence(s) having at least one N-protected N-terminal amino acid group and/or attaching chemical moieties, and
(c3) deprotecting, if present, any N-terminal amino groups (prior or after step (d)).

The method provides i.a. LPAs presenting desired sequences of the invention with N to C orientation (step (c)). And also simultaneously sequences with C to n orientation (step (c2))

Thus, to obtain a compound of the invention two peptide chains attached to a solid phase are to be assembled by means of achiral di-, tri- or tetracarboxylic acids. Suitable achiral di-, tri- or tetracarboxylic acids to be used in the present method have the general formula

X[(A)nCOOH][(B)mCOOH]

wherein n and m independently are an integer of from 1 to 20, X is HN, A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

In another embodiment suitable achiral di-, tri- or tetracarboxylic acids to be used in the present method have the general formula

X[(A)nCOOH][(B)mCOOH]

wherein n and m are 0 or an integer of from 1 to 20, X is H₂N(CR₂)pCR, or RHN(CR₂)pCR, wherein p is 0 or integer of from 1 to 20, wherein each R is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

In still another embodiment suitable achiral di-, tri- or tetracarboxylic acids to be used in the present method have the general formula

X[(A)nCOOH][(B)mCOOH]

wherein n and m are 0 or an integer of from 1 to 20, X is HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, or [HOOC(A)n][HOOC(B)m]CR(CR₂)pCR, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety. In yet another embodiment suitable achiral di-, tri- or tetracarboxylic acids to be used in the present method have the general formula

X[(A)nCOOH][(B)mCOOH]

Wherein n and m are 0 or an integer of from 1 to 20, X is H₂N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, or [HOOC(A)n][HOOC(B)m](CR₂)p, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

Under the term C₁₋₁₀ alkyl is meant straight or branched chain alkyl groups having 1-10 carbon atoms, e.g. methyl, ethyl, isopropyl, butyl, and tertbutyl.

Under the term C₂₋₁₀ alkenyl is meant straight or branched chain alkenyl groups having 2-10 carbon atoms, e.g. ethynyl, propenyl, isopropenyl, butenyl, and tert-butenyl.

Under the term cyclic moiety is meant cyclohexan, and cyclopentane.

Under the term aromatic moiety is meant phenyl.

The wording "A and B forms a cyclic, heterocyclic or aromatic moiety" denotes cyclohexan, piperidine, benzene, and pyridine.

By reaction with a carboxylic acid, a construct of the type
X(CO-sequence)2-solid phase, wherein X as defined above,
is obtained.

By the term "sequence" is in the present content meant a peptide comprising naturally occurring and/or non-naturally occurring amino acids, a PNA-sequence, or peptidomimetic. By "naturally occurring amino acids" is meant L- and D-forms of the 20 acids found in nature. Non-naturally occurring amino acids are e.g. modified naturally occurring amino acids. The term sequence is further intended to comprise one or more of such sequences. Examples of suitable peptidomimetics are described in Marshall G.R.,(1993) Tetrahedron, 49:3547-3558. The term "chemical moieties" denotes an entity enhancing the solubility or biological activity of the LPA, and entity for directing the LPA to its target or a marker. Preferred embodiments for the sequences are described above.

The group X permits directly or indirectly continued stepwise synthesis or a fragment coupling of the same sequence, or of one or more different sequences and/or moieties. Orientation of peptide fragments (N to C or C to N) in LPA is defined as desired. In one embodiment the present invention features LPAs with N to C orientation, in another embodiment it concerns the compounds with simultaneous N to C and C to N presentation of the sequences, and in yet another embodiment the sequences have C to N orientation.

In the case where X comprises a temporally protected amino function, synthesis or coupling can be carried out directly after protection. Suitable activation of all carboxyl-containing groups providing effective formation of the ring system (on step (c), see above) can be ensured using half-equivalent carboxy acid. In case of tri- or tetracarboxylic acids the activated carboxy group may further be derivatised with a diamine such as ethylenediamine or an amine suitably functionalised for further reactions such as mercapto-, an oxy-, an oxo or carboxyl group. In the case of diamine, peptide synthesis or fragment coupling can be continued directly according to the desired sequence or chemical moiety. In a preferred embodiment, the Fmoc-protection strategy is used, but any amino protection group may be used depending on the synthesis or coupling strategy. Examples are the Boc-protection group strategy.

Since the continued stepwise synthesis or fragment coupling is performed with one or in case of a bifunctional chemical moiety such lysine with two amino acid groups, it has surprisingly been found that a much better result can be obtained as compared to conventional tetrameric lysine dendimers obtained by the MAP synthesis. Furthermore, optimal peptide synthesis procedures or coupling procedures can be used for the single chains attached to the solid phase, and their homogeneity can be verified prior to forming the LPA. Cleavage from the solid phase and simultaneous side-chain deprotection can be performed by standard peptide synthesis procedures (described above). A final product may thus be obtained having optimal and well-defined composition. Purification by standard chromatography methods such as HPLC or gel filtration can easily be performed, if desired or needed.

Favourable di-, tri- and tetracarboxilyc acids for providing the ring structure may be selected from imino diacetic acid, 2-amino malonic acid, 3-amino glutaric acid, 3-methylamino glutaric acid, 3-chloro glutamic acid, 3-methoxy-carbonyl glutaric acid, 3-acetyl glutaruc acid, glutaric acid, tricarballylic acid,3,4-bis-carboxymethyl adipic acid, 4-(2-carboxyethyl)-pimelic acid, (3,5-bis-carboxymethyl-phenyl)-acetic acid, 3,4-bis-carboxymethyl-adipic acid, benzene-1,2,4,5-tetra carboxylic acid, 4-(3-carboxy-allylamino)-but-2-enoic acid, 4,4-imino-dibenzoic acid, 1,4-dihydropyridine-3,5-dicarboxylic acid, 5-amino isophthalic acid, 2-chloro malonic acid, 3-hydroxy glutaric acid, and benzene-1,3,5-tricarboxylic acid.

Fragment coupling (fragment coupling or fragment condensation) may be performed according to standard procedures, e.g. as described in Peptide Synthesis protocols, Methods in Molecular Biology Vol. 35, Chapter 15, 303-316, Nyfeler R, Pennington MW and Dunne BM Eds.,Humana Press, 1994. Accordingly, fragments may be synthesised on a solid phase, cleaved from the solid phase with full preservation of protecting groups, purified and characterised as described above. Suitable fragments may also be obtained by other techniques described above.

It is a preferred embodiment of the invention to use the above LPA method for the production of a compound of the invention.

### 3. Biological activity

According to the disclosure the compound can bind and modulate activity of a cell surface receptor through a binding site located within the peptide sequence(s) of the compound, said binding site comprising at least one amino acid sequence of the formula

L1-A-L2-B-L3-C-L4-D-L5

wherein
one of A, B, C, D is selected from a hydrophobic amino acid residue,
one of A, B, C, D is selected from a basic amino acid residue, Asn or Gln,
one of A, B, C, D is selected from an acidic amino acid residue, Asn or Gln,
one of A, B, C, D is Gly or Ala, and
L1, L2, L3, L4 and L5 is selected from a chemical bond or an amino acid sequence having n amino acid residues, wherein n is an integer of from 0 to 5, wherein said amino acid sequence is EVYVVAENQQGKSKA (SEQ ID NO:1).

Therefore, all peptide sequences having the length of 4 to 80 amino acid residues comprising the above receptor binding structural motif are disclosed. Preferred embodiments of the peptide sequences are discussed above.

### Receptor

Thus, a compound of the invention is the ligand of a receptor, in particular a functional cell-surface receptor.

A cell-surface receptor is defined in the content of the present invention as any molecule comprising at least one polypeptide chain, said molecule being associated with the plasma membrane of a cell in such a way that enables said molecule to receive a signal at the outer side of the membrane, transduce the signal through the membrane, and convey said signal further by inducing a certain action on molecular level inside the cell, for example by inducing association or dissociation of molecular complexes, or initiating a biochemical reaction, for example auto-phosphorylation of the receptor, or proteolytic cleavage of the receptor leading to initiation of intracellular signal transduction.

The invention relates to a functional cell-surface receptor. By "functional cell-surface receptor" is meant that the cell-surface receptor of the invention has an identifiable group of ligands, the binding of these ligands to the receptor induces intracellular signal transduction, which results in a physiological response of the cell. The physiological response, such as for example differentiation, proliferation, survival, apoptosis or motility of a cell, depends on the ligand that is involved in the interaction with the receptor, and/or characteristics of said interaction, such as the affinity or duration, and/or a species of the cell which expresses the receptor. By the term "ligand" is defined a compound which is capable to bind to the receptor and thereby to activate or inhibit said receptor. "Activation" of a receptor is meant that after extracellular binding of a ligand the receptor became capable to transmit the effect of "ligand binding" into a cascade of biochemical reactions collectively termed "receptor signalling" or "signal transduction" inside the cell resulting in one of the above mentioned physiological responses of the cell. "inhibition" of a receptor is meant that after extracellular binding of a ligand the receptor became "silent" or "inactive" and is not capable anymore initiate a cascade of biochemical reactions which is normally initiated in response to receptor ligand binding. According to the invention compounds featured above are capable of activation or inhibition of a functional cell-surface receptor.

The functional cell-surface receptor of the invention, is a receptor of the family of fibroblast growth factor receptors (FGFRs) comprising FGFR1, FGFR2, FGFR3 and FGFR4. In most preferred embodiment, a receptor of the invention is FGFR1 or a functional homologue thereof.

By the term "functional homologue of the receptor " is meant a molecule which is capable of
i) extracellular binding of a ligand of FGFR and thereby activating the receptor dependent signal transduction cascade in the cell, and/or
ii) intracellular binding of an adapter molecule of FGFR and thereby activating the receptor dependent signal transduction cascade in the cell.

The term "binding" refers to a direct or indirect interaction between FGFR or an FGFR homologue and a counter molecule having a binding site for FGFR. In the present context the counter molecule is an extracellular ligand of FGFR, or an intracellular adapter molecule. An "adapter molecule" is defined in the content of the invention as a molecule, which is capable to recognise an "active state" of the receptor, selectively bind to such receptor and convey the signal of "activated receptor" in the cell by inducing a cascade of reactions of signal transduction. An intracellular adapter molecule may be represented by for example STN, FRS, Grb, SHP2, PLCγ, or PIP3.

Activation of a receptor often depends on the receptor dimerisation induced by ligand binding. Therefore, the capability of a compound to promote dimerisation (or multimerisation) of the receptor molecules is concerned by the invention as an advantageous feature. Thus, in one embodiment the invention features a compound that is capable of dimerising of FGFR. Dimerisation of the receptor by a compound of the invention may take place for example in situation when a natural high affinity ligand of the receptor, e.g. FGF, is absent in receptor environment and the receptor is temporary "silent", inactive. However, in the presence of one of the FGFs the receptor is occupied and activated by this FGF, and the compounds of the invention have therefore a low capacity or are not capable at all to modulate FGFR receptor signalling induced by the FGF. Nevertheless, compounds of the invention may attenuate binding of natural high affinity ligands to FGFR by occupying an alternative binding site(s), if the receptor has been exposed to the compound(s) prior to FGF exposure. In this way the compounds of the invention may modulate the receptor signalling dependent on FGF. It is known in the art that the cellular response to activation of a receptor depends on the strength of receptor stimulation, which may, for example, be characterised by the value of affinity of interaction of a ligand with the receptor, and/or by the duration of such interaction. Thus, both affinity and duration of interaction of FGF and the receptor may be affected by a compound of the invention. Accordingly, it is another embodiment of the invention to provide a compound, which is capable of modulating the receptor signalling induced by a high affinity ligand. Further embodiments concern 1) a compound(s), which is capable of inhibiting FGFR activation induced by a low affinity ligand discussed above by competing with said ligand for FGFR binding at the binding site of the invention , 2) a compound(s), which is capable of activating FGFR inhibited by a low affinity ligand discussed above or another molecule, said another molecule being a natural FGFR inhibitor or artificial substance.

### Affinity of interaction

By the term "interaction" is meant that a compound has a transient or permanent, direct or indirect contact with a cell-surface receptor.

Interaction between two molecules may be characterised by affinity of the interaction. The affinity of interaction is commonly expressed by the value of dissociation equilibrium constant, Kd, expressed in moles (M). Kd reflects the ratio between the rate of dissociation of a ligand molecule from a receptor molecule and the rate of binding of said ligand molecule to said receptor molecule, and thus represents a measure of the strength of binding between these two molecules. Stronger the binding, lower is the value of Kd.

The invention relates to compounds having a relatively low affinity binding to FGFR. The low affinity interaction of the invention is characterised by Kd having a value in the range of 10⁻³ to 10⁻⁸ M, such for example from 10⁻³ to 10⁻⁷M, such for example from 10⁻³ to 10⁻⁶ M, such for example from 10⁻³ to 10⁻⁵ M, or about 10⁻⁴ M, or about 10⁻⁵ M.

### Modulation of biological function of the receptor

A capability of a cell-surface receptor to induce and/or maintain a cellular process upon the ligand binding is herein termed "biological function" of the receptor.

The invention features compounds that are capable of modulating the biological function of FGFR by modulating the receptor signalling. By "modulating receptor signalling" is meant activation or inhibition of the production of a cascade of messenger molecules, which normally takes place in the cell in response to activation of the receptor by an extracellular stimulus.

Activation or inhibition of the receptor signalling is normally reflected by a physiological response of the cell expressing said receptor. Therefore, by modulating the receptor signalling it is possible to modulate cell response.

The invention preferably concerns activation or inhibition of FGFR1-associated signalling, which may for example be reflected by a change in i) the degree of tyrosine phosphorylation of FGFR1; ii) the activation status of one or more intracellular proteins involved in any of the FGFR1-associated signal transduction pathways, such as for example the STAT1, JNK, PLCγ, ERK, STAT5, PI3K, PKC, FRS2 and/or GRB2 proteins; and/or iii) cellular response.

When concerned FGFR dependent cellular response, the invention relates to a cellular response selected from but not limited by induction/inhibition of cell differentiation or dedifferentiation, apoptosis or cell survival, cell motility.

### Medicament

Cell death plays a key role in normal neuronal development, where 50% of the developing neurons are eliminated through programmed cell death, and in the pathophysiology of neurodegenerative conditions, such as Alzheimer's and Parkinson's diseases. FGFRs and their ligands has been shown to be important determinants of neuronal survival both during development and during adulthood (Reuss and von Bohlen und Halbach (2003) Cell. tissue Res, 313:139-57). Therefore, a compound, which is capable to promote neuronal cell survival by binding and activation FGFR is highly desirable. Thus, in one aspect the invention features compounds that promote survival of neural cells and can be used as medicaments for the treatment of conditions involving neural cell death. However, a compound of the invention may also be used as a medicament for promotion of survival of another type of cells, e.g. different type of muscle cells, or, alternatively, for promotion of cell death of still another type of cells, e.g. cancer cells, as the FGFR signalling has been shown to be a survival factor for both muscle and cancer cells (Ozen et al. (2001) J Nat Cancer Inst. 93:1783-90; Miyamoto et al. (1998) J Cell Physiol. 177:58-67; Detilliux et al. (2003) Cardiovasc Res. 57:8-19).

Activity of cell-surface receptors is strictly regulated in a healthy organism. Mutations, abnormal expression or processing of a receptor or the receptor ligands lead to abnormalities in activity of the receptor and therefore lead to dysfunction of the receptor. The dysfunction of the receptor is in turn a reason for dysfunction of the cells which use the receptor for induction and/or maintenance of various cellular processes. The latter is the manifestation of a disease. It has also been shown that attenuation of FGFR signalling leads to development of a number of different pathologic conditions, e.g. diabetes (Hart et al., Nature 2000, 408:864-8). Activation of FGF receptors is involved in normal, as well as in pathologic angiogenesis (Slavin, Cell Biol Int 1995, 19:431-44). It is important for development, proliferation, functioning and survival skeletal muscle cells, cardiomyocytes and neurons (Merle at al., J Biol Chem 1995,270:17361-7; Cheng and Mattson, Neuron 1991, 7:1031-41; Zhu et al., Mech Ageing Dev 1999, 108:77-85). It plays a role in maintenance of normal kidney structure (Cancilla et al., Kidney Int 2001, 60:147-55), and it is implicated in mound healing and cancer disease (Powers et al., Endocr Relat Cancer. 2000, 7:165-97).

The present invention provides compounds capable of modulation of the activity of FGFRs. Consequently, said compounds are concerned by the invention as medicament for the treatment of diseases, wherein modulation of the activity of FGFRs may be considered as an essential condition for the curing.

Thus, the medicament of the invention is in one embodiment for prevention and/or treatment of
1) diseases and conditions of the central and peripheral nervous system, or of the muscles or of various organs, and/or
2) diseases or conditions of the central and peripheral nervous system, such as postoperative nerve damage, traumatic nerve damage, impaired myelination of nerve fibers, postischaemic damage, e.g. resulting from a stroke, Parkinson's disease, Alzheimer's disease, Huntington's disease, dementias such as multiinfarct dementia, sclerosis, nerve degeneration associated with diabetes mellitus, disorders affecting the circadian clock or neuro-muscular transmission, and schizophrenia, mood disorders, such as manic depression;
3) for treatment of diseases or conditions of the muscles including conditions with impaired function of neuro-muscular connections, such as after organ transplantation, or such as genetic or traumatic atrophic muscle disorders; or for treatment of diseases or conditions of various organs, such as degenerative conditions of the gonads, of the pancreas such as diabetes mellitus type I and II, of the kidney such as nephrosis and of the heart, liver and bowel, and/or
4) cancer disease, and/or
5) prion diseases.

The invention concerns cancer being any type of solid tumors requiring neoangiogenesis.

The invention concerns prion diseases selected from the group consisting of scrapie, Creutzfeldt-Jakob disease. It has been shown that FGFRs plays a distinct role in prion diseases (Castelnau et al. (1994) Exp Neurobiol. 130:407-10; Ye and Carp (2002) J Mol Neurosci. 18:179-88).

In another embodiment a compound of the invention is for the manufacture of a medicament for
1) promotion of wound-healing, and/or
2) prevention of cell death of heart muscle cells, such as after acute myocardial infarction, or after angiogenesis, and/or
3) revascularsation.

FGFRs and their ligands play important roles in CNS, in particular they are involved in the processes associated with memory and learning (Reuss and von Bohlen und Halbach (2003) Cell Tissue Res. 313:139-57). Thus, in still another embodiment a compound of the invention is for stimulation of the ability to learn and/or of the short and/or long-term memory. This embodiment is one of the preferred embodiments of the invention.

In yet another embodiments a compound of the invention is for the manufacture of a medicament for the
1) prevention of cell death due to ischemia;
2) prevention of body damages due to alcohol consumption;

In particular, the invention concerns normal, degenerated or damaged NCAM presenting cells.

The medicament of the invention comprises an effective amount of one or more compounds as defined above, or a pharmaceutical composition comprising one or more compounds and pharmaceutically acceptable additives.

Thus, the invention in another aspect also concerns a pharmaceutical composition comprising at least one compound of the invention.

A further aspect of the invention is a process of producing a pharmaceutical composition, comprising mixing an effective amount of one or more of the compounds of the invention, or a pharmaceutical composition according to the invention with one or more pharmaceutically acceptable additives or carriers. In one embodiment the compounds are used in combination with a prosthetic device, wherein the device is a prosthetic nerve guide. Thus, in a further aspect, the present invention relates to a prosthetic nerve guide, characterised in that it comprises one or more of the compounds or the pharmaceutical composition as defined above. Nerve guides are known in the art.

The invention relates to use of a medicament and/or pharmaceutical composition comprising the compound of invention for the treatment or prophylaxis of any of the diseases and conditions mentioned below.

Such medicament and/or pharmaceutical composition may suitably be formulated for oral, percutaneous, intramuscular, intravenous, intracranial, intrathecal, intracerebroventricular, intranasal or pulmonal administration.

Strategies in formulation development of medicaments and compositions based on the compounds of the present invention generally correspond to formulation strategies for any other protein-based drug product. Potential problems and the guidance required to overcome these problems are dealt with in several textbooks, e.g. "Therapeutic Peptides and Protein Formulation. Processing and Delivery System" Ed. A.K. Banga, Technomic Publishing AG, Basel, 1995.

Injectables are usually prepared either as liquid solutions or suspensions, solid forms suitable for solution in, or suspension in, liquid prior to injection. The preparation may also be emulsified. The active ingredient is often mixed with excipients which are pharmaceutically acceptable and compatible with the active ingredient. Suitable excipients are, for example, water, saline, dextrose, glycerol, ethanol or the like, and combinations thereof. In addition, if desired, the preparation may contain minor amounts of auxiliary substances such as wetting or emulsifying agents, pH-buffering agents, or which enhance the effectiveness or transportation of the preparation.

Formulations of the compounds of the invention can be prepared by techniques known to the person skilled in the art. The formulations may contain pharmaceutically acceptable carriers and excipients including microspheres, liposomes, microcapsules, nanoparticles or the like.

The preparation may suitably be administered by injection, optionally at the site, where the active ingredient is to exert its effect. Additional formulations which are suitable for other modes of administration include suppositories, nasal, pulmonal and, in some cases, oral formulations. For suppositories, traditional binders and carriers include polyalkylene glycols or triglycerides. Such suppositories may be formed from mixtures containing the active ingredient(s) in the range of from 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and generally contain 10-95% of the active ingredient(s), preferably 25-70%.

Other formulations are such suitable for nasal and pulmonal administration, e.g. inhalators and aerosols.

The active compound may be formulated as neutral or salt forms. Pharmaceutically acceptable salts include acid addition salts (formed with the free amino groups of the peptide compound) and which are formed with inorganic acids such as, for example, hydrochloric or phosphoric acids, or such organic acids as acetic acid, oxalic acid, tartaric acid, mandelic acid, and the like. Salts formed with the free carboxyl group may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are per kilo body weight normally of the order of several hundred µg active ingredient per administration with a preferred range of from about 0.1 µg to 5000 µg per kilo body weight. Using monomeric forms of the compounds, the suitable dosages are often in the range of from 0.1 µg to 5000 µg per kilo body weight, such as in the range of from about 0.1 µg to 3000 µg per kilo body weight, and especially in the range of from about 0.1 µg to 1000 µg per kilo body weight. Using multimeric forms of the compounds, the suitable dosages are often in the range of from 0.1 µg to 1000 µg per kilo body weight, such as in the range of from about 0.1 µg to 750 µg per kilo body weight, and especially in the range of from about 0.1 µg to 500 µg per kilo body weight such as in the range of from about 0.1 µ9 to 250 µg per kilo body weight. In particular, when administering nasally smaller dosages are used than when administering by other routes. Administration may be performed once or may be followed by subsequent administrations. The dosage will also depend on the route of administration and will vary with the age and weight of the subject to be treated. A preferred dosage of multimeric forms would be in the interval 1 mg to 70 mg per 70 kg body weight.

For some indications a localised or substantially localised application is preferred.

For other indications, intranasal application is preferred.

Some of the compounds of the present invention are sufficiently active, but for some of the others, the effect will be enhanced if the preparation further comprises pharmaceutically acceptable additives and/or carriers. Such additives and carriers will be known in the art. In some cases, it will be advantageous to include a compound, which promotes delivery of the active substance to its target.

In many instances, it will be necessary to administrate the formulation multiple times. Administration may be a continuous infusion, such as intraventricular infusion or administration in more doses such as more times a day, daily, more times a week, weekly, etc. It is preferred that administration of the medicament is initiated before or shortly after the individual has been subjected to the factor(s) that may lead to cell death. Preferably the medicament is administered within 8 hours from the factor onset, such as within 5 hours from the factor onset. Many of the compounds exhibit a long term effect whereby administration of the compounds may be conducted with long intervals, such as 1 week or 2 weeks.

In connection with the use in nerve guides, the administration may be continuous or in small portions based upon controlled release of the active compound(s). Furthermore, precursors may be used to control the rate of release and/or site of release. Other kinds of implants and well as oral administration may similarly be based upon controlled release and/or the use of precursors.

### Treatment

Treatment by the use of the compounds/compositions according to the invention is in one embodiment useful for inducing differentiation, modulating proliferation, stimulate regeneration, neuronal plasticity and survival of cells, for example cells being implanted or transplanted. This is particularly useful when using compounds having a long term effect.

In further embodiment the treatment may be for stimulation of survival of cells which are at risk of dying due to a variety of factors, such as traumas and injuries, acute diseases, chronic diseases and/or disorders, in particular degenerative diseases normally leading to cell death, other external factors, such as medical and/or surgical treatments and/or diagnostic methods that may cause formation of free radicals or otherwise have cytotoxic effects, such as X-rays and chemotherapy, In relation to chemotherapy the FGFR binding compounds according to the invention are useful in cancer treatment.

Thus, the treatment comprises treatment and/or prophylaxis of cell death in relation to diseases or conditions of the central and peripheral nervous system, such as postoperative nerve damage, traumatic nerve damage, e.g. resulting from spinal cord injury, impaired myelination of nerve fibers, postischaemic damage, e.g. resulting from a stroke, multiinfarct dementia, multiple sclerosis, nerve degeneration associated with diabetes mellitus, neuro-muscular degeneration, schizophrenia, Alzheimer's disease, Parkinson's disease, or Huntington's disease.

Also, in relation to diseases or conditions of the muscles including conditions with impaired function of neuro-muscular connections, such as genetic or traumatic atrophic muscle disorders; or for the treatment of diseases or conditions of various organs, such as degenerative conditions of the gonads, of the pancreas, such as diabetes mellitus type I and II, of the kidney, such as nephrosis the compounds according to the invention may be used for inducing differentiation, modulating proliferation, stimulate regeneration, neuronal plasticity and survival , i.e. stimulating survival.

Furthermore, the compound and/or pharmaceutical composition may be for preventing cell death of heart muscle cells, such as after acute myocardial infarction, in order to induce angiogenesis. Furthermore, in one embodiment the compound and/or pharmaceutical composition is for the stimulation of the survival of heart muscle cells, such as survival after acute myocardial infarction. In another aspect the compound and/or pharmaceutical composition is for revascularisation, such as after injuries.

It is also within the scope of the invention a use of the compound and/or pharmaceutical composition for the promotion of wound-healing. The present compounds are capable of stimulating angiogenesis and thereby they can promote the wound healing process.

The invention further discloses a use of the compound and/or pharmaceutical composition in the treatment of cancer. Regulation of activation of FGFR is important for tumor agiogenesis, proliferation and spreading.

In yet a further embodiment a use of the compound and/or pharmaceutical composition is for the stimulation of the ability to learn and/or of the short and/or long term memory, as FGFR activity is important for differentiation of neural cells.

In still another embodiment a compound and/or pharmaceutical composition of the invention is for the treatment of body damages due to alcohol consumption. Developmental malformations of foetuses, long-term neurobehavioral alterations, alcoholic liver disease are particularly concerned.

Therapeutic treatment of prion diseases including using a compound and/or pharmaceutical composition is still another embodiment of the invention.

In particular the compound and/or pharmaceutical composition of the invention may be used in the treatment of clinical conditions, such as Neoplasms such as malignant neoplasms, benign neoplasms, carcinoma in situ and neoplasms of uncertain behavior, diseases of endocrine glands, such as diabetes mellitus, psychoses, such as senile and presenile organic psychotic conditions, alcoholic psychoses, drug psychoses, transient organic psychotic conditions, Alzheimer's disease, cerebral lipidoses, epilepsy, general paresis [syphilis], hepatolenticular degeneration, Huntington's chorea, Jakob-Creutzfeldt disease, multiple sclerosis, Pick's disease of the brain, syphilis,Schizophrenic disorders, affective psychoses, neurotic disorders, personality disorders, including character neurosis, nonpsychotic personality disorder associated with organic brain syndromes, paranoid personality disorder, fanatic personality, paranoid personality (disorder), paranoid traits, sexual deviations and disorders, mental retardation, disease in the nervesystem and sense organs, cognitive anomalies, inflammatory disease of the central nervous system, such as meningitis, encephalitis, Cerebral degenerations such as Alzheimer's disease, Pick's disease, senile degeneration of brain, communicating hydrocephalus, obstructive hydrocephalus, Parkinson's disease including other extra pyramidal disease and abnormal movement disorders, spinocerebellar disease, cerebellar ataxia, Marie's, Sanger-Brown, Dyssynergia cerebellaris myoclonica, primary cerebellar degeneration, such as spinal muscular atrophy, familial, juvenile, adult spinal muscular atrophy, motor neuron disease, amyotrophic lateral sclerosis, motor neuron disease, progressive bulbar palsy, pseudobulbar palsy, primary lateral sclerosis, other anterior horn cell diseases, anterior horn cell disease, unspecified, other diseases of spinal cord, syringomyelia and syringobulbia, vascular myelopathies, acute infarction of spinal cord (embolic) (nonembolic), arterial thrombosis of spinal cord, edema of spinal cord, subacute necrotic myelopathy, subacute combined degeneration of spinal cord in diseases classified elsewhere, myelopathy, drug-induced, radiation-induced myelitis, disorders of the autonomic nervous system, disorders of peripheral autonomic, sympathetic, parasympathetic, or vegetative system, familial dysautonomia [Riley-Day syndrome], idiopathic peripheral autonomic neuropathy, carotid sinus syncope or syndrome, cervical sympathetic dystrophy or paralysis, peripheral autonomic neuropathy in disorders classified elsewhere, amyloidosis, diseases of the peripheral nerve system, brachial plexus lesions, cervical rib syndrome, costoclavicular syndrome, scalenus anterior syndrome, thoracic outlet syndrome, brachial neuritis or radiculitis, including in newborn. Inflammatory and toxic neuropathy, including acute infective polyneuritis, Guillain-Barre syndrome, Postinfectious polyneuritis, polyneuropathy in collagen vascular disease, disorders affecting multiple structures of eye, purulent endophthalmitis, diseases of the ear and mastoid process, chronic rheumatic heart disease, ischaemic heart disease, arrhythmia, diseases in the pulmonary system, abnormality of organs and soft tissues in newborn, including in the nerve system, complications of the administration of anesthetic or other sedation in labor and delivery, diseases in the skin including infection, insufficient circulation problem, injuries, including after surgery, crushing injury, bums. Injuries to nerves and spinal cord, including division of nerve, lesion in continuity (with or without open wound), traumatic neuroma (with or without open wound), traumatic transient paralysis (with or without open wound), accidental puncture or laceration during medical procedure, injury to optic nerve and pathways, optic nerve injury, second cranial nerve, injury to optic chiasm, injury to optic pathways, injury to visual cortex, unspecified blindness, injury to other cranial nerve(s), injury to other and unspecified nerves. Poisoning by drugs, medicinal and biological substances, genetic or traumatic atrophic muscle disorders; or for the treatment of diseases or conditions of various organs, such as degenerative conditions of the gonads, of the pancreas, such as diabetes mellitus type I and II, of the kidney, such as nephrosis. Scrapie, Creutzfeldt-Jakob disease, Gerstmann-Straussler-Sheinker (GSS) disease.

According to the invention the treatment and/or prevention of the above conditions and symptoms comprises a step of administering an effective amount of a compound and/or pharmaceutical composition to an individual in need.

### Examples

### Example 1. Production of different formulations of the FGL peptide (SEQ ID NO: 1) and other peptides of the invention

### Solid Phase Synthesis of the individual peptide chain of FGL

The individual peptide chain of FGL and other peptide sequences of the invention, such as for example the EFL peptide, is synthesized by the standard Fmoc-solid phase method as described above. The synthesis of the peptide used in the experiments is performed on TentaGel S RAM resin (90 mg, 0.22 mmol/g). The resin was placed in a polyethylene vessel equipped with a polypropylene filter for filtration. The resin was swelled in DMF, and treated with 20% piperidine in DMF to secure the presence of non-protected amino groups on the resin. Afterwards the resin was drained and washed with DMF until no yellow colour could be detected after addition of Dhbt-OH to the drained DMF. The amino acids were coupled one at a time alternating with removal of Fmoc-groups. The Fmoc-amino acids were preactivated in DMF by TBTU /HOBt before the coupling to the growing resin-bound peptide chain. For the removal of Fmoc-groups a solution of piperidine in DMF was used.

### LPA production of the FGL dimer

The LPA-type dimer of FGL (FGL_{L}) of the application was made by coupling Boc-iminodiacetic acid to the peptide on the resin, using TBTU/HOBt as described in WO 00/18791. To reduce side reactions multiple coupling were performed with Boc-iminodiacetic acid as the limiting component. The peptide was cleaved from the resin and simultaneously deprotected on the side chains in TFA at the presence of TES and water as scavengers to yield the peptide amide. The amount of TFA was reduced by evaporation and the peptide precipitated. Final purification of FGL_{L} was done by reversed phase HPLC. The conditions of HPLC were as follows:

| | |
|---|---|
| Column: | YMC ODS-AMQ, 5 µm, 4.6 x 250 mm, 200 A |
| Flow: | 1.0 ml/min |
| Mobile phases: | A: 0.12% TFA, 95% H₂O, 5% ACN |
| | B: 0.1 % TFA, 95% ACN, 5% H₂O |
| Gradient: | 15% B to 35% B in 20 min, |
| | 35% B to 95% B in 1 min and hold for 5 min. |
| Detection: | Diode array detector 190-400 nm |
| Wavelength: | 220 nm |
| injection volume: | 20 µl, |
| Concentration of FGL_{L} | 0.5 mg/ml |

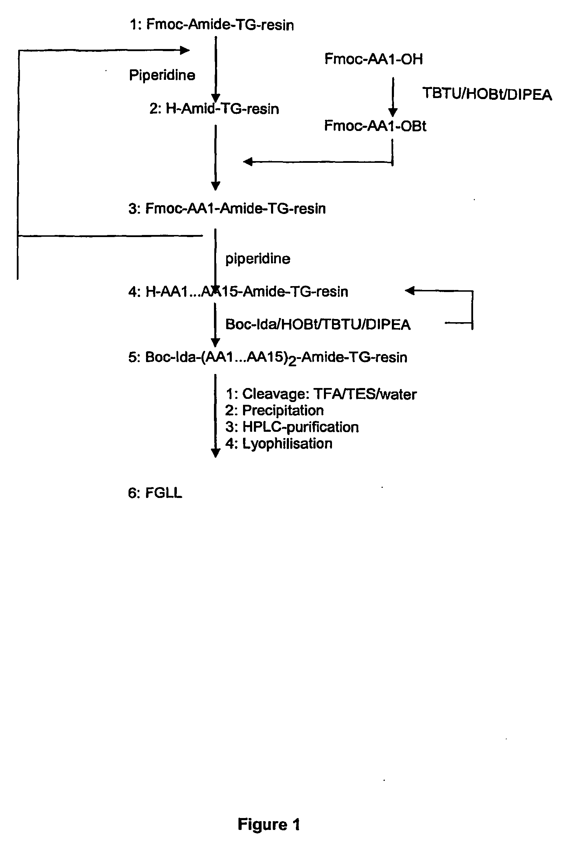

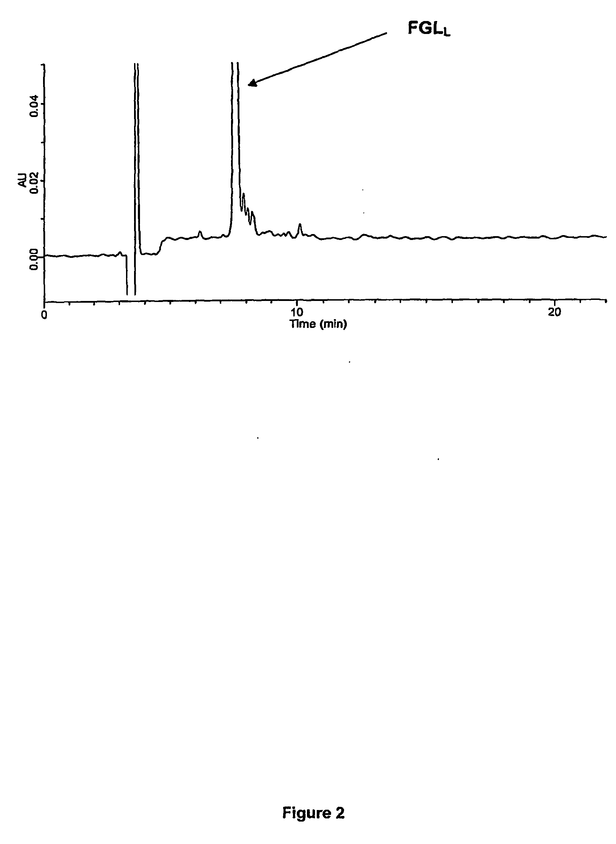

The HPLC purified FGL_{L} was isolated by lyophilisation. The flowchart of FGL_{L} synthesis is shown in Figure1. Figure 2 depicts the HPLC elution profile of the final purification of FGL_{L}.

### Abbreviations

Abbreviations for amino acids are in accordance with the recommendations in the IUPAC-IUB Joint Commission on Biochemical Nomenclature Eur. J. Biochem, 1984, vol. 184, pp 9-37

### Other abbreviations:

- AcOH: Acetic acid
- TBTU: O-(Benzotriazol-1-yl)-N,N,N',N'-tetramethyluronium tetrafluoroborate
- Ida: Boc iminodiacetic acid
- MTBE: t-Butyl methyl ether
- DMF: Dimethylformamide
- EtOH: Ethanol, 99,9%
- DIPEA: N-Ethyl-diisopropylamine
- HOBt: 1-Hydroxybenzotriazol
- NMP: N-methylpyrrolidone
- TFA: Trifluoroacetic acid
- TES: Triethylsilane
- Boc: N-tertButyl oxycarbonyl
- Fmoc: 9-Fluorenylmethyloxycarbonyl
- tBu: tert-Butyl
- HPLC: High pressure liquid chromatography
- R: Amide-TG-resin
- AA: Amino acid

### Physico-chemical properties of FGL_{L}

FGL_{L} has the structural formula:

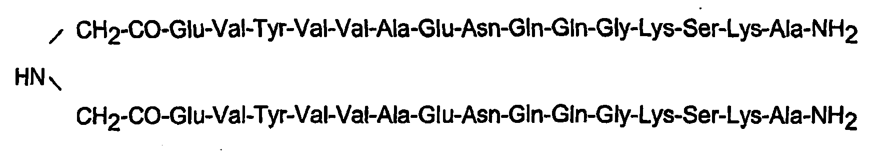

It consists of two identical peptide sequences of 15 amino acid residues co-joined by the linker group, amidoacetic acid (N-(carboxymethyl)glycine), on their N-terminals. The molecular weight of FGL_{L} is 3394.8 Dalton. Non-covalent aggregation of the peptide compound in concentrated water solutions (higher then 0,5 mg/ml) is prevented by addition of acetic acid.

### FGL_{cys} and FGL_{lys} dimmers synthesis

The FGLcys dimer consisting of two individual FGL sequences, each containing an additional cysteine residue at the N-terminal end bound through an S-S bond, was synthesized using solid-phase synthesis described in in Goodwin et al. (1998) Bioorg Med Chem Lett 8:2231-2234.

The FGLlys dimer, constructed as two individual FGL monomer sequences coupled through their C-terminal to lysine, was made using solid-phase synthesis described in Rajagopalan et al. (1995) Int J Pept Protein Res.45:173-179.

### Synthesis of dendrimeric tetramer of FGL (FGL_{d})

The MAP-type dendrimer of FGL, FGL_{d}, was synthesised according to the standard method as for example described in PCT/US90/02039.

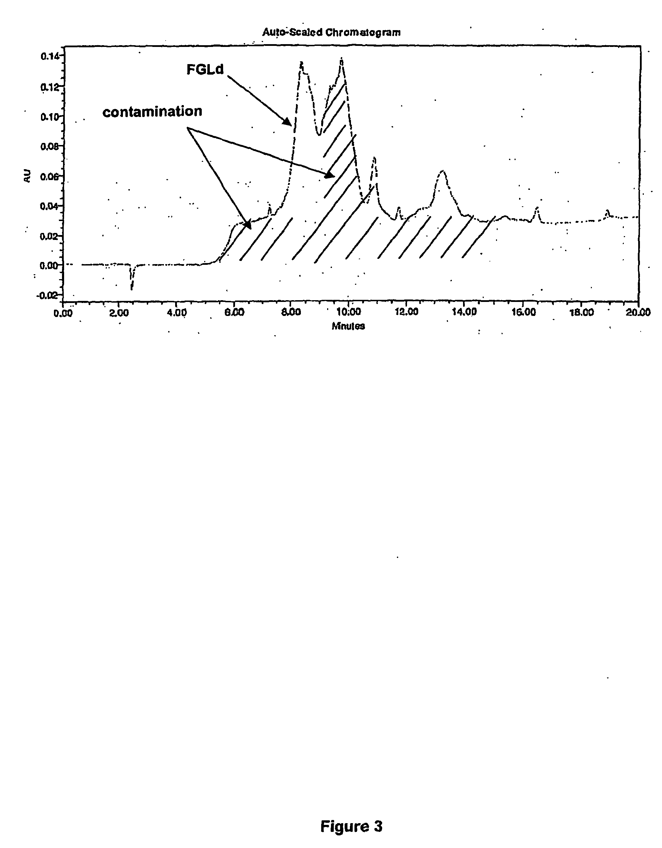

Figure 3 demonstrates the HPLC profile of the final purification of FGL_{D}. The hatched area indicates the level of heterogeneity of the synthesised product.

### Examples 2. The effect of different formulations of FGL on survival of neurons in vitro

Different formulations of the FGL peptide were copared for biological activity in the survival assays well known in the art and described in detaled below.

### Dopaminergic neurons (DN)

Dopaminergic neurons were prepared from Wistar rat embryos at embryonic day 15 (Charles River, Sulzfeld, Germany or Møllegaard, Denmark). A pregnant rat was sacrificed and the uterus was taken out and kept on ice in Hank's balanced salt solution (HBSS; Gibco, BRL). The ventral part of the mesencephalon was dissected from the embryonic brains, homogenised on ice in Gey's balanced salt solution (GBSS; Gibco, BRL) supplemented with 5 g glucose/l (Sigma-Aldrich) and thereafter trypsinised. The dissociated cells were washed in the presence of DNAse 1 and soybean trypsin inhibitor (Sigma-Aldrich).

For the survival assay isolated neurons were seeded at a density of 150,000 cells/cm² in 24-well cell culture plates coated with poly-D-lysine as described above. The neurons were left to differentiate for six days without or with various concentrations of the FGL peptide, after which 6-OHDA was added at a concentration of 100 µM for two hours. A stock solution was prepared by dissolving 6-OHDA to a concentration of 10 mM in 0.1 % (w/v) sodium metabisulfite in order to prevent oxidation. After two hours with 6-OHDA, the medium was changed to Neurobasal medium with supplements and peptide, and the cell cultures were further incubated for 24 hours, fixed and immunostained for tyrosine hydroxylase. 98 images for each experimental condition in each individual experiment were automatically recorded as described for the dopaminergic neurite outgrowth assay below.

### Hippocampal neurons (HN)

For the experiments dissociated hippocampal neurons were isolated from Wistar rat embryos at embryonic day 19 or newborn rats as described by Rønn et al. (1999) Nat Biotechnol. 17:1000-5.

Briefly, hippocampus was isolated from the brain in ice cold modified Krebs Ringer solution, cleared of blood vessels, roughly homogenised by chopping and then trypsinised. The dissociated cells were washed in the presence of DNAse 1 and soybean trypsin inhibitor.

For the assay neurons were plated at a density of 40,000 cells/cm² on 8-well permanox slides coated with 10 µg/ml poly-l-lysine in Neurobasal medium. 15 minutes after plating, Aβ 25-35 was added to a final concentration of 20 µM. The Aβ 25-35 had prior to plating been dissolved in sterile deionized water to a concentration of 3 mM and incubated at 37°C for four days according to Pigino et al. (2001) J Neurosci. 21:834-42. This treatment induces fibrillation of the Aβ-peptide fragment, thereby increasing its neurotoxic activity. The neurons were incubated with Neurobasal medium containing the FGL peptide for 70 hours, fixed with 4 % (v/v) formaldehyde, and stained with Hoechst 33258 for 25 minutes as described by Kruman et al. (1997). Images of 1000-1500 neurons were randomly recorded for each group in each experiment using computer assisted fluorescence microscopy as described for the hippocampal neurite outgrowth assay. Nuclei from dead and live neurons were counted using the software package Prima developed at the Protein Laborator, Copenhagen University, and the fraction of live neurons relative to the total number of neurons was estimated.

### Cerebellar granule neurons (CGN)

Cerebellar granule neurons (CGN) are prepared from postnatal day seven Wistar rats !argely as previously described by Drejer and Schousboe (1989) Neurochem Res. 14:751-4.

For the experiments cerebellar tissue was dissected in modified Krebs-Ringer solution kept on ice, and treated as described for the hippocampal neurons above. All cell cultures were incubated at 37°C in a humidified atmosphere containing 5 % CO₂. All animals were handled in accordance with the national guidelines for animal welfare.

Primary cultures of CGN were plated at a density of 100,000 cells/cm² on poly-L-lysine coated 8-well permanox slides in Neurobasal-A medium (Gibco, BRL) supplemented with 2 % (v/v) B27, 0.5 % (v/v) glutamax, 100 U/ml penicillin, 100 µg/ml streptomycin and KCl, making the final concentration of KCI in the medium 40 mM. 24 hours after plating, cytosine-β-D-arabinofuranoside (Ara-C; Sigma-Aldrich) was added to a final concentration of 10 µM to avoid proliferation of glial cells, after which the neurons were allowed to differentiate for further six days at 37°C. Apoptotic cell death was induced by washing twice and changing the medium to Basal Medium Eagle (BME; Gibco BRL) supplemented with 1 % (v/v) glutamine, 100 U/ml penicillin and 100 µg/ml streptomycin, 3.5 g D-glucose/I and 1 % (v/v) sodium pyruvate (Gibco BRL) together with various concentrations of peptide. Thereby the concentration of potassium in the cultures was reduced to 5 mM KCI. Two days after induction of apoptosis, the cells were fixed with 4 % formaldehyde and stained with Hoechst 33258 as described for the survival assay employing hippocampal neurons.

The assay is performed as described by D'Mello et al., (1993) Proc Natl Acad Sci U S A. 90:10989-93.

Other survival assays using the cultures of CGN.

### 1. PACE assay

Detection of phosphorylated Erk1/2 and Akt relative to the total number of CGN is carried out according to Versteeg et al. (2000) FEBS Lett. 465:69-73.

For the experiments CGN were plated at a density of 290,000 cells/cm² in poly-L-lysine coated 96-well microtiter plates. The neurons were grown in Neurobasal-A medium supplemented with 0.5 % (v/v) glutamax, 100 U/ml penicillin and 100 µg/ml streptomycin. 24 hours after plating, Ara-C was added to the cultures as described above. After 72 hours of incubation, half of the medium was replaced with Neurobasal-A medium containing the peptide to be tested. For the Erk1/2 phosphorylation assay, the cultures were further incubated for 0-90 minutes, centrifuged at 70 g for 8 minutes, fixed with 4 % (v/v) formaldehyde and immunostained using primary phospho-p42-44 rabbit antibodies and peroxidase-conjugated anti-rabbit secondary antibodies. For the detection of phosphorylated Akt, the cultures were further incubated for 10 or 30 minutes, centrifuged at 70 g for 8 minutes, fixed with 4 % (v/v) formaldehyde and immunostained using polyclonal antibodies against Akt phosphorylated at Ser473 and peroxidase-conjugated secondary antibodies. The total number of neurons was in both phosphorylation-assays estimated by staining with crystal violet.

### 2. TUNEL assay

CGN were plated at a density of 60,000 cels/cm² on poly-L-lysine coated 8-well permanox slides. The cultures were grown in Neurobasal-A medium supplemented with 2 % (v/v) B27, 0.5 % (v/v) glutamax, 100 U/ml penicillin, 100 µg/ml streptomycin and 40 mM KCI (final concentration) for six days, and apoptosis was induced as described for the CGN survival assay above. 24 hours after induction of apoptotic cell death, the cultures were fixed and the extent of DNA-fragmentation was measured using the ApoAlert apoptosis detection kit, essentially as described by the manufacturer. Briefly, blunt ends of double-stranded DNA molecules were enzymatically labelled with fluorescein-dUTP, and all the neurons were stained with propidium iodide at a concentration of 750 ng/ml. Images of at least 200 neurons from each group in each experiment were obtained using a BioRad Radiance laser scanning system 2000 coupled to a Nikon Eclipse TE 200 confocal microscope equipped with an oil immersion 60x 1.4 NA objective (Nikon, Tokyo, Japan). The fraction of TUNEL-positive neurons was determined by counting.

### RESULTS

### FGL promotes survival of dopaminergic, hippocampal and cerebellar granule neurons

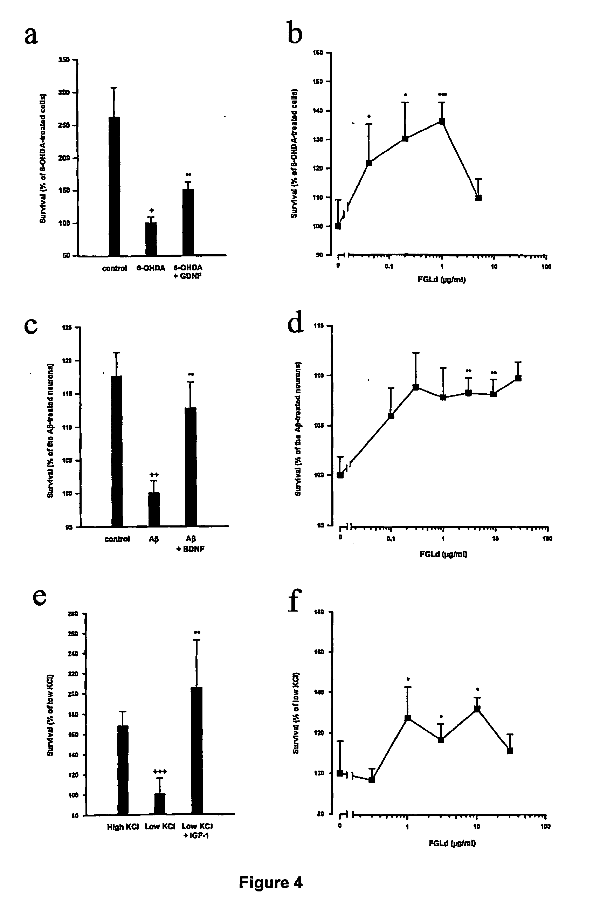

Since FGL is an agoinist of the FGF receptor with a growth factor-like activity, it was investigated whether FGL could act as a neuroprotectant under various neurotoxic conditions. Cell death in dopaminergic neurons was induced by addition of 6-OHDA. In Figure 4a it can be seen that the number of live dopaminergic neurons was strongly decreased after exposure to 6-OHDA as compared to the untreated control. Addition of 10 ng/ml glia derived neurotrophic factor (GDNF) partially prevented this neuronal loss (150 % as compared to the 6-OHDA-treated control cells set to 100 %). When cultures were grown in the presence of various concentrations of FGLd (Figure 4b), survival of dopaminergic neurons treated with 6-OHDA increased statistically significantly with a maximal rescue of approximately 135 % at a concentration of 1 µg/ml FGLd and the effect exhibited a bell-shaped dose-response relationship.

Thus, FGLd is able to rescue dopaminergic neurons to approximately the same extent as GDNF in a survival model employing 6-OHDA as the neurotoxic compound.

It was also tested whether FGLd is able to protect hippocampal neurons from the death induced by Aβ 25-35. Cultures of hippocampal neurons were incubated with pre-aggregated Aβ 25-35 together with various concentrations of FGLd. In Figure 4c it can be seen that treatment of hippocampal cell cultures with 20 µM Aβ 25-35 induced a moderate-low neuronal cell loss. This loss could in part be rescued by 50 ng/ml brain derived neurotrophic factor (BDNF). As shown in Figure 4d, treatment with FGLd in concentrations ranging from 0.1 - 50 µg/ml also partly rescued hippocampal neurons from neurodegeneration induced by 20 µM Aβ 25-35. The FGLd-induced neuroprotection reached a maximal level already at a concentration of 0.3 µg/ml FGLd (110 % as compared to the Aβ 25-35-treated controls), remaining more or less constant at concentrations of FGLd up to 50 µg/ml.

Thus, FGLd can protect hippocampal neurons against the neurotoxic effect of the Aβ 25-35 peptide to the same extent as that achieved by treatment with BDNF.

It has previously shown that if primary cultures of CGN are grown at high levels of KCI, and the concentration of KCI subsequently is reduced, apoptosis is induced. A neuroprotective effect of FGLd was investigated in CGN cultures induced to differentiate in 40 mM KCI for seven days. Subsequently the KCI concentration was reduced to 5 mM, and after two days, cell viability was estimated. As seen in Figure 4e, a low KCI concentration induced cell death in CGN cultures, and this could be rescued by treatment with insulin-like growth factor 1 (IGF-1). As shown in Figure 4f, cell death could also be partially prevented by treatment with FGLd. A maximal neuroprotection of 135 % as compared to controls grown at a low KCl concentration was seen at peptide concentrations between 1-10 µg/ml.

Thus, the results indicate that FGL is capable of promoting survival of dopaminergic, hippocampal and cerebellar granule neurons in vitro.

### FGL protects cerebellar granule neurons against apoptosis

In order further to characterise the mechanism of the neuroprotective effects of FGLd, the extent of DNA-fragmentation (normally associated with apoptosis) in KCl-deprived CGN was measured (as described in Eldadah et al., (2000) J Neurosci. 20:179-86). CGN maintained in the high KCI medium displayed very few neurons with fragmented DNA. A large proportion of the cells treated with low KCI displayed DNA-fragmentation. In CGN cultures treated with FGLd in a concentration of 10-20 µg/ml, very few of the neurons displayed fragmented DNA upon KCI-deprivation. In contrast, the control peptide, FGL9, 10diAlad, with a double alanine substitution exhibited no neuroprotective effect. The neuroprotective effect of FGLd was subsequently quantified, and in Figure 5 it can be seen that KCl-deprivation resulted in a clear increase of the number of CGN containing fragmented DNA as compared to CGN maintained in a high KCl medium. Treatment with the control peptide, FGL9, 10diAlad, did not rescue CGN from apoptosis. Addition of FGLd in various concentrations reduced the number of apoptotic CGN with a maximal rescue of 40 % at concentrations of 10 - 15 µg/ml FGLd, relative to apoptotic neurons without FGLd-treatment.

### FGL-induced signal transduction results in phosphorylation of Erk and Akt

The FGL peptide has been shown to bind and activate the FGFR. Signalling pathways initiated by the FGFR include among others the Ras-MAPK pathway, as reflected by activation of MAPK Erk1/2, and the P13K pathway, which activates the serine/threonine protein kinase Akt. Both pathways are known to be involved in neuronal differentiation and survival. The neurite outgrowth and survival promoting effects of FGL may therefore depend on activation of the MAPK and P13K pathways. **It** was therefore investigated whether Erk1/2 and Akt were phosphorylated in response to treatment of CGN cultures with the FGL peptide. From Figure 8 it appears that the peptide in a concentration of 10 µg/ml induced a sustained phosphorylation of Erk1/2 in contrast to a concentration of 5 µg/ml, which was unable to induce phosphorylation of Erk1/2. This activation (115-120 %, as compared to control cultures) was seen 10 minutes after stimulation with FGLd and lasted for at least 90 minutes.

The phosphorylation of Akt by FGL was analysed after exposure of CGN cultures to the peptide for 10 or 30 minutes (Figure 7). A 10-minute exposure period in concentrations above 0.5 µg/ml led to a significant increase of phosphorylation of Akt as compared to the untreated control. The phosphorylation level reached a maximum at a peptide concentration of 1 µg/ml, and stayed relatively constant at doses increasing up to 20 µg/ml. At exposure times of 30 minutes, no phosphorylation of Akt was detected indicating that activation of Akt was transient and terminated within 30 minutes in contrast to the phosphorylation response of Erk1/2.

Thus, the results indicate that FGL induces a sustained (at least 90 minutes) activation of the MAPK pathway and a transient (10 minutes) activation of Akt.

### FGL promotes neuronal cell survival through FGFR, MEK and PI3K

In order to investigate whether the FGFR, MEK and PI3K also were involved in FGL-induced survival, CGN cultures induced to undergo apoptosis were treated with the above-described inhibitors. As shown in Figure 8, survival of cells in the apoptosis-induced CGN cultures treated with FGLd, but without inhibitors, were set to 100 %, and the apoptosis-induced control neurons not treated with FGLd (marked with a dashed line) had a lower survival rate, as compared to the FGLd-treated neurons. The FGFR inhibitor, SU5402 (■), significantly inhibited the survival response to FGLd already at a concentration of 20 µM and a concentration of 40 µM SU5402 reduced the FGLd-induced survival to the survival rate of the untreated apoptosis-induced control neurons. The MEK inhibitor, PD98059 (●), inhibited cell survival induced by FGLd to the level of the control cultures at a concentration of 12.5 µM. The PI3K inhibitor LY294002 (▲) inhibited FGLd-induced cell survival significantly at a concentration of 3.5 µM, indicating that LY294002 is a very potent and general inhibitor of neuronal survival.

Thus, the FGL-induced survival after KCl-deprivation of CGN cultures is dependent on activation of the FGFR and of the intracellular kinases MEK and PI3K. The results show that LY294002 is the most potent inhibitor of neuronal survival, followed by the MEK inhibitor, PD98059, which appeared to be less efficient in the neurite outgrowth assay than in the survival assay (see below), possibly indicating that the main functions of the FGL-induced MEK activation is neuroprotection, rather than differentiation.

### Example 3. In vitro neurite outgrowth stimulation by different formulations of FGL

Primary cultures of DN, HN and CGN were prepared as described above.

### Dopaminergic neurons (DN)

DN were plated at a density of 100,000 cells/cm² in 24-well cell culture plates (previously coated with 12.5 µg/ml poly-D-lysine; Sigma-Aldrich) in a medium containing 50 % (v/v) Optimem 1 (Gibco, BRL), 25 % (v/v) horse serum (Gibco, BRL), 25 % (v/v) HBSS and 5 g glucose/l. The cells were left to adhere for one-two hours, before the medium was changed to Neurobasal medium supplemented with 2 % (v/v) B27 Neurobasal Supplement, 0.5 % (v/v) glutamine, 100 U/ml penicillin and 100 µg/ml streptomycin (all from Gibco, BRL) without or with various concentrations of peptide. After 72 hours of incubation, the neurons were fixed with 4 % (v/v) formaldehyde and immunostained using a primary mouse monoclonal antibody against tyrosine hydroxylase and secondary biotinylated sheep anti-mouse antibodies followed by incubation with peroxidase conjugated streptavidin. 98 images for each experimental condition in each individual experiment were automatically recorded using a computerised microscope workstation described by Walmod et al. (2002) Toxicol Appl Pharmacol. 181:1-15. Briefly, the workstation consisted of an Eclipse TE 300 inverted microscope (Nikon, Tokyo, Japan) equipped with a motorised, movable microscope stage (LUDL Electronic Production Ltd, Hawthorne, NY, USA), a 10x objective and a 1100 analogue b/w CCD video camera (DFA, Denmark). A software package Prima developed at the Protein Laboratory (Copenhagen University, Denmark) was used to make a stereologically based determination of neurite length according to Rønn et al., (2000) J Neurosci Methods. 100:25-32).

### Hippocampal neurons (HN) and cerebellar granule neurons (CGN)

Postnatal HN or CGN were plated at a density of 10,000 cells/cm² on uncoated 8-well permanox Lab-Tek chamber slides in Neurobasal medium supplemented with 0.4 % (w/v) bovine serum albumin (BSA; Sigma-Aldrich), 2 % (v/v) B27 Neurobasal supplement, 1 % (v/v) glutamax, 100 U/ml penicillin, 100 µg/ml streptomycin and 2 % 1 M HEPES (all from Gibco, BRL). Peptide solutions without or with inhibitors of various signal transduction pathways were added to a total volume of 300 µl/cm², and the slides were incubated at 37°C. After 24 hours, the neurons were fixed with 4 % (v/v) formaldehyde for 20 minutes and thereafter immunostained using primary rabbit antibodies against GAP-43 and Alexa Fluor secondary goat anti-rabbit antibodies. Images of at least. 200 neurons for each group in each individual experiment were obtained systematically by using computer assisted fluorescence microscopy as previously described (Ronn et al., 2000 op. cit.). Briefly, a Nikon Diaphot inverted microscope with a Nikon Plan 20x objective (Nikon, Tokyo, Japan) coupled to a video camera (Grundig Electronics, Germany) was used for recordings. The same software package as described above for the dopaminergic neurite outgrowth assay was used to process the recorded images.

### Results

### FGL induces neurite outgrowth in primary cultures of dopaminergric, hippocampal and cerebellar granule neurons

A monomeric form of FGL has been demonstrated to induce neurite outgrowth from primary hippocampal neurons. It is known that multimeric forms of peptide ligands have a higher potency for receptor activation than monomeric forms (discussed above). Therefore, various dimeric and tetrameric forms of FGL were manufactured and tested on their neurite outgrowth stimulatory capability employing primary cultures of dopaminergic, hippocampal and cerebellar granule neurons (CGN).

The cultures were grown in the absence or presence of the tetrameric (dendrimeric) FGL peptide (FGLd). Neurite outgrowth induced by FGLd in all three types of neurons was subsequently quantified and shown in Figure 9. Dopaminergic neurons treated with FGLd (●) exhibited a statistically significant increase in neurite length in a dose-dependent manner with a maximal average neurite length (125 % as compared to the untreated control) obtained at a concentration of 1 µg/ml FGLd. Hippocampal neurons treated with FGLd (▲) exhibited an increase in neurite length already at a dose of 0.04 µg/ml FGLd, reaching a plateau-like level with a stimulation of approximately 150 % as compared to the untreated control cultures employing concentrations of FGLd up to 20 µg/ml. CGN cultures treated with FGLd (■) also exhibited a dose-dependent neurite outgrowth response. A maximal stimulation of 255 % as compared to the untreated control was seen at a concentration of 50 µg/ml FGLd.

Primary cultures of hippocampal neurons were selected to test the effect of three different dimeric versions of the FGL peptide, FGL_{L}, FGL_{lys} and FGL_{cys}, on neurite outgrowth. As shown in Figure 10, the dimeric lysine dendrimers, FGL_{lys}, (▲) and FGL_{cys} (●) were both unable to stimulate neurite outgrowth at any of the concentrations tested. In contrast, FGL_{L} (■) induced a significant neurite outgrowth response exhibiting a dose-dependent relation with a bell-shaped curve reaching a maximal stimulation at a concentration of 1 µg/ml. The maximal stimulation was 155 % as compared to the untreated control (Figure 10), which is roughly the same as the response induced by FGLd (see Figure 9).

### Neurite outgrowth induced by FGL involves activation of FGFR, MEK and PI3K

In order to elucidate the biological correlates of activation of FGFR, MEK and PI3K, it was tested the effect of inhibitors of these kinases on neurite outgrowth induced by FGLd in CGN. The FGFR inhibitor, SU5402, is known to inhibit the tyrosine kinase activity of the FGFR subtype 1 by interacting with the catalytic domain of the receptor. In Figure 11 the average neurite length in FGLd-treated CGN cultures is set at 100 % and the average neurite length for control neurons not treated with FGLd is marked with a dashed line. It appears that SU5402 (■) significantly inhibited the FGLd-induced neurite outgrowth at a concentration of 80 µM (to 35 % of the initial value). Treatment with the MEK inhibitor PD98059 (●) in concentrations above 25 µM statistically significantly decreased neurite outgrowth induced by FGLd to 75 %. Also, the PI3K inhibitor LY294002 (▲) significantly inhibited FGLd-induced neurite outgrowth already at a concentration of 3.5 µM to 90 % and at a concentration of 10 µM LY294002 the neurite outgrowth response to FGLd was reduced to almost 50 %, which is lower than for the untreated control neurons. SU5402 and LY294002 where without significant effect on neurite outgrowth in the control cultures not treated with FGLd (data not shown). PD98059 has previously been found not to affect the basal neurite outgrowth in CGN. Thus, the FGLd-induced neurite outgrowth response of CGN is dependent on the activation of the FGFR and the subsequent activation of the MAPK and the PI3K intracellular signalling pathways.

**Table 1. Summary of the effects of different formulations of the FGL peptide on neuronal survival In primary cultures of rat CGN, DN and HN Induced to undergo cell death by withdrawal of high potassium, or addition of 6-hydroxy dopamine (6-OHDA) or (25-35) β-amylold peptide fragment.**

| Mechanism for inducing cell death | Tissue source (age of rat) | FGL peptide/effective concentration | Neuronal Survival (% of Control)^{a} |
|---|---|---|---|
| Low K⁺ | CGN | *FGLₘ:* | |
| (5 mM) | (PND 3-4) | 250 µg/mL | 125%* |
| Low K⁺ | CGN | *FGL_{d}:* | |
| (5 mM) | (PND 3-4) | 5 µg/mL | 110%* |
| | | 20 µg/mL | 117%** |
| Low K⁺ | CGN | *FGL_{d}:* | |
| (5 mM) | (PND 3-4) | 1 µg/mL | 131%* |
| | | 10 µg/mL | 132%* |
| Low K⁺ | CGN | *FGLₘ:* | |
| (5 mM) | (PND 7-8) | 10 µg/mL | 110%* |
| | | 50 µg/mL | 119%* |
| | | 250 µg/mL | 142%* |
| Low K⁺ | CGN | *FGL_{d}:* | |
| (5 mM) | (PND 7-8) | 20 µg/mL | 175%* |
| 6-OHDA | DA | *FGL_{d}:* | |
| | (E14) | 1 µg/mL | 143%* |
| β-amylold | Hippocampal | *FGL_{d}:* | |
| | (E19) | 0.01 µg/mL | 109%** |
| | | 3 µg/mL | 107%** |
| | | 9 µg/mL | 107%** |
| β-amyloid | Hippocampal | *FGL_{L}*: | |
| | (E19) | 0.3 µg/mL | 108%* |

| | | | |
|---|---|---|---|
| ^{a}Survival in cultures induced to undergo cell death without FGL treatment is designated as 100% (control) and compared to cultures where FGL was added. Statistics (+FGL vs. control treatment): *p<0.05; **p<0.01, ***p<0.001 | | | |

**Table 2. Summary of the effects of FGL peptides on neurite outgrowth in primary cultures of rat CGN, DN and HN.**

| Assay | Tissue Source/ Age of Rat | Peptide Concentration (µg/mL) | Effect on Neurite Length (Percent Increase vs. Control) |
|---|---|---|---|
| Primary culture; | CGN/ | | |
| Incubation: 24 h +/ FGL; - | PND 2-4 | FGL_{d} - 9 | 24%** |
| | - PND 5-8 | FGL_{d} - 3, 9, 27 | 30%**, 93%**, 121%* |
| Analysis: computer assisted fluorescence microscopy | | FGL₄ -10, 25 50, 100 | 37%*, 79%*, 179%**, 98%** |
| Primary culture; | DN/ | | |
| Incubation: 72 h +/- FGL; | E14 | FGL_{d} - 0.04, 0.2, 1, 5 | 12%***, 23%***, 24%***, 13%* |
| Analysis: computer assisted fluorescence microscopy | | FGL_{L} - 0.3, 1,3 | 19%*, 34%**, 33%** |
| | | FGLₘ-10, 50, 250 | 15%*, 23%***, 28%*** |
| Primary culture; | HN/ | | |
| Incubation: 24 h +/- FGL; | - E19 | FGL_{d} - 3, 9, 27 | 65%*, 51%*, 69%* |
| | - Newborn | FGL_{d} - 0.04,5 | 38%-,45%* |
| Analysis: computer assisted fluorescence microscopy | - Newborn | FGL_{L} - 0.2, 1,5 | 35%** 53%** 30%** |

| | | | |
|---|---|---|---|
| Statistics (+FGL vs. Control): *p<0.05; **p<0.01; ***p<0.001 PND = Post-natal day; E = Embryonic day | | | |

### Conclusions

1. The FGL peptide is a survival promoting compound for a variety of neuronal cell types in vitro, which is essential in the treatment of neuro-degenerative disorders.
2. The FGL peptide is capable of inducing neurite outgrowth in a variety of neuronal cell types in vitro and FGL has a potential to promote the growth of both axons and dendrites, which is essential in the treatment of neuro-degenerative disorders.
3. All, monomeric FGLₘ, dimeric FGL_{L} and dendrimeric FGL_{D}, formulations of the FGL peptide are potent in promotion of both survival and neurite outgrowth.
4. FGL_{L} and FGL_{D} both are more then 200 times more potent then FGLₘ in promotion of neurite outgrowth.
5. FGL_{L} is as potent in promotion of both neurite outgrowth and survival as FGL_{D}.
6. Other formulations of the FGL peptide such as dimers FGL_{lys} and FGL_{cys} are inactive in promotion of neurite outgrowth.

### Example 4. The effect of FGL on synaptic plasticity of primary cell cultures of rat hippocampal neurons

In the adult nervous system, structures such as the hippocampus continuously undergo plastic changes to adjust structure and function. In particular, the acquisition of memory is believed to require structural and functional changes of already established neuronal connections. NCAM has been shown to play an important role in neuronal plasticity during development and in learning and memory (Welzl and Stork, (2003) News Physiol Sci. 18:147-50); however, the mechanism for these effects is unknown. The present study it was investigated whether the FGL peptide is able to affect synaptic plasticity.

The effects of FGL_{d} on vesicle turnover was estimated in primary cultures of hippocampal neurons from embryonic rats (E19). The cultures were prepared as above. Following 13-16 days in vitro, FGL_{d} was added to the cultures and incubated for 1, 24 or 48 hours. After 48 hours of incubation, the FGFR-inhibitor (SU 5402) was added simultaneously with FGL_{d} to some of the cultures.

After treatment with FGL_{d} the neurons were labeled with the fluorescent membrane probe FM 1-43 as described by Kiryushko et al (2003) J Biol Chem. 278:12325-34. The rate of FM 1-43 release (destaining of cells) evoked by depolarisation with 90 mM potassium was then evaluated by a time-lapse image acquisition using the Radiance 2000 scanning system and a Nikon eclipse TE200 confocal microscopy.

The effect of FGL_{d} on synapse formation was further evaluated using double-immunostaining of neurites and synapses in primary embryonic (E19) hippocampal neurons grown in culture for 13 days. Cells were incubated with FGL_{d} for 48 or 96 hours, before fixation and immunostaining. The synapses were detected by antibodies against synaptophysin and the neyrites were labelled by antibodies against the growth-associated protein-43 (GAP-43- also called B-50 or F1). The immunostaining was visualised and quantified by confocal fluorescence microscopy as described by Rønn et al. (2000) (op. cit.).

### Results

The treatment of hippocampal neurons with 20 µg/ml FGL_{d} for 1 h and 48 h resulted in a statistically significant increase of the rate of FM 1-43 destaining, compared with the control (from 0.008 sec⁻¹ to 0.016 sec⁻¹, and from 0.013 sec⁻¹ to 0.028 sec⁻¹, respectively); whereas 24 h treatment did not result in a significant effect (Figure 12). This suggests that FGL_{d} causes both a short-term facilitation of transmitter release (1 h) and a long-term (48 h) increase of synaptic efficiency. The FGFR inhibitor SU5402 abolished the effect of FGL_{d} indicating that the effect of FGL_{d} was mediated through its interactions with FGFR.

The double immunostaining of hippocampal neurons for synaptophysin and GAP-43 showed that FGL_{d} (20 µg/ml) administered to hippocampal cultures for 48 and 96 hours significantly increased the number of synapses, as reflected by approximately 20% increase in the number of synaptophysin positive spots per 100 µm length of neurite (from 2.40 to 2.96 and from 2.73 to 3.24, respectively).

These results suggest that the FGL peptide influences synaptic plasticity by increasing the rate of presynaptic vesicle turnover and the number of the synapses.

### Example 5. The effect of FGL on organotypic hippocampal neuronal slice cultures and primary cultures of dissociated hippocampal neurons exposed to ischemic conditions

The brain has a high consumption of oxygen and glucose and depends almost exclusively on oxidative phosphorylation for energy production. Deficiency of oxygen and glucose supply results in neuronal cell damage and death. The susceptibility to ischemic injury differs among different parts of the brain and even different neuronal populations. The neurons of the hippocampal CA1 area are one of the regions in the brain of high vulnerability.

### Methods

In the present study it was investigated the effect of the FGL peptide on synaptic plasticity, neuronal-function and vulnerability following oxygen and glucose deprivation (OGD) in organotypic hippocampal neuronal slice cultures (OHSC) from 7-day-old Wistar rats according to method of Stoppini et al. (1991) Neurosci Methods. 37:173-82 and primary cultures of dissociated hippocampal neurons from 1-day-old rats according to method of Maar et al.(1997) J Neurosci Res. 47:163-72. Primary cell cultures were maintained for 11-12 days and OHSC for 12-14 days prior to adding FGL_{d}.

The primary cell cultures and OHSC were exposed to OGD for 10 and 20 minutes. Following OGD, the cultures were again supplied with oxygen and glucose and analysed at 1, 4 or 24 hour time point after introduction of OGD exposure. FGL_{d} (or the control peptide FGL_{c}) was added 24 hr before, immediately, or 1 hr or 24 hr after OGD. When administered prior to OGD, FGL_{d} was removed from the medium when introducing OGD. In some cases, the slice cultures were treated with FGL_{d} only at 4 hr after OGD. In addition, the FGFR inhibitor, SU5402 (25 µM), was added together with FGL_{d} in some of the set-ups. In hippocampal neuronal cultures, FM 1-43 staining was used to evaluate pre-synaptic function (described above). Cell viability in both primary cell cultures and OHSC was measured using the MTS [3-(4,5-dimethylthiazol-2-yl)-2,5-diphenyltetrazolium bromide] assay according to Malich et al, (1997) Toxicology. 124:179-92. In, addition, cell viability in OHSC was measured using propidium iodide (PI) staining according to Laake et al. (1999) Brain Res Brain Res Protoc. 4:173-84.

### Results

The metabolic activity of dissociated hippocampal cell cultures was significantly reduced after 1 hour (31%) and 4 hours (30%) following 20 min of OGD, compared to control cultures, as measured by the MTS assay. When cell cultures were treated with FGL_{d} (24 hours before or immediately after OGD) the effect of OGD on metabolic activity was diminished. The metabolic activity in OHSC showed a different response to OGD. One hour after OGD, the metabolic activity was significantly increased (19%) compared to control, while a longer period of reoxygenation (24 hours) resulted in a decrease (24%) of metabolic activity. At both time-points, treatment with FGL_{d} 24 hours prior to OGD caused a normalization of the metabolic activity in the hippocampal slices, compared to the control. In both the dissociated cell cultures and OHSC, FGL_{d} treatment alone led to an increase in metabolic activity. In addition, pretreatment with FGL_{d} for 24 hours did not diminish the OGD-induced reduction in metabolic activity if the FGFR-inhibitor SU 5402 was present together with FGL_{d}, indicating that FGL_{d} might influence metabolic activity through FGFR activation.

In dissociated hippocampal cell cultures, the rate of pre-synaptic vesicle release (FM 1-43 destaining) upon high potassium stimulation was statistically significantly enhanced (from 0.04 sec⁻¹ to 0.06 sec⁻¹) by FGL_{d} treatment and decreased after OGD. when analyzed at 1 h and 4 h (both from 0.04 sec⁻¹ to 0.02 sec⁻¹). If cell cultures were treated with FGL_{d} either 24 hours before or immediately after OGD, the OGD-induced decrease in the rate of FM 1-43 release was diminished. Furthermore, the FGFR-inhibitor SU 5402 abrogated the effect of FGL_{d} on the OGD-induced decrease in FM 1-43 release.

In OHSC, 10 minutes of OGD induced delayed cell damage as estimated by propidium iodide (PI) staining. There was a significant increase in the PI staining of OHSC (% of total CA1 area) at both 4 hours (0.3% to 5.0%) and 24 hours (0.6% to 23.1 %) after OGD. This effect could be almost completely abolished by pre-treating the slices for 24 hours with FGL_{d}. Cell damage was also avoided by treating slices with FGL_{d} immediately after OGD, but not if FGL_{d} treatment was delayed until 4 hours after OGD. The control peptide FGL_{c} did not affect cell damage induced by OGD, and if slices were pre-incubated with the FGFR-inhibitor SU 5402 together with FGL_{d}, the PI staining was similar to that of OGD alone.

### Discussion

The FGL_{d} peptide has neuroprotective effects when applied 24 hours before or immediately after a transient deprivation of oxygen and glucose in two different *in vitro* model systems. The OGD caused a reduction in metabolic activity in dissociated hippocampal cell cultures and in OHSC, and in both cases treatment with FGL_{d} could partially or completely normalize metabolic activity after OGD.

Ischemia has been reported to cause either enhanced synaptic transmission or reduced synaptic transmission, depending on the model used and the severity of the ischemic challenge. In the model used in this study, 20 minutes of OGD caused a reduction in presynaptic vesicle release upon potassium stimulation, and this reduction was attenuated by administering the FGL_{d} peptide either 24 hours before or immediately after OGD.

Thus, for all three parameters investigated (metabolic activity, presynaptic vesicle release and cell viability), the FGL peptide could partially or completely inhibit the effects of OGD. Furthermore, this neuroprotective ability of FGL could in all three cases be inhibited by the FGFR-inhibitor SU 5402, indicating that FGFR activity is necessary for the action of FGL.

### Example 6. Effect of FGL on social behaviour of rats and β-amyloid Induced neurotoxicity in vivo.

### Description of the model

The present study employed a rat model, in which neurotoxicity and cognitive impairment were induced by the (25-35) β-amyloid peptide fragment (see Maurice T., et al. (1996) (Brain Res. 706:181-93) and Delobette S., et al. (1997) Eur J Pharmacol.319:1-4). Short-term memory deficit induced by the (25-35) β-amyloid peptide fragment in rat is of special importance to study, as it resembles one of the clinical manifestations of Alzheimer's disease. Short-term memory deficit in rats was evaluated using three different behavioural tests: the Social Recognition (Kogan et al, (2000) Hippocampus 10:47-56), Open Field (Cerbone and Sadile, (1994) Neurosci Biobehav Rev. 18:497-518.; Vianna et al, (2000) Learn Mem. 7:333-40) and Y-maze (Clayton & Williams, (2000) Neurobiol Learn Mem. 74:135-45) tests. In addition, neuropathological investigations were performed on brain tissue collected at the time of sacrifice. The FGL peptide was administered either sub-occipitally or intranasally.

### Methods

The timeline of a representative social-recognition study with early treatment is schematically shown in Figure 26 (A).

Following an acclimatisation period of five days, rats were treated with the (25-35) β-amyloid peptide fragment (A-β) (25 µg per animal), which was injected i.c.v. in the right lateral ventricle of the brain (day 0).

The FGL peptide (either FGL_{L} or FGL_{d}) was administered sub-occipitally (5µg per animal per administration) or intranasally (400 µg per animal per administration) at days 7, 10 and 13 or at days 30, 33 and 36. Behaviour of animals was evaluated using the Social Recognition test, the Open Field test or the Light-dark Discriminating Y-maze. On day 28-30 or day 51 after i.c.v. administration of the (25-35) β-amyloid fragment, the animals were sacrificed, and in selected studies, the brain tissue was collected, histological sections were immunostained with A-β specific antibodies and the amytoid-burden was calculated as a percentage of the A-β positive area. In addition, the number of neurons in selected areas of brain-sections was calculated using a stereological approach.

### Effects of (25-35) A-β/Vehicle-administration on social behaviour and memory

In the Social Recognition test, an increase in the time spent exploring a juvenile rat during the second trial was detected in rats that received the (25-35) β-amyloid fragment as compared to the control animals receiving vehicle (V). In the Open Field test, (25-35) β-amyloid fragment-treated rats did not decrease exploratory activity during a 20-minute session, in contrast to the decrease noted in the group of control animals. In the Light-dark Discriminating Y-maze with positive food reinforcement, rats treated with the (25-35) β-amyloid fragment demonstrated an increase in the number of errors (entries into the wrong arms).

Thus, in all three tests, i.c.v. administration of the (25-35) β-amyloid fragment resulted in a cognitive deficit displayed as short-term memory impairments.

### Effects of FGL administration on behaviour and memory of rats in which cognitive impairment was induced by i.c.v.-administration of the (25-35) β-amyloid fragment

The effects of FGL were estimated in different tests in vivo described below in several independent studies.

### Social Recognition test and Neuro-histopathology

o Study 1: FGL was administered sub-occipitally at days 7, 10 and 13 following i.c.v. administration of the (25-35) β-amyloid fragment. No effect of FGL_{d} was observed in the Social Recognition test at day 14, the day after the last administration of FGL_{d} and no significant effect was observed in the neuro-histopathology evaluation of the CA3 area of hippocampus comparing placebo-treated rats with rats receiving either the (25-35) β-amyloid fragment alone or with FGL_{d}.
o Study 2: FGL was administered sub-occipitally at days 7, 10 and 13 following i.c.v. administration of the (25-35) β-amyloid fragment. Administering the (25-35) β-amyloid fragment resulted in a statistically significant increase in the time spent by the rat exploring a juvenile animal at a second meeting as well as in the amyloid burden and neuronal cell death when compared to control rats. However, treatment with FGL_{L} and FGL_{d} significantly reduced the increased time spent by the treated animal exploring a juvenile rat during the second trial, when compared to the animals treated only with the β-amyloid peptide fragment. Treatment with FGL_{L} and FGL_{d} also significantly reduced the amyloid burden in the cingulate cortex (from 100% to 39% & 42%, respectively) and in the CA3 area of the hippocampus (from 100% to 17% & 44%, respectively). In addition, FGL_{L} and FGL_{d} caused an increase in the density of neurons (from 15 to 21 & 22 neurons/mcm²x10⁻⁴, respectively) in the CA3 zone of the hippocampus, when compared to the group of rats treated only with the (25-35) β-amyloid fragment. The results are graphically presented in Figure 13-16.
o Study 3: FGL_{L} was administered sub-occipitally at days 30, 33 and 36 following i.c.v. administration of the (25-35) β-amyloid fragment. A statistically significant reduction was observed at day 44, one week after the last administration of FGL_{L}, in the ratio of the time the rats spent investigating a novel object (juvenile rat) on the second exposure compared to the (25-35) A-β/Vehicle treated control rats (from 0.50 to 0.41), when FGL_{L} was administered sub-occipitally. In addition, neuropathology investigations showed a statistically significant reduction in amyloid burden (from 100% to 24%) in the cingulate cortex and the CA3 area of Hippocampus (from 100% to 29%). In addition, a statistically significant decrease in the percentage of damaged neurons (from 27 to 21 neurons/mcm²x10⁻⁴) was observed in the cingulate cortex, compared to the group of rats treated only with the (25-35) β-amyloid fragment. The results are graphically presented in Figures 17-20.
o Study 4: FGL_{L} was administered intranasally at days 7, 10 and 13 following i.c.v. administration of the (25-35) β-amyloid fragment. Treatment with the Amyloid beta peptide induced a short term memory deficit, which was abrogated by treatment with FGL-peptides. In the cingulate cortex, a statistically significant increase in the neuronal density (from 11 to 14 µm²x 10⁻⁴) was observed in response to FGL_{L} administration, compared with the group of rats treated only with the (25-35) β-amyloid fragment. The results are graphically presented in Figures 21-23.

### Open Field study

The rats that received FGL_{d} administered sub-occipitally at days 7, 10 and 13, following i.c.v.-administration of the (25-35) β-amyloid fragment, showed a statistically significant decrease in the time spent exploring holes (from 3.3 s to 0.1 s) and rearing (from 7.0 s to 0.7 s) in the last measurement period, compared with the first measurement period at day 14, the day after the last administration of FGL. The group of rats treated with only the (25-35) β-amyloid fragment showed no statistically significant difference between the two measurement periods. The locomotion activity was unaffected at day 14.

### Light-dark Discriminating Y-maze study with positive food-reinforcement

The rats that received FGL_{d} administered sub-occipitally showed a statistically significant reduction (from 3.8 to 1.7) in the number of errors at day 25, the last day of training, while no significant differences were observed at training days 21-24 in comparison with the (25-35) β-amyloid fragment-treated control rats.

### Discussion

The model used in this study, the (25-35) β-amyloid fragment induced neuro-toxicity, reflects many aspects of neuro-pathology and cognitive impairment seen in Alzheimer's patients. In this model, i.c.v.-administration of the (25-35) β-amyloid peptide fragment results in deposition of the β-amyloid peptide, starting at day 14 after the (25-35) β-amyloid peptide fragment administration, which lead to neuronal cell death and cognitive impairment (short-term memory deficit).

The experiments of early administration of the FGL peptide (at days 7, 10 and 13 after the i.c.v.-injection of the (25-35) β-amyloid fragment) clearly demonstrate that both sub-occipital and intranasal administration of FGL results in prevention of short-term memory deficit, a decrease of β-amyloid burden in the various brain areas and a decrease in neuronal cell death. The administration of the FGL peptide at a later stage, at days 30, 33 and 36, after i.c.v.-injection of the (25-35) β-amyloid peptide fragment ameliorated both the neuropathological changes in the brain tissue and the memory deficit.

The important aspect of these studies was to compare the effectiveness of different routes of FGL administration. The results obtained clearly indicate that the intranasal administration of the FGL peptide effectively reduced the memory deficit induced by treatment with the (25-35) β-amyloid peptide fragment, although the potency of the FGL peptide administered intranasally was much lower, compared to the potency of the peptide administered sub-occipitally. It should be noted that the effect of intransally administered FGL on memory was dose-dependent.

In conclusion, taken together, these results indicate that the FGL peptide has the potential to reduce neuropathology and to reduce the memory deficits induced by treatment with the (25-35) β-amyloid peptide fragment. These findings also indicate that the FGL peptide has a therapeutic potential for the treatment of cognitive deficits associated with β-amyloid deposition in patients with Alzheimer's Disease.

### Example 7. Sensory Motor Control Development in Rat Pups

The neonatal CNS of immature rodents is a useful model for studying drugs influencing the maturation of brain functions. The maturation of postural adjustment (locomotion) needs integration of motor and sensory systems. The maturation of the postural and locomotor functions during the first post-natal week is connected with the growth of the descending pathways from brainstem and cortico-spinal tracts, reaching the thoracic levels at post-natal day (PND) 3 and the lumbar level at PND 6. Thus, the period between PND 3 and PND 6 can be considered as a period sensitive to the effects of agents able to modify post-natal integration of the sensory-motor system. A number of motor control tests are available, which can be used for determining the effect of neuro-active drugs such as FGL. In the present study, the Surface Righting Response performance (Tilney, 1933), the Pivoting Locomotion performance (Altman et al, (1975) Anim Behav.;23:896-920), the Negative Geotaxis performance [Henck et al, (2001) Toxicol Sci. 62:80-91), and the Forepaw Suspension performance [Kimler et al, (1998) Brain Res Dev Brain Res. 107:49-55) of rat pups were chosen to evaluate the effect of FGL on sensory motor control development. The time-course of the experiments is schematically presented in Figure 26 (B).

*Dose-Response study.* The effect of intranasal administration of 0.026, 0.26 and 2.6 µg FGL_{d} per day at post-natal day (PND) 1, 2 and 3 on performance of the Surface Righting Reflex by rat pups was evaluated. A total of five litters, each consisting of 10 pups (five males and five females) were used in this study.

Administration of FGL_{d} significantly improved performance of the Surface Righting Reflex at PND 5 (from 3.4 s to 1.3 s) administered at a concentration of 2.6 µg per pup per administration compared to the vehicle-treated control animals.

*Sensory Motor Control Development study.* The effect of FGL on sensory motor control development (Surface Righting Reflex, Negative Geotaxis Reflex, Pivoting Locomotion and Forepaw Suspension performances) of rat pups receiving 2.6 µg peptide intranasally daily at PND 1, 2 and 3 was evaluated. A total of six litters, each consisting of 10 pups (five males and five females), were used in the study.

Administration of FGL_{L} statistically significantly improved performance of the Surface Righting reflex at PND 4 (from 2.1 s to 1.6 s) (Figure 24) and of the Negative Geotaxis Reflex at PND 6 (from 56.7% to 80.0%) (Figure 25), compared with the vehicle-treated control rats, without affecting the Pivoting Locomotor activity or the Forepaw Suspension performance. This indicates that FGL promotes the post-natal sensory-motor control development, without disturbing other functions. In addition, no significant effect on Negative Geotaxis performance was observed at PND 9, suggesting that the motor control development was already completed by PND 9.

### Discussion

The present results demonstrate an effect of the FGL peptide in modulation of the initial processes of CNS plasticity during post-natal development, and suggest that the FGL peptide, by stimulating the FGF receptor, may have a potential to accelerate the development of sensori-motor functions.

### Example 8. The Contextual Fear Conditioning Test and the Morris Water Maze Test

The Contextual Fear Conditioning test (CFC) test (Cordero et al, (2003) Horm Behav. 2003 Nov;44(4):338-45) and the Morris Water Maze (MWM) test [Morris R., (1984) J Neurosci Methods. 11:47-60) are the tasks that assess learning and memory, and are used to compare the performance of normal control rats and of rats treated with drugs.

In the Contextual Fear Conditioning test, rats experienced inescapable shocks when exposed to a *new environment, which results in the development of a conditioned fear response, consisting of a characteristic immobility or 'freezing response', when subsequently re-exposed to the same environment.

In the Morris Water Maze test, rats are evaluated in the spatial training task to find a hidden platform in order to escape swimming in the water.

The present study investigated whether post-training administration of the FGL_{d} peptide is effective in modulating the memory consolidation in aversive and/or new situations.

### The Contextual Fear Conditioning test

The rats were handled for 120 seconds every day for 3 days preceding the first day of the experiment. On day 1 (training), the rats received a 1-second shock (unconditioned stimuli) each minute for two minutes. Animals were injected immediately after training with 5 µl FGL_{d} (5 µg), the control peptide (5 µg) or the vehicle. Each treatment group consisted of 11-14 rats. In total, 39 animals were evaluated. Animals were tested on days 2, 7 and 30 by placing them in the chamber used for conditioning, but in the absence of a shock, for 8 minutes. Each rat was rated as either "freezing" or "active", every 2 seconds, using a time-sampling procedure.

The time-course of the experiments is schematically presented in Figure 26 (C).

A statistically significant increase in percentage of freezing time after FGL_{d} administration was detected when animals were tested on day 2, (from 50.5% to 73.5%) and day 7 (from 30.0% to 60.3%) after conditioning compared to rats receiving placebo or the control. This indicates that FGL_{d} is capable of improving the long-term retention of the conditioned fear response when administered post-training in adult male Wistar rats.

### The Morris Water Maze test

In the Morris Water Maze study, rats were pre-trained to locate a hidden platform by daily training for 120 seconds for five days (days -4 to 0) before the experiment. On days 1 and 2, rats were given 3 consecutive trials in which the platform location remained constant. The time during which the rats were able to locate the platform was recorded within a maximum test-period of 120 seconds. The rats received a 5 µl injection of the FGL_{d} peptide (5 µg), the control peptide (5 µg), or the vehicle solution immediately after each training trial on days 1 and 2.

The animals were tested 24 hours (day 3) and 7 days (day 10) after training. Fourteen days after training (day 17), the location of the platform was changed. Rats were trained to find a new location of the platform in three consecutive trials ("rever sal learning"). In total, 48 animals were evaluated. Animals were divided into three groups consisting of 12-18 rats per treatment group.

Administering FGL_{d} improved consolidation of long-term memory in rats as evaluated in the Morris Water Maze test. At day 2, the day after the first administration of FGL_{d}, FGL_{c} or vehicle, the FGL_{d}-treated rats were able to locate the platform statistically significantly faster (65.8 s) than the untreated rats (93.0 s). The effect was also statistically significant at day 17, suggesting an improved learning ability.

The time-course of the experiment is schematically presented in Figure 26 (D).

Additional experiments in the Open Field test showed that FGL_{d} did not influence emotional or locomotor behaviour in rats.

### Discussion

The results of the CFC/MWM studies demonstrated a long-term effect of FGL_{d} on learning and memory of normal rats. FGL may be acting by triggering signalling cascades involved in long-term memory formation. As it is shown above, FGL can modulate synaptic plasticity acting through the FGFR, which results in an increased pre-synaptic vesicle turnover. Together, these results suggest that FGL has a very good therapeutic potential in the treatment of patients with learning and memory impairment.

### Example 9. In vivo pharmaco-kinetics of FGL_{L}: Estimation of FGL_{L} in plasma and CSF

The levels of the administered FGL_{L} in rat and dog plasma and CSF were assessed by the radio-immuno assay.

### Assay

a) Rat and dog plasma samples contained lithium heparin as an anti-coagulant.
b) FGL_{L} was iodinated. Iodination was done by using the lodogen method. The iodinated peptide was purified from unincorporated I¹²⁵ on C18 column.
c) The measurements were done in replicates.
   1. Plasma (or CSF) sample (20 µl) was added to EDTA containing tubes (n=2). Non-specific binding (NSB) tubes containing non-immune rabbit serum (or CSF from control animals) (20 µl) were used as control.
   2. Diluted radiolabelled FGL_{L} (100 µl; approximately 20,000 cpm/tube of tracer) was added. Control tubes containing the tracer only were also prepared.
   3. Diluted antisera sample (1: 20,000 for PAb268; 100 µl) was added to all tubes (except the control tubes. The contents were vortex mixed.
   4. The tubes were incubated overnight at 4°C.
   5. Anti-rabbit SAC-Cell solution (0.1 ml) was added to all tubes except the tubes containing the tracer. The contents were vortex mixed and then incubated at room temperature for 30 min.
   6. Wash buffer (2 ml) was added to all tubes except the tubes containing the tracer and the tubes were centrifuged at 3000 rpm for 5 minutes.
   7. The supernatant was decanted and the tubes were left to drain for no more than 1 minute.
   8. The wash buffer (1.5 ml) was added to the tubes again and the tubes were centrifuged at 3000 rpm for 5 minutes.
   9. The supernatant was decanted and the tubes were left to drain for no more than 1 minute. The tubes were centrifuged at 3000 rpm for 1 minute thereafter.
   10. All tubes were counted two times: first for 3 minutes and for 10 min.

### Results

A calibration curve precision profile was prepared in replicate (n=6) in rat plasma over the concentration range 0.080 ng/ml to 20.48 ng/ml of FGL_{L}. Good precision was observed over the whole concentration range with acceptable accuracy (<25%) down to 0.160 ng/ml, which gives an indication of the potential lower limit of quantification (LLOQ) of the assay in rat plasma. Increasing the counting time did improve the precision of the assay.

A calibration curve precision profile was prepared in replicate (n=6) in dog plasma over the concentration range 0.080 ng/ml to 20.48 ng/ml.

Pharmacokinetic parameters were calculated using the computer program WinNonlin Pro version 3.3 (Pharsight Corporation, USA). Values that were below the limit of quantification (BLQ) were entered as zero in the calculation of the mean.

There were also defined some toxicokinetic parameters. The toxicokinetic parameters that were measured included: maximum plasma concentrations of FGL_{L} (Cₘₐₓ), their times of occurrence (Tₘₐₓ), and plasma concentrations at 24 hours post-dose (C₂₄). Areas under the plasma FGL_{L} concentration-time curves within a 24-hour dosing interval (AUC₂₄) were estimated by the linear trapezoidal rule. In the calculation of AUC₂₄ values following intranasal administration, the times of the samples were relative to the start of administration (assuming a 10-minute break between each of the 5 administration procedures). Terminal rate constants (k) were estimated by fitting a linear regression of log concentration against time. Terminal half-lives (t½) were calculated as ln2/k.

FGL_{L} levels in the CSF from the two rats treated intravenously with 50 mg/kg were 321 and 269 ng/ml. FGL_{L} levels in the CSF from other animals treated subcutaneously with 100 mg/kg were 242, 92 and 81 ng/ml. These data provide evidence that FGL_{L} enters the CNS when administered parenterally.

Toxicokinetic samples taken after the first dose revealed:
Combined male and female exposures (50 mg/kg/day,i.v.): AUC = 51,026 h.ng/mL; Cₘₐₓ = 20,951 ng/mL.
Combined male and female exposures (100 mg/kg/day,s.c.): AUC = 50,119 h.ng/mL; Cₘₐₓ = 8,346 ng/mL.

**Table 12 shows the toxicokinetic parameters calculated in dogs receiving doses of FGL_{L} of 15, 30 and 75 mg/kg, s.c.**

| **Dose** | **Dog No** | **Day** | **Cₘₐₓ** (ng/ml) | **tₘₐₓ** (hr) | **AUC(0-24)** (hr*ng/ml) | **AUC** (hr*ng/ml) |
|---|---|---|---|---|---|---|
| 15 mg/kg | 2 | 1 | 7132 | 2.0 | 62965 | 84829 |
| | | 7 | 2204 | 2.0 | 41531 | 70419 |
| 30 mg/kg | 3 | 1 | 9863 | 2.0 | 81243 | 104608 |
| | | 7 | 2709 | 4.0 | 38208 | 58518 |
| 75 mg/kg | 4 | 1 | 9222 | 4.0 | 130497 | 161489 |
| | | 7 | 2281 | 8.0 | 40642 | 63191 |

Mean levels of FGL_{L} in the CSF from the three dogs (sampled one hour after the last dose at day 8) ranged from 4.1 ng/ml for animal No. 2 to 8.1 ng/ml for animal No. 3, indicating penetration of FGL_{L} into the CNS, following subcutaneous dosing.

## Claims

1. A compound comprising two peptide fragments comprising the identical amino acid sequence, said amino acid sequence being EVYVVAENQQGKSKA (SEQ ID NO:1), or a fragment thereof, said fragment being an amino acid sequence which has at least 40% of the length of SEQ ID NO:1 and which is capable of binding to fibroblast growth factor receptor,
wherein
said peptide sequences are connected to each other through a linker of the formula
X[(A)nCOOH][(B)mCOOH],
wherein
n and m independently are an integer of from 1 to 20,
A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, or substituted or unsubstituted aromatic moiety,
X is HN, H₂N(CR₂)pCR, RHN(CR₂)pCR, HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, [HOOC(A)n][HOOC(B)m]CR(CR₂)pCR, H₂N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, or [HOOC(A)n][HOOC(B)m](CR₂)p, wherein p is 0 or integer of from 1 to 20, each R indepentently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety.

2. A pharmaceutical composition comprising a compound as defined in claim 1.

3. Use of a compound as defined in claim 1 for the manufacture of a medicament for the treatment of postoperative nerve damage, traumatic nerve damage, impaired myelination of nerve fibers, postischaemic, e.g. resulting from a stroke, Parkinson's disease, Alzheimer's disease, Huntington's disease, dementias such as multiinfarct dementia, sclerosis, nerve degeneration associated with diabetes mellitus, disorders affecting the circadian clock or neuro-muscular transmission, and schizophrenia, mood disorders, such as manic depression.

4. Use of a compound as defined in claim 1 for the manufacture of a medicament for the promotion of wound-healing.

5. Use of a compound as defined in claim 1 for the manufacture of a medicament for the treatment of cancer.

6. Use of a compound as defined in claim 1 for the manufacture of a medicament for the prevention of death of heart muscle cells, such as after acute myocardial infarction, or after angiogenesis.

7. Use of a compound as defined in claim 1 for the manufacture of a medicament for revascularisation.

8. Use of a compound as defined in claim 1 for the manufacture of a medicament for the stimulation of the ability to learn and/or the short and/or long-term memory.

9. Use of a compound as defined in claim 1 for the manufacture of a medicament for the prevention of cell death due to ischemia.

10. Use of a compound as defined in claim 1 for the manufacture of a medicament for the prevention of body damages due to alcohol consumption.

11. Use of a compound as defined in claim 1 for the manufacture of a medicament for the treatment of prion diseases.

## Patentansprüche

1. Verbindung, die zwei Peptidfragmente aufweist, die die identische Aminosäuresequenz aufweisen, wobei die Aminosäuresequenz EVYVVAENQQGKSKA (SEQ ID NO:1) oder ein Fragment davon ist, wobei das Fragment eine Aminosäuresequenz ist, die zumindest 40 % der Länge von SEQ ID NO:1 hat und die in der Lage ist, an Fibroblasten-Wachstumsfaktorrezeptor zu binden,
worin die Peptidsequenzen durch einen Linker der Formel
X[(A)nCOOH][(B)mCOOH]
miteinander verbunden sind, worin
n und m unabhängig eine ganze Zahl von 1 bis 20 sind,
A und B unabhängig ein substituiertes oder unsubstituiertes C₁₋₁₀-Alkyl, ein substituiertes oder unsubstituiertes C₂₋₁₀-Alkenyl, eine substituierte oder unsubstituierte cyclische Gruppe, eine substituierte oder unsubstituierte heterocyclische Gruppe, eine substituierte oder unsubstituierte aromatische Gruppe sind, oder A und B zusammen eine substituierte oder unsubstituierte cyclische Gruppe, eine substituierte oder unsubstituierte heterocyclische Gruppe oder eine substituierte oder unsubstituierte aromatische Gruppe bilden,
X HN, H₂N(CR₂)pCR, RHN(CR₂)pCR, HO(CR₂)pCR, HS(CR₂)pCR, Halogen-(CR₂)pCR, HOOC(CR₂)pCR, ROOC(CR₂)pCR, HCO(CR₂)pCR, RCO(CR₂)pCR, [HOOC(A)n][HOOC(B)m]CR(CR₂)pCR, H₂N(CR₂)p, RHN(CR₂)p, HO(CR₂)p, HS(CR₂)p, Halogen-(CR₂)p, HOOC(CR₂)p, ROOC(CR₂)p, HCO(CR₂)p, RCO(CR₂)p oder [HOOC(A)n][HOOC(B)m](CR₂)p ist, worin p 0 oder eine ganze Zahl von 1 bis 20 ist, wobei jedes R unabhängig H oder ein substituiertes oder unsubstituiertes C₁₋₁₀-Alkyl, ein substituiertes oder unsubstituiertes C₂₋₁₀-Alkenyl, eine substituierte oder unsubstituierte cyclische Gruppe, eine substituierte oder unsubstituierte heterocyclische Gruppe, eine substituierte oder unsubstituierte aromatische Gruppe ist.

2. Pharmazeutische Zusammensetzung, die eine Verbindung aufweist, wie in Anspruch 1 definiert.

3. Verwendung einer Verbindung, wie in Anspruch 1 definiert, für die Herstellung eines Medikaments für die Behandlung einer postoperativen Nervenbeschädigung, einer traumatischen Nervenbeschädigung, einer beeinträchtigten Myelinisierung von Nervenfasern, einer postischämischen, z.B. von einem Schlaganfall resultierenden, Parkinson-Erkrankung, Alzheimer-Erkrankung, Huntington-Erkrankung, Demenzen wie etwa Multiinfarktdemenz, Sklerose, Diabetes mellitus zugeordneter Nervendegeneration, Störungen, welche die Circadiane Uhr oder neuromuskulare Übertragung beeinträchtigen, sowie Schizophrenie, Gemütsbeeinträchtigungen wie etwa manische Depression.

4. Verwendung einer Verbindung wie in Anspruch 1 definiert für die Herstellung eines Medikaments zur Förderung der Wundheilung.

5. Verwendung einer Verbindung wie in Anspruch 1 definiert für die Herstellung eines Medikaments für die Behandlung von Krebs.

6. Verwendung einer Verbindung wie in Anspruch 1 definiert für die Herstellung eines Medikaments für die Verhinderung vom Tod von Herzmuskelzellen, wie etwa nach einem akuten Herzmuskelinfarkt, oder nach Angiogenese.

7. Verwendung einer Verbindung wie in Anspruch 1 definiert für die Herstellung eines Medikaments zur Revaskularisierung.

8. Verwendung einer Verbindung wie in Anspruch 1 definiert für die Herstellung eines Medikaments für die Stimulation der Fähigkeit zu lernen und/oder des Kurz- und/oder Langzeitgedächtnisses.

9. Verwendung einer Verbindung wie in Anspruch 1 definiert für die Herstellung eines Medikaments für die Verhinderung von Zelltod aufgrund von Ischämie.

10. Verwendung einer Verbindung wie in Anspruch 1 definiert für die Herstellung eines Medikaments für die Verhinderung von Körperschäden aufgrund von Alkoholkonsum.

11. Verwendung einer Verbindung wie in Anspruch 1 definiert für die Herstellung eines Medikaments für die Behandlung von Prion-Erkrankungen.

## Revendications

1. Composé comprenant deux fragments peptidiques comprenant la séquence d'acides aminés identique, ladite séquence d'acides aminés étant EVYVVAENQQGKSKA (SEQ ID NO : 1), ou un fragment de celle-ci, ledit fragment étant une séquence d'acides aminés qui représente au moins 40 % de la longueur de SEQ ID NO : 1 et qui est capable de se lier au récepteur du facteur de croissance des fibroblastes,
dans lequel
lesdites séquences peptidiques sont reliées l'une à l'autre par l'intermédiaire d'un lieur de formule
X[(A)nCOOH][(B)mCOOH],
dans laquelle
n et m représentent indépendamment un nombre entier de 1 à 20,
A et B représentent indépendamment un groupe alkyle C₁₋₁₀ substitué ou non substitué, un groupe alcényle C₂₋₁₀ substitué ou non substitué, un radical cyclique substitué ou non substitué, un radical hétérocyclique substitué ou non substitué, un radical aromatique substitué ou non substitué, ou A et B forment ensemble un radical cyclique substitué ou non substitué, un radical hétérocyclique substitué ou non substitué ou un radical aromatique substitué ou non substitué,
X représente HN, H₂N(CR₂)pCR, RHN(CR₂)pCR, HO(CR₂)pCR, HS(CR₂)pCR, halogène-(CR₂)-CR, HOOC(CR₂)pCR, ROOC(CR₂)pCR, HCO(CR₂)pCR, RCO(CR₂)pCR, [HOOC(A)n][HOOC(B)m]CR(CR₂)pCR, H₂N(CR₂)p, RHN(CR₂)p, HO(CR₂)p, HS(CR₂)p, halogène-(CR₂)p, HOOC(CR₂)p, ROOC(CR₂)p, HCO(CR₂)p, RCO(CR₂)p ou [HOOC(A)n][HOOC(B)m](CR₂)p, dans laquelle p vaut 0 ou représente un nombre entier de 1 à 20, chaque R représente indépendamment H ou un groupe alkyle en C₁ à C₁₀ substitué ou non substitué, un groupe alcényle en C₂ à C₁₀ substitué ou non substitué, un radical cyclique substitué ou non substitué, un radical hétérocyclique substitué ou non substitué, un radical aromatique substitué ou non substitué.

2. Composition pharmaceutique comprenant un composé tel que défini dans la revendication 1.

3. Utilisation d'un composé tel que défini dans la revendication 1 pour la fabrication d'un médicament destiné au traitement de lésions nerveuses postopératoires, de lésions nerveuses traumatiques, d'une altération de la myélinisation des fibres nerveuses, post-ischémique, par exemple résultant d'un accident vasculaire cérébral, de la maladie de Parkinson, de la maladie d'Alzheimer, de la maladie de Huntington, de démences telles que la démence vasculaire, de la sclérose, d'une dégénérescence nerveuse associée à un diabète, de troubles affectant le rythme circadien ou la transmission neuromusculaire, et de la schizophrénie, de troubles de l'humeur, tels que la psychose maniaco-dépressive.

4. Utilisation d'un composé tel que défini dans la revendication 1 pour la fabrication d'un médicament destiné à favoriser la cicatrisation.

5. Utilisation d'un composé tel que défini dans la revendication 1 pour la fabrication d'un médicament destiné au traitement du cancer.

6. Utilisation d'un composé tel que défini dans la revendication 1 pour la fabrication d'un médicament destiné à la prévention de la mort des cellules du muscle cardiaque, comme après un infarctus aigu du myocarde ou après une angiogenèse.

7. Utilisation d'un composé tel que défini dans la revendication 1 pour la fabrication d'un médicament destiné à la revascularisation.

8. Utilisation d'un composé tel que défini dans la revendication 1 pour la fabrication d'un médicament destiné à la stimulation de la capacité d'apprentissage et/ou de la mémoire à court et/ou long terme.

9. Utilisation d'un composé tel que défini dans la revendication 1 pour la fabrication d'un médicament destiné à la prévention de la mort cellulaire due à une ischémie.

10. Utilisation d'un composé tel que défini dans la revendication 1 pour la fabrication d'un médicament destiné à la prévention de lésions corporelles dues à la consommation d'alcool.

11. Utilisation d'un composé tel que défini dans la revendication 1 pour la fabrication d'un médicament destiné au traitement de maladies à prion.
